# EUROPEAN PATENT APPLICATION

(11) **EP 4 850 689 A2**
(43) Date of publication of application: **09.12.2026**
(21) Application number: 26199216.8
(22) Date of filing: 20.08.2013
(51) Int. Cl.: A61K 39/395

(54) **METHODS FOR TREATING OR PREVENTING ASTHMA BY ADMINISTERING AN IL-4R ANTAGONIST**

(30) Priority: 21.08.2012 US 201261691625 P; 29.01.2013 US 201361758097 P; 06.02.2013 US 201361761279 P; 14.03.2013 US 201361783796 P; 27.03.2013 US 201361805797 P; 16.07.2013 FR 1356994
(62) Document number(s) of the earlier application(s) in accordance with Art. 76 EPC: 23202532.0 / 4 324 479; 21199451.2 / 4 011 915; 18194745.8 / 3 470 432; 13759908.0 / 2 888 281
(71) Applicant: Sanofi Biotechnology, 94250 Gentilly (FR); Regeneron Pharmaceuticals, Inc., Tarrytown, NY 10591 (US)
(72) Inventor: ARDELENAU, Marius, Tarrytown 10591-6707 (US); GANDHI, Namita, Tarrytown 10591-6707 (US); GRAHAM, Neil, Tarrytown 10591-6707 (US); KIRKESSELI, Stephane C., Bridgewater 08807 (US); KUNDU, Sudeep, Bridgewater 08807 (US); RADIN, Allen, Tarrytown 10591-6707 (US); ROCKLIN, Ross E., Bridgewater 08807 (US); WEINSTEIN, Steven P., Tarrytown 10591-6707 (US); DAVIDSON HAMILTON, Jennifer, Tarrytown 10591-6707 (US); MING, Jeffrey, Bridgewater 08807 (US)
(74) Representative: Zwicker, Jörk

(57) **Abstract**

The present invention provides methods for treating or preventing asthma and associated conditions in a patient. The methods of the present invention comprise administering to a subject in need thereof a therapeutic composition comprising an interleukin-4 receptor (IL-4R) antagonist, such as an anti-IL-4R antibody.

## Description

### RELATED APPLICATIONS

This application claims the benefit of priority of US Provisional Application No. 61/691,625, filed August 21, 2012; US Provisional Application No. 61/758,097 filed January 29, 2013; US Provisional Application No. 61/761,279, filed February 6, 2013; US Provisional Application No. 61/783,796, filed March 14, 2013; US Provisional Application No. 61/805,797, filed March 27, 2013; and French Application No. 1356994, filed 16 July 2013. The contents of each of the aforementioned applications are hereby incorporated by reference herein in their entireties.

### FIELD OF THE INVENTION

The present invention relates to the treatment and/or prevention of asthma and related conditions. More specifically, the invention relates to the administration of an interleukin-4 receptor (IL-4R) antagonist to treat or prevent asthma in a patient in need thereof.

### BACKGROUND

Asthma is a chronic inflammatory disease of the airways characterized by airway hyper responsiveness, acute and chronic bronchoconstriction, airway edema, and mucus plugging. The inflammation component of asthma is thought to involve many cell types, including mast cells, eosinophils, T lymphocytes, neutrophils, and epithelial cells, and their biological products. Patients with asthma most often present with symptoms of wheezing, shortness of breath, cough, and chest tightness. For most asthma patients, a regimen of controller therapy and bronchodilator therapy provides adequate long-term control. Inhaled corticosteroids (ICS) are considered the "gold standard" in controlling asthma symptoms, and inhaled beta2-agonists are the most effective bronchodilators currently available. Studies have shown that combination therapy of an ICS with an inhaled long-acting beta2-agonist (LABA) provides better asthma control than high doses of ICS alone. Consequently, combination therapy has been the recommended treatment for subjects who are not controlled on low doses of ICS alone.

Nonetheless, it is estimated that 5% to 10% of the population with asthma has symptomatic disease despite maximum recommended treatment with combinations of antiinflammatory and bronchodilator drugs. Furthermore, this severe asthma population accounts for up to 50% of the total health cost through hospital admissions, use of emergency services, and unscheduled physician visits. There is an unmet need for a new therapy in this severe asthma population as many of these patients are poorly responsive to ICS due to a number of cellular and molecular mechanisms. In addition, the long term adverse effects of systemic and inhaled corticosteroids on bone metabolism, adrenal function, and growth in children lead to attempts to minimize the amount of corticosteroid usage. Although a large portion of asthma patients are managed reasonably well with current treatments, patients with severe corticosteroid-refractory asthma have few therapeutic treatment options that can adequately control the disease. The consequence of unresponsiveness to therapy or lack of compliance with therapy is loss of asthma control and ultimately asthma exacerbation.

One of the reasons for the poor response to medication in some patients with severe asthma may be the heterogeneity of the disease. Interest is increasing in understanding these distinct phenotypes because targeted therapy is more likely to be successful in patients with similar underlying pathobiological features. Recent therapeutic approaches in asthma have focused on trying to control the T helper cell-2 response. Up-regulation of interleukin-4 (IL-4) and interleukin-13 (IL-13) has been implicated as an important inflammatory component of asthma disease progression.

Accordingly, a need exists in the art for novel targeted therapies for the treatment and/or prevention of asthma.

### BRIEF SUMMARY OF THE INVENTION

According to one aspect of the present invention, methods are provided for reducing the incidence of asthma exacerbations in a subject in need thereof. In a related aspect, methods are provided for improving one or more asthma-associated parameter(s) in a subject in need thereof. In yet another aspect of the present invention, methods are provided for treating asthma, *e.g.*, moderate-to-severe eosinophilic asthma, in a subject in need thereof.

The methods featured in the invention comprise administering to a subject a therapeutically effective amount of a pharmaceutical composition comprising an interleukin-4 receptor (IL-4R) antagonist. According to certain embodiments, the IL-4R antagonist is an antibody or antigen-binding fragment thereof that specifically binds IL-4R. Exemplary anti-IL-4R antibodies that can be used in the context of the methods of the present invention are described elsewhere herein, including working Example 1. For example, in one embodiment, the IL-4R antagonist is an antibody or antigen-binding fragment thereof that specifically binds to an IL-4R, and comprises the heavy chain and light chain (complementarity determining region) CDR sequences from the heavy chain variable region (HCVR) and light chain variable region (LCVR) of SEQ ID NOs:162 and 164, respectively.

In one embodiment, a method for reducing the incidence of one or more asthma exacerbations in a subject in need thereof is provided by administering an antibody or antigen binding fragment thereof that specifically binds IL-4R. The asthma exacerbation can be one or more of the following: (a) a 30% or greater reduction from baseline in morning peak expiratory flow (PEF) on two consecutive days; (b) six or more additional reliever puffs of albuterol or levalbuterol in a 24 hour period (compared to baseline) on two consecutive days; and (c) a deterioration of asthma requiring: (i) systemic (oral and/or parenteral) steroid treatment, or (ii) an increase in inhaled corticosteroids to at least 4 times the last dose received prior to discontinuation, or hospitalization.

In various embodiments, methods for improving one or more asthma-associated parameters comprise administering to a subject in need thereof, a therapeutically effective amount of an IL-4R antagonist, wherein the improvement in an asthma-associated parameter is defined as one of the following: an increase from baseline of FEV1; an increase from baseline of AM PEF; an increase from baseline of PM PEF; a decrease from baseline of albuterol/levalbuterol use; a decrease from baseline of nighttime awakenings; and/or a decrease from baseline of SNOT-22 score. Examples of asthma-associated parameters include: (a) forced expiratory volume in 1 second (FEV1); (b) peak expiratory flow rate (PEF), including morning PEF (AM PEF) and evening PEF (PM PEF); (c) use of an inhaled bronchodilator, such as albuterol or levalbuterol; (d) five-item Asthma Control Questionnaire (ACQ5) score; (d) nighttime awakenings; and (e) 22-item Sino-Nasal Outcome Test (SNOT-22) score. In one embodiment, the improvement in an asthma-associated parameter is an increase of at least 0.10 L from baseline of FEV1. In one embodiment, the improvement in an asthma-associated parameter is an increase of at least 10.0 L/min from baseline of AM PEF. In one embodiment, the improvement in an asthma-associated parameter is an increase of at least 1.0 L/min from baseline of PM PEF. In one embodiment, the improvement in an asthma-associated parameter is a decrease in albuterol/levalbuterol use of at least 1 puff(s) per day from baseline. In one embodiment, the improvement in an asthma-associated parameter is a decrease of at least 0.5 points from baseline in ACQ5 score. In one embodiment, the improvement in an asthma-associated parameter is a decrease of at least 0.2 times per night from baseline of nighttime awakenings. In one embodiment, the improvement in an asthma-associated parameter is a decrease of at least 5 points from baseline in SNOT-22 score.

The invention also provides methods for reducing the incidence of asthma exacerbations, or improving one or more asthma-associated parameter(s) in a subject in need thereof, wherein the methods comprise sequentially administering to a subject in need thereof a single initial dose of a pharmaceutical composition comprising an IL-4R antagonist (*e.g.*, an anti-IL-4R antibody or antigen-binding fragment thereof), followed by one or more secondary doses of the pharmaceutical composition comprising the IL-4R antagonist. The pharmaceutical composition comprising the IL-4R antagonist may be administered subcutaneously, intranasally or intravenously to the subject in need thereof.

According to certain embodiments, the invention provides methods for reducing the incidence of asthma exacerbations, or improving one or more asthma-associated parameter(s) in a subject in need thereof, wherein the methods comprise administering to the subject about 75 to about 300 mg of a pharmaceutical composition comprising an antibody or antigen-binding fragment thereof that specifically binds IL-4R. According to this aspect, the pharmaceutical composition may be administered to the subject at a dosing frequency of, *e.g.*, once a week.

The invention further includes methods for treating asthma (*e.g.*, eosinophilic asthma, moderate to severe eosinophilic asthma, etc.) by selecting a subject who exhibits one or more symptoms or indicia of asthma, and administering to the patient a pharmaceutical composition comprising an IL-4R antagonist (*e.g.*, an anti-IL-4R antibody or antigen-binding fragment thereof), wherein the subject exhibits one or more of the following symptoms or indicia of asthma: (1) the subject has been treated with a stable dose of either fluticasone/salmeterol combination therapy (250/50 µg BID or 500/50 µg BID) or budesonide/formoterol combination therapy (160/9 µg BID or 320/9 µg BID) for at least 3 months prior to screening; (2) the subject has blood eosinophils greater than or equal to 300 cell/µL; (3) the subject has sputum eosinophils greater than or equal to 3%; (4) the subject has elevated levels of IgE, thymus and activation regulation chemokine (TARC), eotaxin-3, carcinoembryonic antigen (CEA), YKL-40, or periostin; (5) the subject has an elevated level of fractional exhaled nitric oxide (FeNO); and/or (6) the subject has an Asthma Control Questionnaire (ACQ5) score greater than or equal to 1.0.

Embodiments featured in the invention are directed to methods of treatment, as described above, further comprising administration of a second therapeutic agent in combination with the IL-4R antagonist. The second therapeutic agent may be administered to a subject in need thereof before, after or concurrent with IL-4R antagonist. Exemplary second therapeutic agents include, but are not limited to, one or more of the following in combination: IL-1 inhibitors, IL-5 inhibitors, IL-8 inhibitors, IgE inhibitors, tumor necrosis factor (TNF) inhibitors, corticosteroids, long acting beta2-agonists, and leukotriene inhibitors.

In another aspect, the invention provides methods to reduce or eliminate an asthma patient's dependence on background asthma therapy comprising selecting a patient who has moderate-to-severe asthma that is uncontrolled or partially controlled with background asthma therapy; administering to the patient a defined dose of an IL-4R antagonist while maintaining the patient's background therapy; and gradually reducing the dosage of one or more components of the background therapy over a subsequent treatment period while continuing to administer the IL-4R antagonist. In certain embodiments, the background therapy comprises an inhaled corticosteroid

(ICS), a long-acting beta-agonist (LABA), or a combination of an ICS and a LABA. In some embodiments, the background therapy is gradually reduced or withdrawn over a period of 2 - 8 weeks. In some embodiments, one component of the background therapy is eliminated after an initial treatment period. In one embodiment, the background therapy is gradually reduced over a subsequent treatment period.

In yet another aspect, the invention provides a method for identifying a patient and treating moderate-to-severe asthma by selecting a patient with an elevated level of a biomarker, such as thymus and activation-regulated chemokine (TARC), IgE, eotaxin-3, periostin, carcinoembryonic antigen (CEA), or YKL-40, or having an increased level of fractional exhaled nitric oxide (FeNO); and administering to the patient a therapeutically effective amount of an IL-4R antagonist.

In another aspect, the invention features a method for monitoring effectiveness of treatment of moderate-to-severe asthma in a subject, such as by (a) determining the expression level of a biomarker, such as one or both of TARC or eotaxin-3, or the total serum level of IgE in a biological sample acquired from the subject before treatment with an IL-4R antagonist; (b) determining the expression level of the biomarker in a biological sample acquired from the subject after treatment with the IL-4R antagonist; (c) comparing the expression level determined in step (a) with the level in step (b), and (d) concluding that the treatment is effective when the level determined in step (b) is lower than the level determined in step (a), or concluding that the treatment is not effective when the level determined in step (b) is the same as or higher than the level determined in step (a).

In one embodiment, the biomarker is FeNO, and if FeNO levels decrease following administration of the antagonist, then treatment with the IL-4R antagonist is determined to be effective.

The expression level of the biomarker can be determined, for example, 1 week, 2 weeks, 3 weeks, 4 weeks, 5 weeks, or longer after administration of the IL-4R antagonist, and compared to the expression level prior to administration of the antagonist. The dose or the dosing regimen of the IL-4R antagonist (e.g., an anti-IL4R antibody) can be adjusted following the determination. For example, if the expression of the biomarker fails to decrease within 1 week, 2 weeks, 3 weeks, 4 weeks, 5 weeks, or longer following administration of the antagonist, then treatment with the antagonist can be stopped, or the dose of the antagonist can be increased. If expression of the biomarker decreases following administration of the antagonist, the dosage of the antagonist can be maintained or decreased, such as to identify a minimal effective dose. In some embodiments, treatment is maintained at the minimal effective dose.

In another aspect, the invention features a method for monitoring a subject's response to treatment with an IL-4R antagonist, wherein the subject has moderate-to-severe asthma, such as by acquiring information regarding expression level of a biomarker, such as one or both of TARC or eotaxin-3, or total serum level of IgE in a biological sample from the subject following administration of the IL-4R antagonist to the subject, and providing an indication that treatment should be continued if the expression level of the biomarker has decreased as compared to the level before treatment with the IL-4R antagonist. In one embodiment the biomarker is FeNO, and if FeNO levels are determined to decrease following administration of the antibody, then an indication is provided to continue treatment with the IL-4R antagonist.

The invention also includes an IL-4R antagonist as disclosed herein for use in the manufacture of a medicament for the treatment and/or prevention of asthma (*e.g.*, eosinophilic asthma, moderate to severe eosinophilic asthma, etc.) or for treating any of the other indications or conditions disclosed herein.

The invention also includes an IL-4R antagonist as disclosed herein for use in the treatment and/or prevention of asthma (*e.g.*, eosinophilic asthma, moderate to severe eosinophilic asthma, etc.) or for treating and/or prevention of any of the other indications or conditions disclosed herein.

The invention includes a pharmaceutical composition comprising an anti-IL4R antibody antagonist or an antigen binding fragment thereof for use in the treatment and/or prevention of asthma and related conditions.

The invention also includes a pharmaceutical composition comprising an anti-IL4R antibody antagonist or an antigen binding fragment thereof for use in reducing the incidence of one or more asthma exacerbations in a subject in need thereof.

In addition, the invention includes a pharmaceutical composition comprising an anti-IL4R antibody antagonist or an antigen binding fragment thereof for use in improving one or more asthma-associated parameter(s) in a subject in need thereof.

The invention includes a pharmaceutical composition comprising an anti-IL4R antibody antagonist or an antigen binding fragment thereof for use in the treatment of asthma and related conditions in a patient having an elevated level of a biomarker selected from the group consisting of thymus and activation-regulated chemokine (TARC), IgE, eotaxin-3, periostin, carcinoembryonic antigen (CEA), YKL-40, and fractional exhaled nitric oxide (FeNO).

The invention further includes a pharmaceutical composition comprising an anti-IL4R antibody antagonist or an antigen binding fragment thereof for use in the treatment of asthma or moderate to severe eosinophilic asthma in a subject in need thereof wherein the treatment comprises testing the patient for the presence of a blood eosinophil level of at least 300 cells per microliter and/or a sputum eosinophil level of at least 3% and beginning/continuing administration of the pharmaceutical composition if such blood eosinophil level and/or sputum eosinophil level is found.

Other embodiments of the invention will become apparent from a review of the ensuing detailed description.

### BRIEF DESCRIPTION OF THE FIGURES

**Figure 1** is a graph that shows a Kaplan-Meier plot of time to asthma exacerbation in patients treated with placebo (open circles) as compared to patients treated with anti-IL-4R antibody mAb1 (asterisks). The effect of the treatment with an anti-IL-4R antibody mAb1 is sustained over time, including after 8 weeks, when patients are at higher risk of developing exacerbations due to steroid withdrawal. Broken vertical lines indicate withdrawal of LABA.
**Figure 2** is a graph that shows the mean change from baseline in forced expiratory volume in 1 second (FEV1) in liters in patients treated with placebo (open triangles) as compared to patients treated with anti-IL-4R antibody mAb1 (closed circles). Broken vertical lines indicate withdrawal of LABA.
**Figure 3** is a graph that shows the mean change from baseline in morning peak expiratory flow rate (AM PEF) in liters per minute in patients treated with placebo (open triangles) as compared to patients treated with anti-IL-4R antibody mAb1 (closed circles).
**Figure 4** is a graph that shows the mean change from baseline in evening peak expiratory flow rate (PM PEF) in liters per minute in patients treated with placebo (open triangles) as compared to patients treated with anti-IL-4R antibody mAb1 (closed circles).
**Figure 5** is a graph that shows the mean change from baseline in albuterol use in inhalations per day in patients treated with placebo (open triangles) as compared to patients treated with anti-IL-4R antibody mAb1 (closed circles). Broken vertical lines indicate withdrawal of LABA.
**Figure 6** is a graph that shows the mean change from baseline in five-item asthma control questionnaire (ACQ5) score in patients treated with placebo (open triangles) as compared to patients treated with anti-IL-4R antibody mAb1 (closed circles). Broken vertical lines indicate withdrawal of LABA.
**Figure 7** is a graph that shows the mean change from baseline in nocturnal awakenings in number of times per night in patients treated with placebo (open triangles) as compared to patients treated with anti-IL-4R antibody mAb1 (closed circles). Broken vertical lines indicate withdrawal of LABA.
**Figure 8** is a graph that shows the mean percentage change from baseline in TARC by visit at week 0, 1, 4, 8, and 12 of the mITT population treated with placebo (closed circles) as compared to patients treated with anti-IL-4R antibody mAb1 (closed squares). Broken vertical lines indicate withdrawal of LABA.
**Figure 9** is a graph that shows the mean percentage change from baseline in Eotaxin-3 by visit at week 0, 1, 4, 8, and 12 of the mITT population treated with placebo (closed circles) as compared to patients treated with anti-IL-4R antibody mAb1 (closed squares). Broken vertical lines indicate withdrawal of LABA.
**Figure 10** is a graph that shows the mean percentage change from baseline in total IgE by visit at week 0, 1, 4, 8, and 12 in the mITT population treated with placebo (closed circles) as compared to patients treated with anti-IL-4R antibody mAb1 (closed squares). Broken vertical lines indicate withdrawal of LABA.
**Figure 11** is a graph that shows the mean percentage change from baseline in periostin by visit at week 0, 1, 4, 8, and 12 in the mITT population treated with placebo (closed circles) as compared to patients treated with anti-IL-4R antibody mAb1 (closed squares).
**Figure 12** is a graph that shows the mean percentage change from baseline in carcinoembryogenic antigen (CEA) by visit at week 0, 1, 4, 8, and 12 in the mITT population treated with placebo (closed circles) as compared to patients treated with anti-IL-4R antibody mAb1 (closed squares).
**Figure 13** is a graph that shows the mean percentage change from baseline in YKL-40 by visit at week 0, 1, 4, 8, and 12 in the mITT population treated with placebo (closed circles) as compared to patients treated with anti-IL-4R antibody mAb1 (closed squares).
**Figure 14** is a graph that shows the mean percentage change from baseline in blood eosinophils by visit at week 0, 1, 2, 4, 6, 8, and 12 in the mITT population treated with placebo (closed circles) as compared to patients treated with anti-IL-4R antibody mAb1 (closed squares).
**Figure 15** is a graph that shows the mean percent change in fractional exhaled nitric oxide (NO) level from baseline by visit at week 0, 4, 8, and 12 in the mITT population treated with placebo (closed circles) as compared to patients treated with anti-IL-4R antibody mAb1 (closed squares). Broken vertical lines indicate withdrawal of LABA.
**Figure 16** is a scatter plot of the change in FEV1 (L) from baseline at week 12 versus baseline fraction of exhaled nitric oxide (FeNO) (PPB) in the mITT population treated with placebo (open circles and full line) as compared to patients treated with anti-IL-4R antibody mAb1 (plus sign and dashed line).
**Figure 17** is a scatter plot of the change in AM-PEF (L/min) from baseline at week 12 versus baseline FeNO (PPB) in the mITT population treated with placebo (open circles and full line) as compared to patients treated with anti-IL-4R antibody mAb1 (plus sign and dashed line).
**Figure 18** is a scatter plot of the change in PM-PEF (L/min) from baseline at week 12 versus baseline FeNO (PPB) in the mITT population treated with placebo (open circles and full line) as compared to patients treated with anti-IL-4R antibody mAb1 (plus sign and dashed line).
**Figure 19** is a scatter plot of the change in FEV1 from baseline at week 12 (L) versus blood eosinophils count (GIGA/L) in the mITT population treated with placebo (open circles and full line) as compared to patients treated with anti-IL-4R antibody mAb1 (plus sign and dashed line).
**Figure 20** is a scatter plot of the change in ACQ from baseline at week 12 versus blood eosinophils count (GIGA/L) in the mITT population treated with placebo (open circles and full line) as compared to patients treated with anti-IL-4R antibody mAb1 (plus sign and dashed line).
**Figure 21** is a scatter plot of the change in albuterol/levalbuterol use per day from baseline at week 12 versus blood eosinophils count (GIGA/L) in the mITT population treated with placebo (open circles and full line) as compared to patients treated with anti-IL-4R antibody mAb1 (plus sign and dashed line).
**Figure 22** is a scatter plot of the change in ACQ from baseline at week 12 versus baseline periostin in the mITT population treated with placebo (open circles and full line) as compared to patients treated with anti-IL-4R antibody mAb1 (plus sign and dashed line).
**Figure 23** is a scatter plot of the change in ACQ from baseline at week 12 versus YKL-40 in the mITT population treated with placebo (open circles and full line) as compared to patients treated with anti-IL-4R antibody mAb1 (plus sign and dashed line).
**Figure 24** is a schematic representation of timing and dosing regimens for treatment of asthma patients.
**Figure 25** is a diagram describing the patient disposition of a randomized, placebo-controlled, double-blind, parallel group study conducted with once-a-week subcutaneous administration of either 300 mg mAb1 or placebo for 12 weeks to patients with persistent moderate-to-severe eosinophilic asthma who were partially controlled/uncontrolled by inhaled corticosteroid (ICS) and long-acting beta2 agonist (LABA) therapy.
**Figures 26A and 26B** are scatter plots of morning (A) and evening (B) asthma symptoms measured over 12 weeks following administration of placebo (open triangles) or mAb1 (closed circles).
**Figure 27** is a graph showing the serum IgE levels in humanized IL-4/IL-4R mice (IL-4^{hu/hu} IL-4Rα^{hu/hu}) following house dust mite (HDM) challenge and treatment with either anti-IL-4R antibody or an IL-13Ra2-Fc decoy receptor molecule, or mock treatment. Measurements were made on samples taken at Day 40 (24 hours prior to first dose of treatment) and at the end of the experiment on Day 85.
**Figure 28** is a graph showing the serum IgE levels in wild-type (Balb/c) mice following house dust mite (HDM) challenge and treatment with either isotype control, anti-IL-4R antibody or an IL-13Ra2-Fc decoy receptor molecule, or mock treatment.
**Figure 29** is a graph showing the collagen content (expressed in terms of µg/lobe) of the lungs of humanized IL-4/IL-4R mice following HDM challenge and indicated treatment.

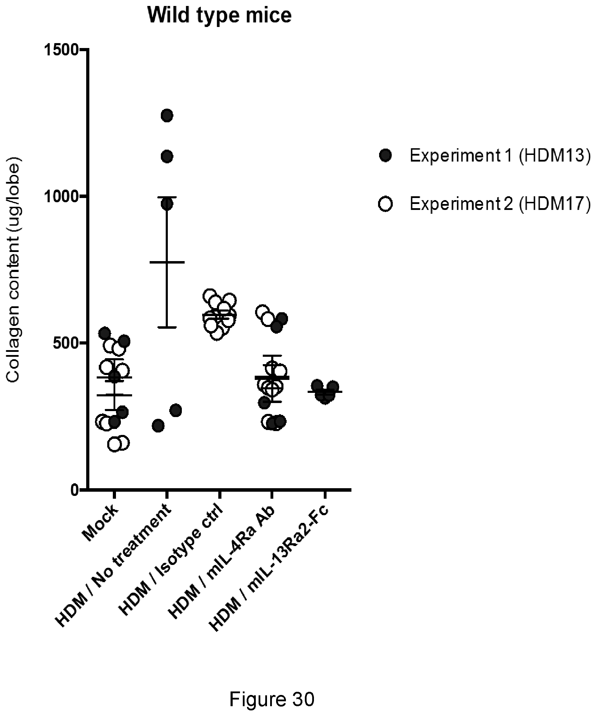

**Figure 30** is a graph showing the collagen content (expressed in terms of µg/lobe) of the lungs of wild-type mice following HDM challenge and indicated treatment.

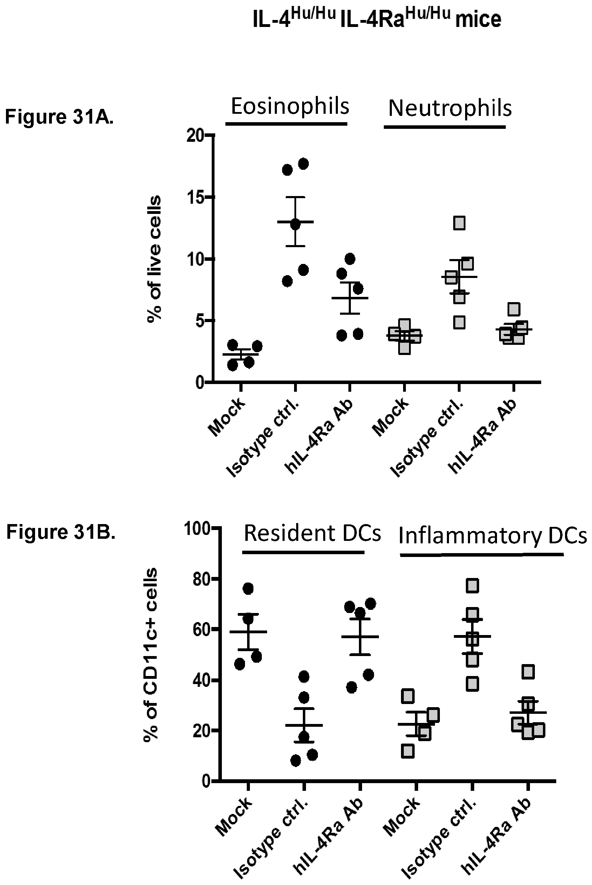

**Figure 31A** is a graph showing the levels of eosinophils and neutrophils in humanized IL-4/IL-4R mice following HDM challenge and indicated treatment, and **Figure 31B** is a graph showing the levels of resident dendritic cells and inflammatory dendritic cells, in humanized IL-4/IL-4R mice following HDM challenge and indicated treatment.

### DETAILED DESCRIPTION

Before the present invention is described, it is to be understood that this invention is not limited to particular methods and experimental conditions described, as such methods and conditions may vary. It is also to be understood that the terminology used herein is for the purpose of describing particular embodiments only, and is not intended to be limiting, because the scope of the present invention will be limited only by the appended claims.

Unless defined otherwise, all technical and scientific terms used herein have the same meaning as commonly understood by one of ordinary skill in the art to which this invention belongs.

As used herein, the term "about," when used in reference to a particular recited numerical value, means that the value may vary from the recited value by no more than 1%. For example, as used herein, the expression "about 100" includes 99 and 101 and all values in between (*e.g.*, 99.1, 99.2, 99.3, 99.4, etc.).

As used herein, the terms "treat", "treating", or the like, mean to alleviate symptoms, eliminate the causation of symptoms either on a temporary or permanent basis, or to prevent or slow the appearance of symptoms of the named disorder or condition.

Although any methods and materials similar or equivalent to those described herein can be used in the practice of the present invention, the preferred methods and materials are now described. All publications mentioned herein are incorporated herein by reference in their entirety.

### Methods for Reducing the Incidence of Asthma Exacerbations

The invention includes methods for reducing the incidence of asthma exacerbations in a subject in need thereof comprising administering a pharmaceutical composition comprising an interleukin-4 receptor (IL-4R) antagonist to the subject. As used herein, the expression "asthma exacerbation" means an increase in the severity and/or frequency and/or duration of one or more symptoms or indicia of asthma. An "asthma exacerbation" also includes any deterioration in the respiratory health of a subject that requires and or is treatable by a therapeutic intervention for asthma (such as, *e.g.*, steroid treatment, inhaled corticosteroid treatment, hospitalization, etc.). According to certain embodiments of the invention, an asthma exacerbation is defined as one or more of the following: (a) a 30% or greater reduction from baseline in morning peak expiratory flow ("AM PEF," as defined elsewhere herein) on two consecutive days; (b) six or more additional reliever puffs of albuterol or levalbuterol in a 24 hour period (compared to baseline) on two consecutive days; and (c) a deterioration of asthma (*e.g.*, as determined by a physician or other medical practitioner) requiring at least one of: (i) systemic (oral and/or parenteral) steroid treatment, or (ii) an increase in inhaled corticosteroids to at least 4 times the baseline level, or (iii) hospitalization.

In certain instances, an asthma exacerbation may be categorized as a "severe asthma exacerbation." A severe asthma exacerbation means an incident requiring immediate intervention in the form of treatment with either systemic corticosteroids or with inhaled corticosteroids at four or more times the dose taken prior to the incident. The general expression "asthma exacerbation" therefore includes and encompasses the more specific subcategory of "severe asthma exacerbations." Accordingly, the invention includes methods for reducing the incidence of severe asthma exacerbations in a patient in need thereof.

A "reduction in the incidence" of an asthma exacerbation means that a subject who has received a pharmaceutical composition of the present invention experiences fewer asthma exacerbations (*i.e.*, at least one fewer exacerbation) after treatment than before treatment, or experiences no asthma exacerbations for at least 4 weeks (*e.g.*, 4, 6, 8, 12, 14, or more weeks) following initiation of treatment with a pharmaceutical composition of the present invention. A "reduction in the incidence" of an asthma exacerbation alternatively means that, following administration of a pharmaceutical composition of the present invention, the likelihood that a subject experiences an asthma exacerbation is decreased by at least 10% (*e.g.*, 10%, 15%, 20%, 25%, 30%, 35%, 40%, 45%, 50%, or more) as compared to a subject who has not received a pharmaceutical composition of the present invention.

### Methods for Improving Asthma-Associated Parameters

The invention also includes methods for improving one or more asthma-associated parameters in a subject in need thereof, wherein the methods comprise administering a pharmaceutical composition comprising an interleukin-4 receptor (IL-4R) antagonist to the subject. For purposes of the invention, a reduction in the incidence of an asthma exacerbation (as described above) may correlate with an improvement in one or more asthma-associated parameters; however, such a correlation is not necessarily observed in all cases.

Examples of "asthma-associated parameters" include: (a) forced expiratory volume in 1 second (FEV1); (b) peak expiratory flow rate (PEF), including morning PEF (AM PEF) and evening PEF (PM PEF); (c) use of an inhaled bronchodilator such as albuterol or levalbuterol; (d) five-item Asthma Control Questionnaire (ACQ5) score; (d) nighttime awakenings; and (e) 22-item Sino-Nasal Outcome Test (SNOT-22) score. An "improvement in an asthma-associated parameter" means an increase from baseline of one or more of FEV1, AM PEF or PM PEF, and/or a decrease from baseline of one or more of daily albuterol/levalbuterol use, ACQ5 score, average nighttime awakenings or SNOT-22 score. As used herein, the term "baseline," with regard to an asthma-associated parameter, means the numerical value of the asthma-associated parameter for a patient prior to or at the time of administration of a pharmaceutical composition of the present invention.

To determine whether an asthma-associated parameter has "improved," the parameter is quantified at baseline and at a time point after administration of the pharmaceutical composition of the present invention. For example, an asthma-associated parameter may be measured at day 1, day 2, day 3, day 4, day 5, day 6, day 7, day 8, day 9, day 10, day 11, day 12, day 14, or at week 3, week 4, week 5, week 6, week 7, week 8, week 9, week 10, week 11, week 12, week 13, week 14, week 15, week 16, week 17, week 18, week 19, week 20, week 21, week 22, week 23, week 24, or longer, after the initial treatment with a pharmaceutical composition of the present invention. The difference between the value of the parameter at a particular time point following initiation of treatment and the value of the parameter at baseline is used to establish whether there has been an "improvement" in the asthma associated parameter (*e.g.*, an increase or decrease, as the case may be, depending on the specific parameter being measured).

The terms "acquire" or "acquiring" as used herein, refer to obtaining possession of a physical entity, or a value, *e.g.*, a numerical value, by "directly acquiring" or "indirectly acquiring" the physical entity or value, such as an asthma-associated parameter. "Directly acquiring" means performing a process (*e.g.*, performing a synthetic or analytical method) to obtain the physical entity or value. "Indirectly acquiring" refers to receiving the physical entity or value from another party or source (*e.g*., a third party laboratory that directly acquired the physical entity or value). Directly acquiring a physical entity includes performing a process that includes a physical change in a physical substance, *e.g.*, a starting material. Exemplary changes include making a physical entity from two or more starting materials, shearing or fragmenting a substance, separating or purifying a substance, combining two or more separate entities into a mixture, performing a chemical reaction that includes breaking or forming a covalent or non-covalent bond. Directly acquiring a value includes performing a process that includes a physical change in a sample or another substance, *e.g.*, performing an analytical process which includes a physical change in a substance, *e.g*., a sample, analyte, or reagent (sometimes referred to herein as "physical analysis").

Information that is acquired indirectly can be provided in the form of a report, *e.g*., supplied in paper or electronic form, such as from an online database or application (an "App"). The report or information can be provided by, for example, a healthcare institution, such as a hospital or clinic; or a healthcare provider, such as a doctor or nurse.

Forced Expiratory Volume in 1 Second (FEV1). According to certain embodiments of the invention, administration of an IL-4R antagonist to a patient results in an increase from baseline of forced expiratory volume in 1 second (FEV1). Methods for measuring FEV1 are known in the art. For example, a spirometer that meets the 2005 American Thoracic Society (ATS)/European Respiratory Society (ERS) recommendations can be used to measure FEV1 in a patient. The ATS/ERS Standardization of Spirometry may be used as a guideline. Spirometry is generally performed between 6 and 10 AM after an albuterol withhold of at least 6 hours. Pulmonary function tests are generally measured in the sitting position, and the highest measure is recorded for FEV1 (in liters).

The invention includes therapeutic methods that result in an increase of FEV1 from baseline of at least 0.05L at week 12 following initiation of treatment with a pharmaceutical composition comprising an anti-IL-4R antagonist. For example, according to the invention, administration of an IL-4R antagonist to a subject in need thereof causes an increase of FEV1 from baseline of about 0.05L, 0.10L, 0.12L, 0.14L, 0.16L, 0.18L, 0.20L, 0.22L, 0.24L, 0.26L, 0.28L, 0.30L, 0.32L, 0.34L, 0.36L, 0.38L, 0.40L, 0.42L, 0.44L, 0.46L, 0.48L, 0.50L, or more at week 12.

Morning and Evening Peak Expiratory Flow (AM PEF and PM PEF). According to certain embodiments of the invention, administration of an IL-4R antagonist to a patient results in an increase from baseline of morning (AM) and/or evening (PM) peak expiratory flow (AM PEF and/or PM PEF). Methods for measuring PEF are known in the art. For example, according to one method for measuring PEF, patients are issued an electronic PEF meter for recording morning (AM) and evening (PM) PEF (as well as daily albuterol use, morning and evening asthma symptom scores, and number of nighttime awakenings due to asthma symptoms that require rescue medications). Patients are instructed on the use of the device, and written instructions on the use of the electronic PEF meter are provided to the patients. In addition, a medical professional may instruct the patients on how to record pertinent variables in the electronic PEF meter. AM PEF is generally performed within 15 minutes after arising (between 6 am and 10 am) prior to taking any albuterol. PM PEF is generally performed in the evening (between 6 pm and 10 pm) prior to taking any albuterol. Subjects should try to withhold albuterol for at least 6 hours prior to measuring their PEF. Three PEF efforts are performed by the patient and all 3 values are recorded by the electronic PEF meter. Usually the highest value is used for evaluation. Baseline AM PEF may be calculated as the mean AM measurement recorded for the 7 days prior to administration of the first dose of pharmaceutical composition comprising the IL-4R antagonist, and baseline PM PEF may be calculated as the mean PM measurement recorded for the 7 days prior to administration of the first dose of pharmaceutical composition comprising the IL-4R antagonist.

The invention includes therapeutic methods that result in an increase in AM PEF and/or PM PEF from baseline of at least 1.0 L/min at week 12 following initiation of treatment with a pharmaceutical composition comprising an anti-IL-4R antagonist. For example, according to the invention, administration of an IL-4R antagonist to a subject in need thereof causes an increase in PEF from baseline of about 0.5 L/min, 1.0 L/min, 1.5 L/min, 2.0 L/min, 2.5 L/min, 3.0 L/min, 3.5 L/min, 4.0 L/min, 4.5 L/min, 5.0 L/min, 5.5 L/min, 6.0 L/min, 6.5 L/min, 7.0 L/min, 7.5 L/min, 8.0 L/min, 8.5 L/min, 9.0 L/min, 9.5 L/min, 10.0 L/min, 10.5 L/min, 11.0 L/min, 12.0 L/min, 15 L/min, 20 L/min, or more at week 12.

Albuterol/Levalbuterol Use. According to certain embodiments of the invention, administration of an IL-4R antagonist to a patient results in a decrease from baseline of daily albuterol or levalbuterol use. The number of albuterol/levalbuterol inhalations can be recorded daily by the patients in a diary, PEF meter, or other recording device. During treatment with the pharmaceutical composition of the invention, use of albuterol/levalbuterol typically may be on an as-needed basis for symptoms, not on a regular basis or prophylactically. The baseline number of albuterol/levalbuterol inhalations/day may be calculated based on the mean for the 7 days prior to administration of the first dose of pharmaceutical composition comprising the IL-4R antagonist.

The invention includes therapeutic methods that result in a decrease in albuterol/levalbuterol use from baseline of at least 0.25 puffs per day at week 12 following initiation of treatment with a pharmaceutical composition comprising an anti-IL-4R antagonist. For example, according to the invention, administration of an IL-4R antagonist to a subject in need thereof causes a decrease in albuterol/levalbuterol use from baseline of about 0.25 puffs per day, 0.50 puffs per day, 0.75 puffs per day, 1.00 puff per day, 1.25 puffs per day, 1.5 puffs per day, 1.75 puffs per day, 2.00 puffs per day, 2.25 puffs per day, 2.5 puffs per day, 2.75 puffs per day, 3.00 puffs per day, or more at week 12.

5-Item Asthma Control Questionnaire (ACQ) Score. According to certain embodiments of the invention, administration of an IL-4R antagonist to a patient results in a decrease from baseline of five-item Asthma Control Questionnaire (ACQ5) score. The ACQ5 is a validated questionnaire to evaluate asthma control.

The invention includes therapeutic methods that result in a decrease in ACQ5 score from baseline of at least 0.10 points at week 12 following initiation of treatment with a pharmaceutical composition comprising an anti-IL-4R antagonist. For example, according to the invention, administration of an IL-4R antagonist to a subject in need thereof causes a decrease in ACQ score from baseline of about 0.10 points, 0.15 points, 0.20 points, 0.25 points, 0.30 points, 0.35 points, 0.40 points, 0.45 points, 0.50 points, 0.55 points, 0.60 points, 0.65 points, 0.70 points, 0.75 points, 0.80 points, 0.85 points, or more at week 12.

Night-Time Awakenings. According to certain embodiments of the invention, administration of an IL-4R antagonist to a patient results in a decrease from baseline of average number of nighttime awakenings.

The invention includes therapeutic methods which that in a decrease in average number of nighttime awakenings from baseline of at least about 0.10 times per night at week 12 following initiation of treatment with a pharmaceutical composition comprising an anti-IL-4R antagonist. For example, according to the invention, administration of an IL-4R antagonist to a subject in need thereof causes a decrease in average number of nighttime awakenings from baseline of about 0.10 times per night, 0.15 times per night, 0.20 times per night, 0.25 times per night, 0.30 times per night, 0.35 times per night, 0.40 times per night, 0.45 times per night, 0.50 times per night, 0.55 times per night, 0.60 times per night, 0.65 times per night, 0.70 times per night, 0.75 times per night, 0.80 times per night, 0.85 times per night, 0.90 times per night, 0.95 times per night, 1.0 times per night, 2.0 times per night, or more at week 12.

22-Item Sinonasal Outcome Test (SNOT-22) Score. According to certain embodiments of the invention, administration of an IL-4R antagonist to a patient results in a decrease from baseline of 22-item Sinonasal Outcome Test (SNOT-22). The SNOT-22 is a validated questionnaire to assess the impact of chronic rhinosinusitis on quality of life (Hopkins et al 2009, Clin. Otolaryngol. 34: 447-454).

The invention includes therapeutic methods that result in a decrease in SNOT-22 score from baseline of at least 1 point at week 12 following initiation of treatment with a pharmaceutical composition comprising an anti-IL-4R antagonist. For example, according to the invention, administration of an IL-4R antagonist to a subject in need thereof causes a decrease in SNOT-22 score from baseline of about 1, 2, 3, 4, 5, 6, 7, 8, 9, 10, 11, 12, 13 points, or more at week 12.

### Methods for Treating Asthma

The invention, according to certain embodiments, provides methods for treating asthma, including, *e.g.*, eosinophilic asthma, in a subject in need thereof, wherein the methods comprise administering a pharmaceutical composition comprising an interleukin-4 receptor (IL-4R) antagonist to the subject. In certain embodiments, the methods of the invention are useful for treating moderate to severe eosinophilic asthma in a subject (*e.g.*, persistent moderate to severe eosinophilic asthma).

According to the invention, a subject is identified as having moderate to severe eosinophilic asthma if the subject exhibits a blood eosinophil level of at least 300 cells per microliter, and/or a sputum eosinophil level of at least 3%. Any methods known and available in the art for measuring blood and/or sputum eosinophil level can be used in the context of the invention to identify a subject as having moderate to severe eosinophilic asthma and who is therefore a suitable subject for the therapeutic methods of the invention.

According to a related aspect of the invention, methods for treating asthma are provided comprising: (a) selecting a patient that exhibits a blood eosinophil level of at least 300 cells per microliter and/or a sputum eosinophil level of at least 3%; and (b) administering to the patient a pharmaceutical composition comprising an IL-4R antagonist.

In another aspect, methods for reducing or eliminating an asthma patient's dependence on inhaled corticosteroids (ICS) and/or long-acting beta-agonists (LABA) during the treatment of moderate-to-severe asthma are provided. In certain embodiments, the methods comprise: selecting a patient with moderate-to-severe asthma that is uncontrolled or partially controlled with a background therapy; administering to the patient a defined dose of an IL-4R antagonist, preferably an anti-IL-4R antibody, for an initial treatment period while maintaining the patient's background therapy for the initial treatment period; and gradually reducing the dosage of one or more components of the background therapy over a subsequent period of treatment while continuing to administer the IL-4R antagonist. The term "background therapy" refers to standard or conventional therapeutic agents known in the art that are used for treating asthma. In certain embodiments, the background therapy comprises an ICS, a LABA or a combination of both. In some embodiments, the dosage of ICS and/or LABA is eliminated or completely withdrawn upon the initial treatment period. For example, a LABA, such as salmeterol or formoterol is administered in an initial treatment period and completely stopped or withdrawn in the subsequent treatment period.

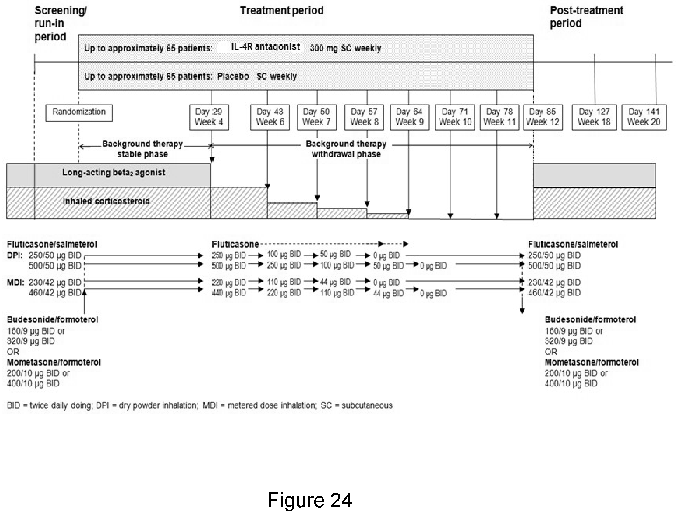

An example of a treatment regimen for a patient with moderate-to-severe asthma is shown in Figure 24, wherein an IL-4R antagonist is administered to a patient with moderate-to-severe asthma. During an initial treatment period (also called the "stable phase"), a LABA and an ICS are administered to the patient as background therapy. During a subsequent treatment period (also called "withdrawal phase"), the administration of the LABA is stopped, *i.e.*, the LABA is withdrawn or eliminated. The ICS is gradually reduced over the subsequent treatment period until it is eliminated.

In a related aspect, methods for treating asthma comprising an add-on therapy to background therapy with systematic background therapy withdrawal are provided. In certain embodiments, an IL-4R antagonist is administered as an add-on therapy to an asthma patient who is on background therapy for a certain period of time (*e.g.*, 1 week, 2 weeks, 3 weeks, 1 month, 2 months, 5 months, 12 months, 18 months, 24 months, or longer) (also called the "stable phase"). In some embodiments, the background therapy comprises a ICS and/or a LABA. The stable phase is followed by a background therapy withdrawal phase, wherein one or more components comprising the background therapy are withdrawn, or reduced or eliminated, while the add-on therapy continues. In some embodiments, the background therapy may be reduced by about 5%, about 10%, about 20%, about 30%, about 40%, about 50% or by more during the withdrawal phase. The withdrawal phase may last 1 week, 2 weeks, 3 weeks, 4 weeks, 5 weeks, 6 weeks, 7 weeks, 8 weeks, 9 weeks, 10 weeks, 11 weeks, 12 weeks, or more. In a preferred embodiment the background therapy may be reduced by about 5% during the withdrawal phase and the withdrawal phase may last 1 week, 2 weeks, 3 weeks, 4 weeks, 5 weeks, 6 weeks, 7 weeks, 8 weeks, 9 weeks, 10 weeks, 11 weeks, 12 weeks, or more. In a preferred embodiment the background therapy may be reduced by about 10% during the withdrawal phase and the withdrawal phase may last 1 week, 2 weeks, 3 weeks, 4 weeks, 5 weeks, 6 weeks, 7 weeks, 8 weeks, 9 weeks, 10 weeks, 11 weeks, 12 weeks, or more. In a preferred embodiment the background therapy may be reduced by about 20% during the withdrawal phase and the withdrawal phase may last 1 week, 2 weeks, 3 weeks, 4 weeks, 5 weeks, 6 weeks, 7 weeks, 8 weeks, 9 weeks, 10 weeks, 11 weeks, 12 weeks, or more. In a preferred embodiment the background therapy may be reduced by about 30% during the withdrawal phase and the withdrawal phase may last 1 week, 2 weeks, 3 weeks, 4 weeks, 5 weeks, 6 weeks, 7 weeks, 8 weeks, 9 weeks, 10 weeks, 11 weeks, 12 weeks, or more. In a preferred embodiment the background therapy may be reduced by about 40% during the withdrawal phase and the withdrawal phase may last 1 week, 2 weeks, 3 weeks, 4 weeks, 5 weeks, 6 weeks, 7 weeks, 8 weeks, 9 weeks, 10 weeks, 11 weeks, 12 weeks, or more. In a preferred embodiment the background therapy may be reduced by about 50% or more during the withdrawal phase and the withdrawal phase may last 1 week, 2 weeks, 3 weeks, 4 weeks, 5 weeks, 6 weeks, 7 weeks, 8 weeks, 9 weeks, 10 weeks, 11 weeks, 12 weeks, or more.

In some other embodiments, the invention encompasses methods to treat or alleviate conditions or complications associated with asthma, such as chronic rhino sinusitis, allergic rhinitis, allergic fungal rhino sinusitis, allergic broncho-pulmonary aspergillosis, unified airway disease, Churg-Strauss syndrome, vasculitis, chronic obstructive pulmonary disease (COPD), and exercise-induced bronchospasm.

The invention also includes methods for treating persistent asthma. As used herein, the term "persistent asthma" means that the subject has symptoms at least once a week at day and/or at night, with the symptoms lasting a few hours to a few days. In certain alternative embodiments, the persistent asthma is "mildly persistent" (e.g., more than twice a week but less than daily with symptoms severe enough to interfere with daily activities or sleep and/or where pulmonary function is normal or reversible with inhalation of a bronchodilator), "moderately persistent" (e.g., symptoms occurring daily with sleep interrupted at least weekly and/or with pulmonary function moderately abnormal), or "severely persistent" (e.g., continuous symptoms despite the correct use of approved medications and/or where pulmonary function is severely affected).

### Interleukin-4 Receptor Antagonists

The methods of the invention comprise administering to a subject in need thereof a therapeutic composition comprising an interleukin-4 receptor (IL-4R) antagonist. As used herein, an "IL-4R antagonist" is any agent that binds to or interacts with IL-4R and inhibits the normal biological signaling function of IL-4R when IL-4R is expressed on a cell *in vitro* or *in vivo*. Non-limiting examples of categories of IL-4R antagonists include small molecule IL-4R antagonists, anti-IL-4R aptamers, peptide-based IL-4R antagonists (*e.g.*, "peptibody" molecules), and antibodies or antigen-binding fragments of antibodies that specifically bind human IL-4R.

The term "human IL4R" (hIL-4R) refers to a human cytokine receptor that specifically binds to interleukin-4 (IL-4), such as IL-4Rα (SEQ ID NO:274).

The term "antibody" refers to immunoglobulin molecules comprising four polypeptide chains, two heavy (H) chains and two light (L) chains inter-connected by disulfide bonds, as well as multimers thereof (*e.g.*, IgM). Each heavy chain comprises a heavy chain variable region (abbreviated herein as HCVR or V_{H}) and a heavy chain constant region. The heavy chain constant region comprises three domains, C_{H}1, C_{H}2, and C_{H}3. Each light chain comprises a light chain variable region (abbreviated herein as LCVR or V_{L}) and a light chain constant region. The light chain constant region comprises one domain (C_{L}1). The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. In different embodiments, the FRs of the anti-IL-4R antibody (or antigen-binding portion thereof) may be identical to the human germline sequences, or may be naturally or artificially modified. An amino acid consensus sequence may be defined based on a side-by-side analysis of two or more CDRs.

The term "antibody" also includes antigen-binding fragments of full antibody molecules. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds to an antigen to form a complex. Antigen-binding fragments of an antibody may be derived, *e.g.*, from full antibody molecules using any suitable standard techniques, such as proteolytic digestion or recombinant genetic engineering techniques involving the manipulation and expression of DNA encoding antibody variable and optionally constant domains. Such DNA is known and/or is readily available from, *e.g.*, commercial sources, DNA libraries (including, *e.g.*, phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (*e.g.*, an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (*e.g.* monovalent nanobodies, bivalent nanobodies, etc.), small modular immunopharmaceuticals (SMIPs), and shark variable IgNAR domains, are also encompassed within the expression "antigen-binding fragment".

An antigen-binding fragment of an antibody will typically comprise at least one variable domain. The variable domain may be of any size or amino acid composition and will generally comprise at least one CDR that is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H}-V_{H}, V_{H}-V_{L} or V_{L}-V_{L} dimers. Alternatively, the antigen-binding fragment of an antibody may contain a monomeric V_{H} or V_{L} domain.

In certain embodiments, an antigen-binding fragment of an antibody may contain at least one variable domain covalently linked to at least one constant domain. Non-limiting, exemplary configurations of variable and constant domains that may be found within an antigen-binding fragment of an antibody of the present invention include: (i) V_{H}-C_{H}1; (ii) V_{H}-C_{H}2; (iii) V_{H}-C_{H}3; (iv) V_{H}-C_{H}1-C_{H}2; (v) V_{H}-C_{H}1-C_{H}2-C_{H}3; (vi) V_{H}-C_{H}2-C_{H}3; (vii) V_{H}-C_{L}; (viii) V_{L}-C_{H}1; (ix) V_{L}-C_{H}2; (x) V_{L}-C_{H}3; (xi) V_{L}-C_{H}1-C_{H}2; (xii) V_{L}-C_{H}1-C_{H}2-C_{H}3; (xiii) V_{L}-C_{H}2-C_{H}3; and (xiv) V_{L}-C_{L}. In any configuration of variable and constant domains, including any of the exemplary configurations listed above, the variable and constant domains may be either directly linked to one another or may be linked by a full or partial hinge or linker region. A hinge region may consist of at least 2 (*e.g.*, 5, 10, 15, 20, 40, 60 or more) amino acids that result in a flexible or semi-flexible linkage between adjacent variable and/or constant domains in a single polypeptide molecule, preferably the hinge region may consist of between 2 to 60 amino acids, preferably between 5 to 50, or preferably between 10 to 40 amino acids. Moreover, an antigen-binding fragment of an antibody of the present invention may comprise a homo-dimer or hetero-dimer (or other multimer) of any of the variable and constant domain configurations listed above in non-covalent association with one another and/or with one or more monomeric V_{H} or V_{L} domain (*e.g.*, by disulfide bond(s)).

As with full antibody molecules, antigen-binding fragments may be monospecific or multispecific (*e.g.*, bispecific). A multispecific antigen-binding fragment of an antibody will typically comprise at least two different variable domains, wherein each variable domain is capable of specifically binding to a separate antigen or to a different epitope on the same antigen. Any multispecific antibody format, may be adapted for use in the context of an antigen-binding fragment of an antibody of the invention using routine techniques available in the art.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

The term "human antibody" includes antibodies having variable and constant regions derived from human germline immunoglobulin sequences. The human antibodies featured in the invention may nonetheless include amino acid residues not encoded by human germline immunoglobulin sequences (e.g., mutations introduced by random or site-specific mutagenesis *in vitro* or by somatic mutation *in vivo*), for example in the CDRs and in particular CDR3. However, the term "human antibody" does not include antibodies in which CDR sequences derived from the germline of another mammalian species, such as a mouse, have been grafted onto human framework sequences.

The term "recombinant human antibody" includes all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo*.

Human antibodies can exist in two forms that are associated with hinge heterogeneity. In one form, an immunoglobulin molecule comprises a stable four chain construct of approximately 150-160 kDa in which the dimers are held together by an interchain heavy chain disulfide bond. In a second form, the dimers are not linked via inter-chain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (Angal et al. (1993) Molecular Immunology 30:105) to levels typically observed using a human IgG1 hinge. The instant invention encompasses antibodies having one or more mutations in the hinge, C_{H}2, or C_{H}3 region, which may be desirable, for example, in production, to improve the yield of the desired antibody form.

An "isolated antibody" means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody" for purposes of the present invention. An isolated antibody also includes an antibody *in situ* within a recombinant cell. Isolated antibodies are antibodies that have been subjected to at least one purification or isolation step. According to certain embodiments, an isolated antibody may be substantially free of other cellular material and/or chemicals.

The term "specifically binds," or the like, means that an antibody or antigen-binding fragment thereof forms a complex with an antigen that is relatively stable under physiologic conditions. Methods for determining whether an antibody specifically binds to an antigen are well known in the art and include, for example, equilibrium dialysis, surface plasmon resonance, and the like. For example, an antibody that "specifically binds" IL-4R, as used in the context of the present invention, includes antibodies that bind IL-4R or portion thereof with a K_{D} of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM, or less than about 0.5 nM, as measured in a surface plasmon resonance assay. An isolated antibody that specifically binds human IL-4R may, however, have cross-reactivity to other antigens, such as IL-4R molecules from other (non-human) species.

The anti-IL-4R antibodies useful for the methods of the invention may comprise one or more amino acid substitutions, insertions, and/or deletions (e.g. 1, 2, 3, 4, 5, 6, 7, 8, 9, or 10 substitutions and/or 1, 2, 3, 4, 5, 6, 7, 8, 9, or 10 insertions and/or 1, 2, 3, 4, 5, 6, 7, 8, 9, or 10 deletions) in the framework and/or CDR regions of the heavy and light chain variable domains as compared to the corresponding germline sequences from which the antibodies were derived. Such mutations can be readily ascertained by comparing the amino acid sequences disclosed herein to germline sequences available from, for example, public antibody sequence databases. The invention includes methods involving the use of antibodies, and antigen-binding fragments thereof, that are derived from any of the amino acid sequences disclosed herein, wherein one or more amino acids (e.g. 1, 2, 3, 4, 5, 6, 7, 8, 9, or 10 amino acids) within one or more framework and/or one or more (e.g. 1, 2, 3, 4, 5, 6, 7, 8, 9, 10, 11 or 12 with respect to the tetrameric antibody or 1, 2, 3, 4, 5 or 6 with respect to the HCVR and LCVR of an antibody) CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations"). A person of ordinary skill in the art, starting with the heavy and light chain variable region sequences disclosed herein, can easily produce numerous antibodies and antigen-binding fragments that comprise one or more individual germline mutations or combinations thereof. In certain embodiments, all of the framework and/or CDR residues within the V_{H} and/or V_{L} domains are mutated back to the residues found in the original germline sequence from which the antibody was derived. In other embodiments, only certain residues are mutated back to the original germline sequence, *e.g.*, only the mutated residues found within the first 8 amino acids of FR1 or within the last 8 amino acids of FR4, or only the mutated residues found within CDR1, CDR2 or CDR3. In other embodiments, one or more of the framework and/or CDR residue(s) are mutated to the corresponding residue(s) of a different germline sequence (*i.e.*, a germline sequence that is different from the germline sequence from which the antibody was originally derived). Furthermore, the antibodies of the present invention may contain any combination of two or more germline mutations within the framework and/or CDR regions, *e.g.*, wherein certain individual residues are mutated to the corresponding residue of a particular germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc. The use of antibodies and antigen-binding fragments obtained in this general manner are encompassed within the present invention.

The invention also includes methods involving the use of anti-IL-4R antibodies comprising variants of any of the HCVR, LCVR, and/or CDR amino acid sequences disclosed herein having one or more conservative substitutions. For example, the invention includes the use of anti-IL-4R antibodies having HCVR, LCVR, and/or CDR amino acid sequences with, *e.g.*, 10 or fewer, 8 or fewer, 6 or fewer, 4 or fewer, etc. conservative amino acid substitutions relative to any of the HCVR, LCVR, and/or CDR amino acid sequences disclosed herein.

The term "surface plasmon resonance" refers to an optical phenomenon that allows for the analysis of real-time interactions by detection of alterations in protein concentrations within a biosensor matrix, for example using the BIAcore^{™} system (Biacore Life Sciences division of GE Healthcare, Piscataway, NJ).

The term "K_{D}" refers to the equilibrium dissociation constant of a particular antibody-antigen interaction.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

### Preparation of Human Antibodies

Methods for generating human antibodies in transgenic mice are known in the art. Any such known methods can be used in the context of the invention to make human antibodies that specifically bind to human IL-4R.

Using VELOCIMMUNE^{™} technology (see, for example, US 6,596,541, Regeneron Pharmaceuticals) or any other known method for generating monoclonal antibodies, high affinity chimeric antibodies to IL-4R are initially isolated having a human variable region and a mouse constant region. The VELOCIMMUNE^{®} technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody.

Generally, a VELOCIMMUNE^{®} mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant region. The antibodies are characterized and selected for desirable characteristics, including affinity, selectivity, epitope, etc, using standard procedures known to those skilled in the art. The mouse constant regions are replaced with a desired human constant region to generate a fully human antibody featured in the invention, for example wild-type or modified IgG1 or IgG4. While the constant region selected may vary according to specific use, high affinity antigen-binding and target specificity characteristics reside in the variable region.

In general, the antibodies that can be used in the methods of the invention possess high affinities, as described above, when measured by binding to antigen either immobilized on solid phase or in solution phase. The mouse constant regions are replaced with desired human constant regions to generate the fully human antibodies featured in the invention. While the constant region selected may vary according to specific use, high affinity antigen-binding and target specificity characteristics reside in the variable region.

Specific examples of human antibodies or antigen-binding fragments of antibodies that specifically bind IL-4R that can be used in the context of the methods of the present invention include any antibody or antigen-binding fragment that comprises the three heavy chain CDRs (HCDR1, HCDR2 and HCDR3) contained within a heavy chain variable region (HCVR) having an amino acid sequence selected from the group consisting of SEQ ID NOs: 2, 18, 22, 26, 42, 46, 50, 66, 70, 74, 90, 94, 98, 114, 118, 122, 138, 142, 146, 162, 166, 170, 186, 190, 194, 210, 214, 218, 234, 238, 242, 258, and 262. The antibody or antigen-binding fragment may comprise the three light chain CDRs (LCVR1, LCVR2, LCVR3) contained within a light chain variable region (LCVR) having an amino acid sequence selected from the group consisting of SEQ ID NOs: 10, 20, 24, 34, 44, 48, 58, 68, 72, 82, 92, 96, 106, 116, 120, 130, 140, 144, 154, 164, 168, 178, 188, 192, 202, 212, 216, 226, 236, 240, 250, 260, and 264. Methods and techniques for identifying CDRs within HCVR and LCVR amino acid sequences are well known in the art and can be used to identify CDRs within the specified HCVR and/or LCVR amino acid sequences disclosed herein. Exemplary conventions that can be used to identify the boundaries of CDRs include, *e.g.*, the Kabat definition, the Chothia definition, and the AbM definition. In general terms, the Kabat definition is based on sequence variability, the Chothia definition is based on the location of the structural loop regions, and the AbM definition is a compromise between the Kabat and Chothia approaches. *See*, *e.g.*, Kabat, "Sequences of Proteins of Immunological Interest," National Institutes of Health, Bethesda, Md. (1991); Al-Lazikani et al., J. Mol. Biol. 273:927-948 (1997); and Martin et al., Proc. Natl. Acad. Sci. USA 86:9268-9272 (1989). Public databases are also available for identifying CDR sequences within an antibody.

In certain embodiments of the invention, the antibody or antigen-binding fragment thereof comprises the six CDRs (HCDR1, HCDR2, HCDR3, LCDR1, LCDR2 and LCDR3) from the heavy and light chain variable region amino acid sequence pairs (HCVR/LCVR) selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, and 262/264.

In certain embodiments of the invention, the antibody or antigen-binding fragment thereof comprises six CDRs (HCDR1/HCDR2/HCDR3/LCDR1/LCDR2/LCDR3) having the amino acid sequences selected from the group consisting of SEQ ID NOs: 4/6/8/12/14/16; 28/30/32/36/38/40; 52/54/56/60/62/64; 76/78/80/84/86/88; 100/102/104/108/110/112; 124/126/128/132/134/136; 148/150/152/156/158/160; 172/174/176/180/182/184; 196/198/200/204/206/208; 220/222/224/228/230/232; and 244/246/248/252/254/256.

In certain embodiments of the invention, the antibody or antigen-binding fragment thereof comprises HCVR/LCVR amino acid sequence pairs selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, and 262/264.

### Pharmaceutical Compositions

The invention includes methods that comprise administering an IL-4R antagonist to a patient, wherein the IL-4R antagonist is contained within a pharmaceutical composition. The pharmaceutical compositions featured in the invention are formulated with suitable carriers, excipients, and other agents that provide suitable transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN^{™}), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

The dose of antibody administered to a patient according to the methods of the invention may vary depending upon the age and the size of the patient, symptoms, conditions, route of administration, and the like. The preferred dose is typically calculated according to body weight or body surface area. Depending on the severity of the condition, the frequency and the duration of the treatment can be adjusted. Effective dosages and schedules for administering pharmaceutical compositions comprising anti-IL-4R antibodies may be determined empirically; for example, patient progress can be monitored by periodic assessment, and the dose adjusted accordingly. Moreover, interspecies scaling of dosages can be performed using well-known methods in the art (*e.g.*, Mordenti et al., 1991, Pharmaceut. Res. 8:1351).

Various delivery systems are known and can be used to administer the pharmaceutical compositions featured in the invention, *e.g.*, encapsulation in liposomes, microparticles, microcapsules, recombinant cells capable of expressing the mutant viruses, receptor mediated endocytosis (see, e.g., Wu et al., 1987, J. Biol. Chem. 262:4429-4432). Methods of administration include, but are not limited to, intradermal, intramuscular, intraperitoneal, intravenous, subcutaneous, intranasal, intra-tracheal, epidural, and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents.

A pharmaceutical composition of the invention can be delivered subcutaneously or intravenously with a standard needle and syringe. In addition, with respect to subcutaneous delivery, a pen delivery device readily has applications in delivering a pharmaceutical composition of the invention. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition of the invention. Examples include, but are not limited to AUTOPEN^{™} (Owen Mumford, Inc., Woodstock, UK), DISETRONIC^{™} pen (Disetronic Medical Systems, Bergdorf, Switzerland), HUMALOG MIX 75/25^{™} pen, HUMALOG^{™} pen, HUMALIN 70/30^{™} pen (Eli Lilly and Co., Indianapolis, IN), NOVOPEN^{™} I, II and III (Novo Nordisk, Copenhagen, Denmark), NOVOPEN JUNIOR^{™} (Novo Nordisk, Copenhagen, Denmark), BD^{™} pen (Becton Dickinson, Franklin Lakes, NJ), OPTIPEN^{™}, OPTIPEN PRO^{™}, OPTIPEN STARLET^{™}, and OPTICLIK^{™} (sanofi-aventis, Frankfurt, Germany), to name only a few. Examples of disposable pen delivery devices having applications in subcutaneous delivery of a pharmaceutical composition of the present invention include, but are not limited to the SOLOSTAR^{™} pen (sanofi-aventis), the FLEXPEN^{™} (Novo Nordisk), and the KWIKPEN^{™} (Eli Lilly), the SURECLICK^{™} Autoinjector (Amgen, Thousand Oaks, CA), the PENLET^{™} (Haselmeier, Stuttgart, Germany), the EPIPEN (Dey, L.P.), and the HUMIRA^{™} Pen (Abbott Labs, Abbott Park IL), to name only a few.

For direct administration to the sinuses, the pharmaceutical compositions of the invention may be administered using, *e.g.*, a microcatheter (*e.g.*, an endoscope and microcatheter), an aerosolizer, a powder dispenser, a nebulizer or an inhaler. The methods include administration of an IL-4R antagonist to a subject in need thereof, in an aerosolized formulation. For example, aerosolized antibodies to IL-4R may be administered to treat asthma in a patient. Aerosolized antibodies can be prepared as described in, for example, US8178098, incorporated herein in its entirety.

In certain situations, the pharmaceutical composition can be delivered in a controlled release system. In one embodiment, a pump may be used (see Langer, supra; Sefton, 1987, CRC Crit. Ref. Biomed. Eng. 14:201). In another embodiment, polymeric materials can be used; see, Medical Applications of Controlled Release, Langer and Wise (eds.), 1974, CRC Pres., Boca Raton, Florida. In yet another embodiment, a controlled release system can be placed in proximity of the composition's target, thus requiring only a fraction of the systemic dose (see, *e.g.*, Goodson, 1984, in Medical Applications of Controlled Release, supra, vol. 2, pp. 115-138). Other controlled release systems are discussed in the review by Langer, 1990, Science 249:1527-1533.

The injectable preparations may include dosage forms for intravenous, subcutaneous, intracutaneous and intramuscular injections, drip infusions, etc. These injectable preparations may be prepared by known methods. For example, the injectable preparations may be prepared, *e.g.*, by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc.

Exemplary pharmaceutical compositions comprising an anti-IL-4R antibody that can be used in the context of the invention are disclosed, *e.g.*, in US Patent Application Publication No. 2012/0097565.

### Dosage

The amount of IL-4R antagonist (*e.g.*, anti-IL-4R antibody) administered to a subject according to the methods of the invention is, generally, a therapeutically effective amount. As used herein, the phrase "therapeutically effective amount" means an amount of IL-4R antagonist that results in one or more of: (a) a reduction in the incidence of asthma exacerbations; (b) an improvement in one or more asthma-associated parameters (as defined elsewhere herein); and/or (c) a detectable improvement in one or more symptoms or indicia of an upper airway inflammatory condition. A "therapeutically effective amount" also includes an amount of IL-4R antagonist that inhibits, prevents, lessens, or delays the progression of asthma in a subject.

In the case of an anti-IL-4R antibody, a therapeutically effective amount can be from about 0.05 mg to about 600 mg, *e.g.*, about 0.05 mg, about 0.1 mg, about 1.0 mg, about 1.5 mg, about 2.0 mg, about 3.0 mg, about 5.0 mg, about 7.0 mg, about 10 mg, about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, or about 600 mg, of the anti-IL-4R antibody. In certain embodiments, 300 mg of an anti-IL-4R antibody is administered.

The amount of IL-4R antagonist contained within the individual doses may be expressed in terms of milligrams of antibody per kilogram of patient body weight (*i.e.*, mg/kg). For example, the IL-4R antagonist may be administered to a patient at a dose of about 0.0001 to about 10 mg/kg of patient body weight.

### Combination Therapies

The methods of the invention, according to certain embodiments, comprise administering to the subject one or more additional therapeutic agents in combination with the IL-4R antagonist. As used herein, the expression "in combination with" means that the additional therapeutic agents are administered before, after, or concurrent with the pharmaceutical composition comprising the IL-4R antagonist. In some embodiments, the term "in combination with" includes sequential or concomitant administration of an IL-4R antagonist and a second therapeutic agent. The present invention includes methods to treat asthma or an associated condition or complication or to reduce at least one exacerbation, comprising administration of an IL-4R antagonist in combination with a second therapeutic agent for additive or synergistic activity.

For example, when administered "before" the pharmaceutical composition comprising the IL-4R antagonist, the additional therapeutic agent may be administered about 72 hours, about 60 hours, about 48 hours, about 36 hours, about 24 hours, about 12 hours, about 10 hours, about 8 hours, about 6 hours, about 4 hours, about 2 hours, about 1 hour, about 30 minutes, about 15 minutes, or about 10 minutes prior to the administration of the pharmaceutical composition comprising the IL-4R antagonist. When administered "after" the pharmaceutical composition comprising the IL-4R antagonist, the additional therapeutic agent may be administered about 10 minutes, about 15 minutes, about 30 minutes, about 1 hour, about 2 hours, about 4 hours, about 6 hours, about 8 hours, about 10 hours, about 12 hours, about 24 hours, about 36 hours, about 48 hours, about 60 hours, or about 72 hours after the administration of the pharmaceutical composition comprising the IL-4R antagonist. Administration "concurrent" with the pharmaceutical composition comprising the IL-4R antagonist means that the additional therapeutic agent is administered to the subject in a separate dosage form within less than 5 minutes (before, after, or at the same time) of administration of the pharmaceutical composition comprising the IL-4R antagonist, or administered to the subject as a single combined dosage formulation comprising both the additional therapeutic agent and the IL-4R antagonist.

The additional therapeutic agent may be, *e.g.*, another IL-4R antagonist, an IL-1 antagonist (including, *e.g.*, an IL-1 antagonist as set forth in US Patent No. 6,927,044), an IL-6 antagonist, an IL-6R antagonist (including, *e.g.*, an anti-IL-6R antibody as set forth in US Patent No. 7,582,298), a TNF antagonist, an IL-8 antagonist, an IL-9 antagonist, an IL-17 antagonist, an IL-5 antagonist, an IgE antagonist, a CD48 antagonist, a leukotriene inhibitor, an anti-fungal agent, an NSAID, a long-acting beta₂ agonist (*e.g.*, salmeterol or formoterol), an inhaled corticosteroid (*e.g.*, fluticasone or budesonide), a systemic corticosteroid (e.g., oral or intravenous), methylxanthine, nedocromil sodium, cromolyn sodium, or combinations thereof. For example, in certain embodiments, the pharmaceutical composition comprising an IL-4R antagonist is administered in combination with a combination comprising a long-acting beta₂ agonist and an inhaled corticosteroid (*e.g.*, fluticasone + salmeterol [*e.g.*, Advair^{®} (GlaxoSmithKline)]; or budesonide + formoterol [*e.g.*, Symbicort^{®} (Astra Zeneca)]).

### Administration Regimens

According to certain embodiments of the invention, multiple doses of an IL-4R antagonist may be administered to a subject over a defined time course. Such methods comprise sequentially administering to a subject multiple doses of an IL-4R antagonist. As used herein, "sequentially administering" means that each dose of IL-4R antagonist is administered to the subject at a different point in time, *e.g.*, on different days separated by a predetermined interval (*e.g.*, hours, days, weeks, or months). The present invention includes methods that comprise sequentially administering to the patient a single initial dose of an IL-4R antagonist, followed by one or more secondary doses of the IL-4R antagonist, and optionally followed by one or more tertiary doses of the IL-4R antagonist.

The invention includes methods comprising administering to a subject a pharmaceutical composition comprising an IL-4R antagonist at a dosing frequency of about four times a week, twice a week, once a week, once every two weeks, once every three weeks, once every four weeks, once every five weeks, once every six weeks, once every eight weeks, once every twelve weeks, or less frequently so long as a therapeutic response is achieved. In certain embodiments involving the administration of a pharmaceutical composition comprising an anti-IL-4R antibody, once a week dosing of an amount of about 75 mg, 150 mg, or 300 mg, can be employed. In other embodiments involving the administration of a pharmaceutical composition comprising an anti-IL-4R antibody, once every two weeks dosing of an amount of about 75 mg, 150 mg, or 300 mg, can be employed. In other embodiments involving the administration of a pharmaceutical composition comprising an anti-IL-4R antibody, once every three weeks dosing of an amount of about 75 mg, 150 mg, or 300 mg, can be employed. In other embodiments involving the administration of a pharmaceutical composition comprising an anti-IL-4R antibody, once every four weeks dosing of an amount of about 75 mg, 150 mg, or 300 mg, can be employed. In other embodiments involving the administration of a pharmaceutical composition comprising an anti-IL-4R antibody, once every five weeks dosing of an amount of about 75 mg, 150 mg, or 300 mg, can be employed. In other embodiments involving the administration of a pharmaceutical composition comprising an anti-IL-4R antibody, once every six weeks dosing of an amount of about 75 mg, 150 mg, or 300 mg, can be employed. In other embodiments involving the administration of a pharmaceutical composition comprising an anti-IL-4R antibody, once every eight weeks dosing of an amount of about 75 mg, 150 mg, or 300 mg, can be employed. In other embodiments involving the administration of a pharmaceutical composition comprising an anti-IL-4R antibody, once every twelve weeks dosing of an amount of about 75 mg, 150 mg, or 300 mg, can be employed. A preferred route of administration is subcutaneous.

The term "week" or "weeks" refers to a period of (n x 7 days) ±2 days, preferably (n x 7 days) ±1 day, more preferably (n x 7 days), wherein "n" designates the number of weeks, e.g. 1, 2, 3, 4, 5, 6, 8, 12 or more.

The terms "initial dose," "secondary doses," and "tertiary doses," refer to the temporal sequence of administration of the IL-4R antagonist. Thus, the "initial dose" is the dose that is administered at the beginning of the treatment regimen (also referred to as the "baseline dose"); the "secondary doses" are the doses that are administered after the initial dose; and the "tertiary doses" are the doses that are administered after the secondary doses. The initial, secondary, and tertiary doses may all contain the same amount of IL-4R antagonist, but generally may differ from one another in terms of frequency of administration. In certain embodiments, however, the amount of IL-4R antagonist contained in the initial, secondary and/or tertiary doses varies from one another (*e.g.*, adjusted up or down as appropriate) during the course of treatment. In certain embodiments, two or more (*e.g.*, 2, 3, 4, or 5) doses are administered at the beginning of the treatment regimen as "loading doses" followed by subsequent doses that are administered on a less frequent basis (*e.g.*, "maintenance doses"). In one embodiment, the maintenance dose may be lower than the loading dose. For example, one or more loading doses of 600mg of IL-4R antagonist may be administered followed by maintenance doses of about 75mg to about 300mg.

In one exemplary embodiment of the invention, each secondary and/or tertiary dose is administered 1 to 14 (*e.g.*, 1, 1½, 2, 2½, 3, 3½, 4, 4½, 5, 5½, 6, 6½, 7, 7½, 8, 8½, 9, 9½, 10, 10½, 11, 11½, 12, 12½, 13, 13½, 14, 14½, or more) weeks after the immediately preceding dose. The phrase "the immediately preceding dose" means, in a sequence of multiple administrations, the dose of IL-4R antagonist that is administered to a patient prior to the administration of the very next dose in the sequence with no intervening doses.

The methods may comprise administering to a patient any number of secondary and/or tertiary doses of an IL-4R antagonist. For example, in certain embodiments, only a single secondary dose is administered to the patient. In other embodiments, two or more (*e.g.*, 2, 3, 4, 5, 6, 7, 8, or more) secondary doses are administered to the patient. Likewise, in certain embodiments, only a single tertiary dose is administered to the patient. In other embodiments, two or more (*e.g.*, 2, 3, 4, 5, 6, 7, 8, or more) tertiary doses are administered to the patient.

In embodiments involving multiple secondary doses, each secondary dose may be administered at the same frequency as the other secondary doses. For example, each secondary dose may be administered to the patient 1 to 2 weeks after the immediately preceding dose. Similarly, in embodiments involving multiple tertiary doses, each tertiary dose may be administered at the same frequency as the other tertiary doses. For example, each tertiary dose may be administered to the patient 2 to 4 weeks after the immediately preceding dose. Alternatively, the frequency at which the secondary and/or tertiary doses are administered to a patient can vary over the course of the treatment regimen. The frequency of administration may also be adjusted during the course of treatment by a physician depending on the needs of the individual patient following clinical examination.

The invention includes methods comprising sequential administration of an IL-4R antagonist and a second therapeutic agent, to a patient to treat asthma or an associated condition. In some embodiments, the methods comprise administering one or more doses of an IL-4R antagonist followed by one or more doses (*e.g.*, 2, 3, 4, 5, 6, 7, 8, or more) of a second therapeutic agent. For example, one or more doses of about 75mg to about 300mg of the IL-4R antagonist may be administered after which one or more doses (*e.g.*, 2, 3, 4, 5, 6, 7, 8, or more) of a second therapeutic agent (e.g., an inhaled corticosteroid or a beta2-agonist or any other therapeutic agent, as described elsewhere herein) may be administered to treat, alleviate, reduce or ameliorate one or more symptoms of asthma. In some embodiments, the IL-4R antagonist is administered at one or more doses (*e.g.*, 2, 3, 4, 5, 6, 7, 8, or more) resulting in an improvement in one or more asthma-associated parameters followed by the administration of a second therapeutic agent to prevent recurrence of at least one symptom of asthma. Alternative embodiments pertain to concomitant administration of an IL-4R antagonist and a second therapeutic agent. For example, one or more doses (*e.g.*, 2, 3, 4, 5, 6, 7, 8, or more) of an IL-4R antagonist are administered and a second therapeutic agent is administered at a separate dosage at a similar or different frequency relative to the IL-4R antagonist. In some embodiments, the second therapeutic agent is administered before, after or concurrently with the IL-4R antagonist.

### Treatment Populations

The methods of the invention comprise administering to a subject in need thereof a therapeutic composition comprising an IL-4R antagonist. The expression "a subject in need thereof" means a human or non-human animal that exhibits one or more symptoms or indicia of asthma (*e.g.*, eosinophilic asthma, including moderate to severe eosinophilic asthma), or who has been diagnosed with asthma. For example, "a subject in need thereof" may include, *e.g.*, subjects who, prior to treatment, exhibit (or have exhibited) one or more asthma-associated parameter such as, *e.g.*, impaired FEV1 (*e.g.*, less than 2.0 L), impaired AM PEF (*e.g.*, less than 400 L/min), impaired PM PEF (*e.g.*, less than 400 L/min), an ACQ5 score of at least 2.5, at least 1 nighttime awakenings per night, and/or a SNOT-22 score of at least 20. In various embodiments, the methods may be used to treat mild, moderate-to-severe, and severe asthma in patients in need thereof.

In a related embodiment, a "subject in need thereof" may be a subject who, prior to receiving an IL-4R antagonist, has been prescribed or is currently taking a combination of inhaled corticosteroid (ICS)/long-acting beta₂-adronergic antagonist (LABA). Examples of ICS/LABA therapies include fluticasone/salmeterol combination therapy and budesonide/formotorol combination therapy. For example, the invention includes methods that comprise administering an IL-4R antagonist to a patient who has been taking a regular course of ICS/LABA for two or more weeks immediately preceding the administration of the IL-4R antagonist (such prior treatments are referred to herein as "background treatments"). The invention includes therapeutic methods in which background treatments are discontinued at the time of, or just before (*e.g.*, 1 day to 2 weeks prior to) the first administration of the IL-4R antagonist. Alternatively, background treatments may be continued in combination with administration of the IL-4R antagonist. In yet other embodiments, the amount of the ICS component, the LABA component, or both, is gradually decreased prior to or after the start of IL-4R antagonist administration. In some embodiments, the invention includes methods to treat patients with persistent asthma for at least ≥ 12 months. In one embodiment, a patient with persistent asthma may be resistant to treatment by a therapeutic agent, such as a corticosteroid, and may be administered an IL-4R antagonist according to the present methods.

In some embodiments, a "subject in need thereof" may be a subject with elevated levels of an asthma-associated biomarker. Examples of asthma-associated biomarkers include, but are not limited to, IgE, thymus and activation regulated chemokine (TARC), eotaxin-3, CEA, YKL-40, and periostin. In some embodiments, a "subject in need thereof" may be a subject with blood eosinophils = 300/µl or with sputum eosinophil level ≥ 3%. In one embodiment, a "subject in need thereof" may be a subject with elevated level of bronchial or airway inflammation as measured by the fraction of exhaled nitric oxide (FeNO).

For purposes of the invention, a normal IgE level in healthy subjects is less than about 100 kU/L (*e.g.*, as measured using the ImmunoCAP^{®} assay [Phadia, Inc. Portage, MI]). Thus, the invention involves methods comprising selecting a subject who exhibits an elevated serum IgE level, which is a serum IgE level greater than about 100 kU/L, greater than about 150 kU/L, greater than about 500 kU/L, greater than about 1000 kU/L, greater than about 1500 kU/L, greater than about 2000 kU/L, greater than about 2500 kU/L, greater than about 3000 kU/L, greater than about 3500 kU/L, greater than about 4000 kU/L, greater than about 4500 kU/L, or greater than about 5000 kU/L, and administering to the subject a pharmaceutical composition comprising a therapeutically effective amount of an IL-4R antagonist.

TARC levels in healthy subjects are in the range of 106 ng/L to 431 ng/L, with a mean of about 239 ng/L. (An exemplary assay system for measuring TARC level is the TARC quantitative ELISA kit offered as Cat. No. DDN00 by R&D Systems, Minneapolis, MN.) Thus, the invention involves methods comprising selecting a subject who exhibits an elevated TARC level, which is a serum TARC level greater than about 431 ng/L, greater than about 500 ng/L, greater than about 1000 ng/L, greater than about 1500 ng/L, greater than about 2000 ng/L, greater than about 2500 ng/L, greater than about 3000 ng/L, greater than about 3500 ng/L, greater than about 4000 ng/L, greater than about 4500 ng/L, or greater than about 5000 ng/L, and administering to the subject a pharmaceutical composition comprising a therapeutically effective amount of an IL-4R antagonist.

Eotaxin-3 belongs to a group of chemokines released by airway epithelial cells, which is up regulated by the Th2 cytokines IL-4 and IL-13 (Lilly et al 1999, J. Allergy Clin. Immunol. 104: 786-790). The invention includes methods comprising administering an IL-4R antagonist to treat patients with elevated levels of eotaxin-3, such as more than about 100 pg/ml, more than about 150 pg/ml, more than about 200 pg/ml, more than about 300 pg/ml, or more than about 350 pg/ml. Serum eotaxin-3 levels may be measured, for example, by ELISA.

Periostin is an extracellular matrix protein involved in the Th2-mediated inflammatory processes. Periostin levels are found to be up regulated in patients with asthma (Jia et al 2012 J Allergy Clin Immunol. 130:647-654.e10. doi: 10.1016/j.jaci.2012.06.025. Epub 2012 Aug 1). The present invention includes methods comprising administering an IL-4R antagonist to treat patients with elevated levels of periostin.

Fractional exhaled NO (FeNO) is a biomarker of bronchial or airway inflammation. FeNO is produced by airway epithelial cells in response to inflammatory cytokines including IL-4 and IL-13 (Alwing et al 1993, Eur. Respir. J. 6: 1368-1370). FeNO levels in healthy adults range from 2 to 30 parts per billion (ppb). An exemplary assay for measuring FeNO is by using a NIOX instrument by Aerocrine AB, Solna, Sweden. The assessment may be conducted prior to spirometry and following a fast of at least an hour. The invention includes methods comprising administering an IL-4R antagonist to patients with elevated levels of exhaled NO (FeNO), such as more than about 30ppb, more than about 31 ppb, more than about 32 ppb, more than about 33ppb, more than about 34 ppb, or more than about 35ppb.

Carcinoembryogenic antigen (CEA) is a tumor marker that is found correlated to non-neoplastic diseases of the lung (Marechal et al 1988, Anticancer Res. 8: 677-680). CEA levels in serum may be measured by ELISA. The invention includes methods comprising administering an IL-4R antagonist to patients with elevated levels of CEA, such as more than about 1.0 ng/ml, more than about 1.5 ng/ml, more than about 2.0 ng/ml, more than about 2.5 ng/ml, more than about 3.0 ng/ml, more than about 4.0 ng/ml, or more than about 5.0 ng/ml.

YKL-40 [named for its N-terminal amino acids tyrosine(Y), lysine (K)and leucine (L) and its molecular mass of 40kD] is a chitinase-like protein found to be up regulated and correlated to asthma exacerbation, IgE, and eosinophils (Tang et al 2010 Eur. Respir. J. 35: 757-760). Serum YKL-40 levels are measured by, for example, ELISA. The invention includes methods comprising administering an IL-4R antagonist to patients with elevated levels of YKL-40, such as more than about 40 ng/ml, more than about 50 ng/ml, more than about 100 ng/ml, more than about 150 ng/ml, more than about 200 ng/ml, or more than about 250 ng/ml.

Induced sputum eosinophils and neutrophils are well-established direct markers of airway inflammation (Djukanovic et al 2002, Eur. Respire. J. 37: 1S-2S). Sputum is induced with inhalation of hypertonic saline solution and processed for cell counts according to methods known in the art, for example, the guidelines of European Respiratory Society. The invention includes methods comprising administering an IL-4R antagonist to patients with elevated levels of sputum eosinophils, such as more than about 2.5% or more than about 3%.

### Methods for Assessing Pharmacodynamic Asthma-Associated Parameters

The invention also includes methods for assessing one or more pharmacodynamic asthma-associated parameters a subject in need thereof, caused by administration of a pharmaceutical composition comprising an interleukin-4 receptor (IL-4R) antagonist. A reduction in the incidence of an asthma exacerbation (as described above) or an improvement in one or more asthma-associated parameters (as described above) may correlate with an improvement in one or more pharmacodynamic asthma-associated parameters; however, such a correlation is not necessarily observed in all cases.

Examples of "pharmacodynamic asthma-associated parameters" include, for example, the following: (a) biomarker expression levels; (b) serum protein and RNA analysis; (c) induced sputum eosinophils and neutrophil levels; (d) exhaled nitric oxide (FeNO); and (e) blood eosinophil count. An "improvement in a pharmacodynamic asthma-associated parameter" means, for example, a decrease from baseline of one or more biomarkers, such as TARC, eotaxin-3 or IgE, a decrease in sputum eosinophils or neutrophils, FeNO, or blood eosinophil count. As used herein, the term "baseline," with regard to a pharmacodynamic asthma-associated parameter, means the numerical value of the pharmacodynamic asthma-associated parameter for a patient prior to or at the time of administration of a pharmaceutical composition featured in the invention.

To assess a pharmacodynamic asthma-associated parameter, the parameter is quantified at baseline and at a time point after administration of the pharmaceutical composition of the present invention. For example, a pharmacodynamic asthma-associated parameter may be measured at day 1, day 2, day 3, day 4, day 5, day 6, day 7, day 8, day 9, day 10, day 11, day 12, day 14, or at week 3, week 4, week 5, week 6, week 7, week 8, week 9, week 10, week 11, week 12, week 13, week 14, week 15, week 16, week 17, week 18, week 19, week 20, week 21, week 22, week 23, week 24, or longer, after the initial treatment with a pharmaceutical composition of the present invention. The difference between the value of the parameter at a particular time point following initiation of treatment and the value of the parameter at baseline is used to establish whether there has been change, such as an "improvement", in the pharmacodynamic asthma-associated parameter (*e.g.*, an increase or decrease, as the case may be, depending on the specific parameter being measured).

In certain embodiments, administration of an IL-4R antagonist to a patient causes a change, such as a decrease or increase, in expression of a particular biomarker. Asthma associated biomarkers include the following: (a) total IgE; (b) thymus and activation-regulated chemokine (TARC); (c) YKL-40; and (d) carcinoembryonic antigen (CEA, also known as CEA cell adhesion molecule 5 [CEACAM5]) in serum and (e) eotaxin-3 in plasma. For example, administration of an IL-4R antagonist to an asthma patient can cause one or more of a decrease in TARC or eotaxin-3 levels, or a decrease in total serum IgE levels. The decrease can be detected at week 1, week 2, week 3, week 4, week 5, or longer following administration of the IL-4R antagonist. Biomarker expression can be assayed by methods known in the art. For example, protein levels can be measured by ELISA (Enzyme Linked Immunosorbent Assay), or RNA levels can be measured by reverse transcription coupled to polymerase chain reaction (RT-PCR).

Biomarker expression, as discussed above, can be assayed by detection of protein or RNA in serum. The serum samples can also be used to monitor additional protein or RNA biomarkers related to response to treatment with an IL-4R antagonist, IL-4/IL-13 signaling, asthma, atopy or eosinophilic diseases (e.g., by measuring soluble IL-4Rα, IL-4, IL-13, periostin). In some embodiments, RNA samples are used to determine RNA levels (non-genetic analysis), e.g., RNA levels of biomarkers; and in other embodiments, RNA samples are used for transcriptome sequencing (e.g., genetic analysis).

### EXAMPLES

The following examples are put forth so as to provide those of ordinary skill in the art with a complete disclosure and description of how to make and use the methods and compositions featured in the invention, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1. Generation of Human Antibodies to Human IL-4R

Human anti-hIL-4R antibodies were generated as described in US Patent No. 7,608,693. Table 1 sets forth the sequence identifiers for the heavy and light chain variable region amino acid sequence pairs, and CDR amino acid sequences, of selected anti-IL-4R antibodies and their corresponding antibody designations.

**Table 1**

| | **SEQ ID NOs:** | | | | | | | |
|---|---|---|---|---|---|---|---|---|
| **Antibody Designation** | **HCVR** | **HCDR1** | **HCDR2** | **HCDR3** | **LCVR** | **LCDR1** | **LCDR2** | **LCDR3** |
| H1H095-a | 2 | 4 | 6 | 8 | 10 | 12 | 14 | 16 |
| H1H095-b | 18 | 4 | 6 | 8 | 20 | 12 | 14 | 16 |
| H1H095-c | 22 | 4 | 6 | 8 | 24 | 12 | 14 | 16 |
| H1H097-a | 26 | 28 | 30 | 32 | 34 | 36 | 38 | 40 |
| H1H097-b | 42 | 28 | 30 | 32 | 44 | 36 | 38 | 40 |
| H1H097-c | 46 | 28 | 30 | 32 | 48 | 36 | 38 | 40 |
| H1H093-a | 50 | 52 | 54 | 56 | 58 | 60 | 62 | 64 |
| H1H093-b | 66 | 52 | 54 | 56 | 68 | 60 | 62 | 64 |
| H1H093-c | 70 | 52 | 54 | 56 | 72 | 60 | 62 | 64 |
| H1H093-d | 74 | 76 | 78 | 80 | 82 | 84 | 86 | 88 |
| H1H093-e | 90 | 76 | 78 | 80 | 92 | 84 | 86 | 88 |
| H1H093-f | 94 | 76 | 78 | 80 | 96 | 84 | 86 | 88 |
| H1H094-a | 98 | 100 | 102 | 104 | 106 | 108 | 110 | 112 |
| H1H094-b | 114 | 100 | 102 | 104 | 116 | 108 | 110 | 112 |
| H1H094-c | 118 | 100 | 102 | 104 | 120 | 108 | 110 | 112 |
| H1H096-a | 122 | 124 | 126 | 128 | 130 | 132 | 134 | 136 |
| H1H096-b | 138 | 124 | 126 | 128 | 140 | 132 | 134 | 136 |
| H1H096-c | 142 | 124 | 126 | 128 | 144 | 132 | 134 | 136 |
| H1H098-a | 146 | 148 | 150 | 152 | 154 | 156 | 158 | 160 |
| H1H098-b | 162 | 148 | 150 | 152 | 164 | 156 | 158 | 160 |
| H1H098-c | 166 | 148 | 150 | 152 | 168 | 156 | 158 | 160 |
| H1H099-a | 170 | 172 | 174 | 176 | 178 | 180 | 182 | 184 |
| H1H099-b | 186 | 172 | 174 | 176 | 188 | 180 | 182 | 184 |
| H1H099-c | 190 | 172 | 174 | 176 | 192 | 180 | 182 | 184 |
| H4H083-a | 194 | 196 | 198 | 200 | 202 | 204 | 206 | 208 |
| H4H083-b | 210 | 196 | 198 | 200 | 212 | 204 | 206 | 208 |
| H4H083-c | 214 | 196 | 198 | 200 | 216 | 204 | 206 | 208 |
| H4H121-a | 218 | 220 | 222 | 224 | 226 | 228 | 230 | 232 |
| H4H121-b | 234 | 220 | 222 | 224 | 236 | 228 | 230 | 232 |
| H4H121-c | 238 | 220 | 222 | 224 | 240 | 228 | 230 | 232 |
| H4H118-a | 242 | 244 | 246 | 248 | 250 | 252 | 254 | 256 |
| H4H118-b | 258 | 244 | 246 | 248 | 260 | 252 | 254 | 256 |
| H4H118-c | 262 | 244 | 246 | 248 | 264 | 252 | 254 | 256 |

The exemplary IL-4R antagonist used in the following Examples is the human anti-IL-4R antibody designated in Table 1 as H1H098-b (also referred to herein as "mAb1").

### Example 2: Clinical Trial of Subcutaneously Administered Anti-IL-4R Antibody (mAb1) In Patients with Persistent Moderate-to-Severe Eosinophilic Asthma, Including Asthma Patients with Chronic Hyperplastic Eosinophilic Sinusitis

### A. Study Objectives and Overview

A randomized, placebo-controlled, double-blind, parallel group study was conducted with once-a-week subcutaneous administration of either 300 mg mAb1 or placebo for 12 weeks to patients with persistent moderate-to-severe eosinophilic asthma who were partially controlled/uncontrolled by inhaled corticosteroid (ICS) and long-acting beta2 agonist (LABA) therapy. The primary objective of the study was to investigate the effects of mAb1 administered subcutaneously once weekly for 12 weeks as compared to placebo on reducing the incidence of asthma exacerbations in patients with persistent moderate-to-severe eosinophilic asthma. The secondary objectives of the study were to assess the safety and tolerability of mAb1 administered subcutaneously once weekly for 12 weeks in patients with persistent moderate to severe eosinophilic asthma, and to assess mAb1 serum concentrations following once weekly subcutaneous dosing for 12 weeks in patients with persistent moderate to severe eosinophilic asthma.

Prior to screening, patients were required to be on a stable dose of any of the following doses and formulations of ICS/LABA combination therapy (also called "background therapy") for at least 1 month:
Fluticasone/salmeterol combination therapy
- Advair^{®} Diskus - dry powder inhaler (DPI): 250/50 ug BID or 500/50 ug BID; or
- Advair^{®} HFA - metered dose inhaler (MDI): 230/42 ug BID or 460/42 ug BID; or Budesonide/formoterol combination therapy (Symbicort^{®} 160/9 ug BID or 320/9 ug BID); or Mometasone/formoterol combination therapy (Dulera^{®} 200/10 ug BID or 400/10 ug BID)

Patients who were on budesonide/formoterol or mometasone/formoterol were switched to an equivalent dose of fluticasone/salmeterol at randomization (Day 1) and patients who had been on fluticasone/salmeterol remained on the same as background therapy.

Patients who satisfied the inclusion and exclusion criteria (see below) were randomized to one of the following treatments: 300 mg of mAb1 administered subcutaneously once weekly for 12 weeks; or placebo administered subcutaneously once weekly for 12 weeks.

The study comprised a 2-week screening period, a 12-week treatment period comprising a 4-week background therapy stable phase and an 8-week background therapy withdrawal phase post-randomization, followed by an 8-week post-treatment follow-up period.

### Algorithm for background therapy (ICS/LABA) withdrawal:

Patients remained on BID fluticasone/salmeterol background therapy for 4 weeks after starting add-on therapy or treatment of 300 mg mAb1 (or placebo). At 4 weeks post-randomization, patients were switched from the BID fluticasone/salmeterol combination therapy to an equivalent ICS dose of fluticasone monotherapy (comprising either Flovent^{®} Diskus - DPI formulation of 250ug or 500 ug BID; or Flovent^{®} HFA - MDI formulation of 220 ug or 440 ug BID). The LABA component (i.e., salmeterol) was discontinued. At subsequent visits, beginning with week 6, the fluticasone dose was reduced by approximately 50%, provided the patient did not meet any of the criteria for an asthma exacerbation (as defined below). If no asthma exacerbations occurred, the ICS withdrawal proceeded according to the following dosing schedule:

| **Background therapy stable phase** | **Background therapy withdrawal phase** | | | | |
|---|---|---|---|---|---|
| | **Week 4** | **Week 6** | **Week 7** | **Week 8** | **Week 9** |
| Fluticasone/salmeterol (DPI): 250/50 µg BID | Fluticasone (DPI): 250 µg BID | 100 µg BID | 50 µg BID | 0 µg BID | 0 µg BID |
| Fluticasone/salmeterol (DPI): 500/50 µg BID | Fluticasone (DPI): 500 µg BID | 250 µg BID | 100 µg BID | 50 µg BID | 0 µg BID |
| Fluticasone/salmeterol (MDI): 230/42 µg BID | Fluticasone (MDI): 220 µg BID | 110 µg BID | 44 µg BID | 0 µg BID | 0 µg BID |
| Fluticasone/salmeterol (MDI): 460/42 µg BID | Fluticasone (MDI): 440 µg BID | 220 µg BID | 110 µg BID | 44 µg BID | 0 µg BID |

Upon completing 12 weeks of treatment with investigational product (or after early discontinuation), patients were placed on their original dose of fluticasone/salmeterol, budesonide/formoterol, or mometasone/formoterol (dose at study entry) and albuterol or levalbuterol as-needed to control their symptoms for an additional 8 weeks off study medication before a final safety evaluation.

Adult patients were included in the study based on the following criteria: (1) physician's diagnosis of persistent asthma for at least ≥ 12 months based on the Global Initiative for Asthma (GINA) 2009 Guidelines, whose airway inflammation is likely to be eosinophilic; and (2) whose asthma is partially controlled or uncontrolled in inhaled corticosteroids/long acting beta-agonists combination therapy according to the following criteria: (i) stable dose of either fluticasone/salmeterol combination therapy (DPI formulation: 250/50 µg BID or 500/50 µg BID or MDI formulation: 230/42 µg BID or 460/42 µg BID), or budesonide/formoterol combination therapy (160/9 µg BID or 320/9 µg BID), or mometasone/formoterol combination therapy (200/10 µg BID or 400/10 µg BID) for at least 1 month prior to screening; (ii) blood eosinophils ≥ 300 cells/µl or sputum eosinophils ≥ 3% during the screening phase; (iii) Juniper asthma control questionnaire (5-question version, ACQ) score of ≥ 1.5 and ≤ 3.0 at screening; (iv) FEV1 ≥ 50% predicted normal during the screening phase (3 attempts maximum) and on the randomization day prior to the first dose (3 attempts maximum); (v) has had within the 2 years prior to screening either treatment with one or more systemic (oral and/or parenteral) steroid bursts for worsening asthma or in-patient hospitalization or an emergency care visit for worsening asthma; and (vi) documented history of reversibility within 12 months of screening that meets the criterion - at least 12% and 200 mL in FEV1 after 200 µg to 400 µg (2 to 4 inhalations) of albuterol during the screening phase (3 attempts maximum), or documented history of a positive methacholine challenge (PD20 methacholine ≤ 8 mg) within 12 months prior to screening. Patients with moderate-to-severe asthma that is partially controlled or uncontrolled with moderate to high doses of combination therapy with inhaled corticosteroids and long-acting beta agonists (ADVAIR^{®}, SYMBICORT^{®} or DULERA^{®}) and with blood eosinophils greater than or equal to 300 cells per microliter, or sputum eosinophils greater than or equal to 3% during the screening phase, were included in the study.

Patients who met all the inclusion criteria were screened for the following exclusion criteria: (1) patients less than 18 years of age or greater than 65 years of age; (2) clinically relevant abnormal laboratory values suggesting an unknown disease and requiring further evaluation; (3) chronic obstructive pulmonary disease (COPD) and/or other lung diseases impairing pulmonary function tests; (4) patients requiring beta-adrenergic receptor blockers for any reason; (5) current smoker or cessation of smoking within the 6 months prior to screening; (6) previous smoking with a smoking history > 10 cigarette pack-years; (7) in-patient hospitalization or emergency care visit due to asthma exacerbation in the 2 months prior to screening; (8) plans to begin allergen immunotherapy within the study period; (9) exposure to another investigative antibody within a time period prior to screening that is less than 5 half-lives of the antibody but not less than 30 days, or if the half life of the antibody is not known, then a time period prior to screening that is at least 6 months; (10) previous enrollment into the current study; (11) patient was the investigator, his/her family member or an employee at the investigational site; (12) known or suspected non-compliance, alcohol or drug abuse; (13) inability to follow the procedures of the study (e.g., due to language problems or psychological disorders); (14) reversal of sleep pattern (e.g., night shift worker); (15) treatment with drugs known to prolong QTc interval; (16) concomitant severe disease(s) for which the use of ICS (e.g., active or inactive pulmonary tuberculosis) or LABA (e.g., diabetes, cardiovascular diseases, hypertension, hyperthyroidism, thyrotoxicosis, etc) are contra-indicated; (17) use of injectable glucocorticosteroids or oral systemic glucocorticosteroids within 2 months prior to screening or more than 3 courses within the 6 months prior to screening; (18) pre-treatment with variable doses of ICS, either alone or in combination with a non-steroidal controller (other than fluticasone/salmeterol combination therapy, budesonide/formoterol combination therapy, or mometasone/formoterol combination therapy); (19) patients receiving prohibited concomitant medications (listed below); (20) known allergy to doxycycline or related compounds; (21) pregnancy or intention to become pregnant during the course of the study, breast feeding or unwillingness to use an effective method of contraception; and (22) recent history of a parasitic infection or travel to a parasitic endemic area within 6 months prior to screening.

Patients remained on a constant dose of the background asthma therapy for the first four weeks of the study after which the dose of background therapy was reduced gradually. First, the long-acting beta agonist component of the background therapy was withdrawn at week 4, and then the inhaled corticosteroid dose was reduced by half every 2 weeks until week 12. Patients continued on study treatment until the end of the study or until they were withdrawn due to an asthma exacerbation or for any other reason.

### B. Study Treatments

Investigational Product: Sterile mAb1 150 mg/mL solution for SC injection was provided in a 5 mL glass vial. Each vial contained a withdrawable volume of 2 mL. A 300 mg dose was administered subcutaneously at the study site once weekly in the morning for 12 weeks. Placebo: Sterile placebo for SC injection was provided in an identically matched 5 mL glass vial. Each vial contained a withdrawable volume of 2 mL. Placebo was administered subcutaneously at the study site once weekly in the morning for 12 weeks.

The following concomitant medications were not allowed during the duration of the study: any other inhaled steroid other than fluticasone/salmeterol combination therapy or fluticasone administered per the protocol (or budesonide/formoterol or mometasone/formoterol during the screening period); systemic or ocular steroids; LABAs other than the salmeterol component of the fluticasone/salmeterol combination therapy administered per the protocol; any other ICS/LABA combination products other than those given above; any inhaled anti-cholinergic agents (e.g., Ipratropium bromide or tiotropium); methylxanthines (theophylline, aminophyllines); cromones; anti-IgE therapy; lipoxygenase inhibitors; and leukotriene receptor antagonists or leukotriene synthesis inhibitors.

### C. Efficacy of treatment

The primary endpoint of this study was the occurrence of an exacerbation of asthma as defined by any of the following: (1) A 30% or greater reduction from baseline in morning peak expiratory flow (PEF) on two consecutive days; or (2) Six or more additional reliever puffs of albuterol or levalbuterol in a 24 hour period (compared to baseline) on 2 consecutive days; or (3) Deterioration of asthma, as determined by the Investigator, requiring: (a) systemic (oral and/or parenteral) steroid treatment, or (b) An increase in ICS ≥4 times the last dose received prior to discontinuation from the study, or (c) Hospitalization.

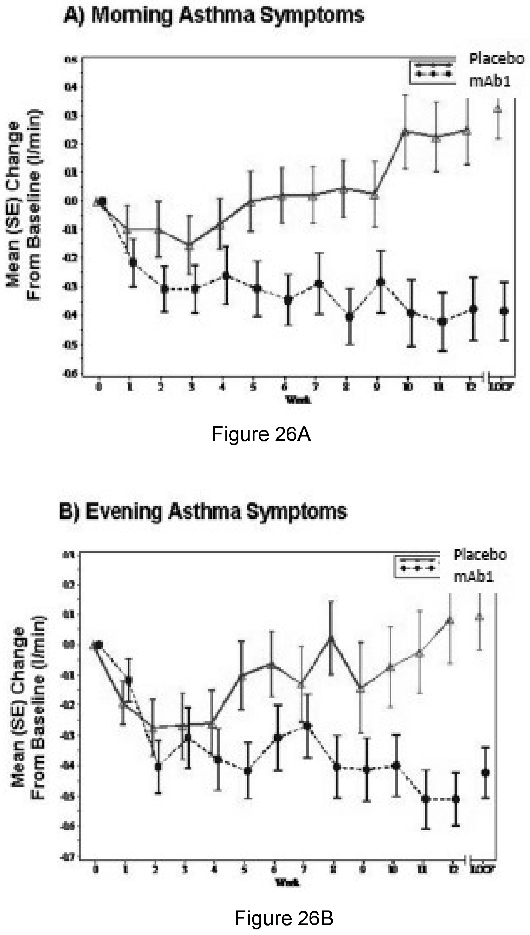

Secondary endpoints of the study included mean changes from baseline of the following parameters: (1) Forced expiratory volume in 1 second (FEV1) in liters measured at every visit; (2) Morning and evening peak expiratory flow rate (AM PEF and PM PEF) in liters/minute measured daily; (3) Daily Albuterol/Levalbuteral use in inhalations/day; (4) Five-item Asthma Control Questionnaire (ACQ5) score at every visit; and (5) Nighttime awakenings (no. of times per night) measured daily and (6) a 22-item Sino-Nasal Outcome Test (SNOT-22), evaluated at baseline and end of treatment (at Week 12), to assess upper airway symptoms. Secondary endpoints also included proportion of patients with a composite asthma event defined by a 30% or greater reduction from baseline in morning PEF on two consecutive days together with ≥ 6 additional reliever puffs of albuterol or levalbuterol in a 24-hour period compared to baseline) on 2 consecutive days. PEF, ACQ5, asthma symptoms scores, nocturnal awakenings, and reliever medication use were captured in an electronic daily diary. Mean daily nocturnal awakenings, ranging from 0-10, were averaged from the previous 7 days. Morning and evening asthma symptom scores consisted of a non-validated patient-reported outcome assessed on a 5-point Likert-type scale, with higher scores indicating worse outcomes (Table 2). Patients recorded overall symptom scores twice a day prior to measuring PEF. Data are described as the average for the 7 days prior to the specified time point (see, *e.g.*, Figures 26A and 26B).

**Table 2: Asthma Symptom Score Assessment**

| A) Morning symptom score: | |
|---|---|
| | 0 = No asthma symptoms, slept through the night |
| | 1 = Slept well, but some complaints in the morning. No nighttime awakenings |
| | 2 = Woke up once because of asthma (including early awakening) |
| | 3 = Woke up several times because of asthma (including early awakening) |
| | 4 = Bad night, awake most of the night because of asthma |

| B) Evening symptom score: | |
|---|---|
| | 0 = Very well, no asthma symptoms |
| | 1 = One episode of wheezing, cough, or breathlessness |
| | 2 = More than one episode of wheezing, cough, or breathlessness without interference of normal activities |
| | 3 = Wheezing, cough, or breathlessness most of the day, which interfered to some extent with normal activities |
| | 4 = Asthma very bad. Unable to carry out daily activities as usual |

### D. Adverse Events Monitoring

Safety was assessed throughout the study by monitoring Adverse Events and Serious Adverse Events.

An Adverse Event (AE) is any untoward medical occurrence in a subject or clinical investigation subject administered a pharmaceutical product. An AE can, therefore, be any unfavorable and unintended sign (including abnormal laboratory finding), symptom, or disease temporally associated with the use of a medicinal product, whether or not considered related to the medicinal (investigational) product. AEs also include: any worsening (i.e., any clinically significant change in frequency and/or intensity) of a pre-existing condition that is temporally associated with the use of the study drug; abnormal laboratory findings considered by the Investigator to be clinically significant; and any untoward medical occurrence.

A Serious Adverse Event (SAE) is any untoward medical occurrence that at any dose results in death; is life-threatening; requires in-patient hospitalization or prolongation of existing hospitalization; results in persistent or significant disability/ incapacity; is a congenital anomaly/birth defect; or is an important medical event.

### E. Statistical methods

For the primary analysis of proportion of patients experiencing an asthma exacerbation, a logistic regression model was used to compare the SAR group with placebo. The model included terms for treatment and stratification factor (prior ICS/LABA combination therapy dose). The primary analysis was performed based on modified intent-to-treat (mITT) population, which included all randomized patients who received at least one dose of investigational medicinal product (IMP). A stratified chi-square test was also used to corroborate the primary analysis.

For secondary efficacy endpoints, except SNOT-22, the change from baseline was analyzed using a mixed-effect model with repeated measures (MMRM) approach. The model included change from baseline values up to week 12 as response variables, and factors (fixed effects) for treatment, stratification factor, visit, treatment-by-visit interaction, baseline value, and baseline-by-visit interaction. Statistical inferences on treatment comparisons for the change from baseline at week 12 were derived from the mixed-effect model. Change from baseline in SNOT-22 was analyzed using an analysis of covariance (ANCOVA), with end of treatment measurements used to impute missing data. Pharmacodynamic effects were evaluated using MMRM models in a *post hoc* fashion. No adjustments were made for multiplicity, since there was only one primary endpoint and analysis. Safety variables including AEs, laboratory parameter, vital signs, ECG, clinical laboratory observations and physical examinations were summarized using descriptive statistics.

Demographic and clinical characteristics were summarized using descriptive characteristics. Plots of secondary and pharmacodynamic variables are presented as mean change from baseline over time with standard error. Comparison of treatment effects from the MMRM analyses are based on least square mean change (95% confidence intervals [CI]) from baseline at Week 12.

### F. Results

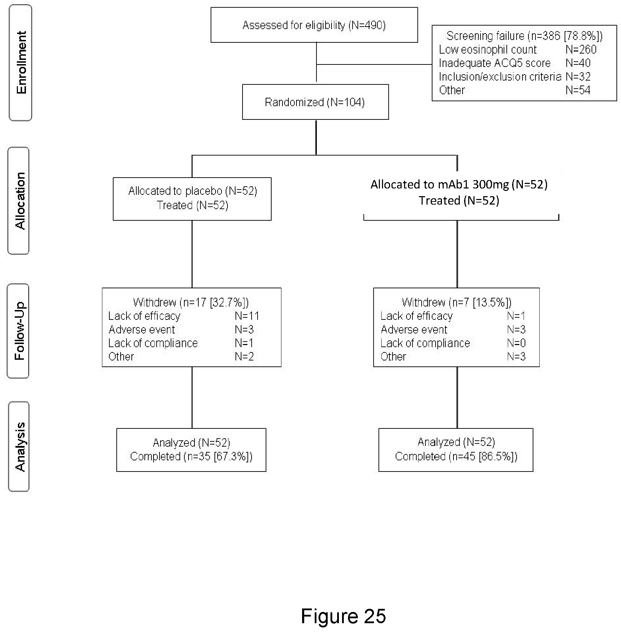

The results observed with all 104 randomized patients (from 491 screened) who either completed or discontinued the treatment phase of the study are summarized below. All randomized patients were exposed to study treatment and included in the mITT population. Baseline characteristics were similar between groups. The demographic and clinical characteristics were also similar between the two groups (Table 3). As noted above, patients were treated either with 300 mg subcutaneous mAb1 once a week, or with placebo. The study treatment period was completed by 86.5% and 67.3% of the mAb1 and placebo patients, respectively (Figure 25). The most common cause of discontinuation was lack of efficacy, which was more frequent with placebo (21.2%) than with mAb1 (1.9%).

**Table 3. Baseline Demographic and Clinical Characteristics of Treatment Groups.***

| **Variable** | | **Placebo (N = 52)** | **mAb1 300 mg (N = 52)** |
|---|---|---|---|
| Age (yr) | | 41.6 ± 13.1 | 37.8 ± 13.2 |

| **Variable** | | **Placebo (N = 52)** | **mAb1 300 mg (N = 52)** |
|---|---|---|---|
| Male sex, no. (%) | | 26 (50.0) | 26 (50.0) |
| Race or ethnic group, no. (%) | | | |
| | White | 38 (73.1) | 45 (86.5) |
| | Black or African American | 9 (17.3) | 5 (9.6) |
| | Asian | 3 (5.8) | 1 (1.9) |
| | Other | 2 (3.8) | 1 (1.9) |
| Body mass index | | | |
| | Mean (kg/m²) | 31.6 ± 7.0 | 31.3 ± 8.0 |
| | | 25 (48.1) | 24 (46.2) |
| Duration of asthma (yr) | | 26.9 ± 14.8 | 24.2 ± 12.6 |
| Number of asthma exacerbations in prior 2 years | | 1.4 ± 1.3 | 1.4 ± 1.0 |
| Prior ICS/LABA combination therapy dose, no. (%) | | | |
| | High Dose | 41 (78.8) | 42 (80.8) |
| | Low Dose | 11 (21.2) | 10 (19.2) |
| Blood eosinophils (x10⁻⁹/l) | | 0.47 ± 0.21 | 0.55 ± 0.19 |
| FEV₁ (l) | | 2.54 ± 0.66 | 2.47 ± 0.65 |
| FEV₁ (% of predicted value) | | 72.0 ± 12.7 | 72.0 ± 12.6 |
| PEF (l/min) | | | |
| | Morning | 406.9 ± 110.7 | 393.0 ± 101.1 |
| | Evening | 416.6 ± 116.8 | 414.6 ± 102.3 |
| ACQ5 score | | 2.1 ± 0.5 | 2.1 ± 0.5 |
| Asthma symptom score | | | |
| | Morning | 0.73 ± 0.63 | 0.75 ± 0.81 |
| | Evening | 1.12 ± 0.73 | 0.92 ± 0.71 |
| Nocturnal awakenings per day | | 0.21 ± 0.50 | 0.44 ± 0.80 |
| SNOT-22 | | 26.2 ± 15.6 | 30.9 ± 14.8 |
| Inhalations of albuterol or levalbuterol/24-hour period | | 2.0 ± 1.8 | 2.2 ± 2.4 |
| FeNO (ppb) | | 35.0 ± 27.1 | 37.6 ± 28.1 |
| TARC (pg/ml) | | 470.5 ± 204.7 | 496.1 ± 342.4 |
| Eotaxin-3 (pg/ml) | | 117.3 ± 349.2 | 75.4 ± 44.0 |
| IgE (IU/ml) | | 694.7 ± 1837.8 | 657.7 ± 1482.3 |

| | | | |
|---|---|---|---|
| *Plus-minus values are means ± SD, except as otherwise noted. ACQ5 denotes the Asthma Control Questionnaire (5 question version), FeNO fraction of exhaled nitric oxide, FEV₁ forced expiratory volume in 1 second, IgE immunoglobulin E, PEF peak expiratory volume, SNOT-22 the 22-item Sinonasal Outcome Test, and TARC thymus and activation regulated chemokine. | | | |

### (i) Primary Efficacy Endpoint

The incidence of asthma exacerbations in the placebo and mAb1 treatment groups is presented in Table 4.

**Table 4: Incidence of Asthma Exacerbations in mITT population**

| | **Placebo (N=52)** | **mAb1 (N=52)** |
|---|---|---|
| Patients With No Asthma Exacerbations | 29 (55.8%) | 49 (94.2%) |
| Patients With Asthma Exacerbations | 23 (44.2%) | 3 (5.8%) |
| Odds Ratio vs Placebo (95% CI) | -- | 0.077 (0.021, 0.279) |

There were a total of 26 asthma exacerbations during the treatment period, and no patients were hospitalized for asthma exacerbations. There were 23 patients (44.2%) who experienced an asthma exacerbation in the placebo group, whereas only 3 patients (5.8%) experienced an asthma exacerbation in the mAb1 treatment group. The odds ratio is 0.077 (p <0.0001) and the relative risk reduction is approximately 87%.

Out of the 26 asthma exacerbations experienced during this study, 9 were considered severe, as demonstrated by a need for immediate intervention in the form of treatment with either systemic corticosteroids or with inhaled corticosteroids at 4 or more times the dose taken prior to the event. A summary of the incidence of severe asthma exacerbations is presented in Table 5.

**Table 5: Incidence of Severe Asthma Exacerbations in mITT population**

| | **Placebo (N=52)** | **mAb1 (N=52)** |
|---|---|---|
| Patients With No Asthma Exacerbations | 29 (55.8%) | 49 (94.2%) |
| Patients With Severe Asthma Exacerbations | 8 (15.4%) | 1 (1.9%) |
| Patients With Non-Severe Asthma Exacerbations | 15 (28.8%) | 2 (3.8%) |

As shown in Table 5, eight severe asthma exacerbations were observed in the placebo group, and only 1 severe asthma exacerbation was observed in the mAb1 treatment group. The remaining 15 asthma exacerbations in the placebo group and 2 in the mAb1 group met the protocol definition of exacerbation based on decreased morning PEF and/or increased albuterol/levalbuterol use. As shown in Table 6, within the active treatment group, a sustained improvement versus baseline was observed during the course of the study for all parameters, despite steroid withdrawal.

**Table 6. Exacerbation Events**

| **Outcome** | **Placebo (N = 52)** | **mAb1 ( N = 52)** | |
|---|---|---|---|
| baseline in morning PEF in a 24-hr period on 2 consecutive days | 10* (19.2) | 1 (1.9) | |
| ≥ 6 additional inhalations of albuterol/levalbuterol in a 24-hr period on 2 consecutive days | | 10 (19.2) | 1 (1.9) |
| | Systemic steroid treatment | 5 (9.6) | 1 (1.9) |
| ≥ 4-fold increase in ICS from the previous dose | | 3 (5.8) | 0 |
| Hospitalization | | 0 | 0 |

| | | | |
|---|---|---|---|
| *4 Placebo patients met both PEF and systemic steroid treatment criteria, and 1 placebo patient met both PEF and additional albuterol/levalbuterol use. | | | |

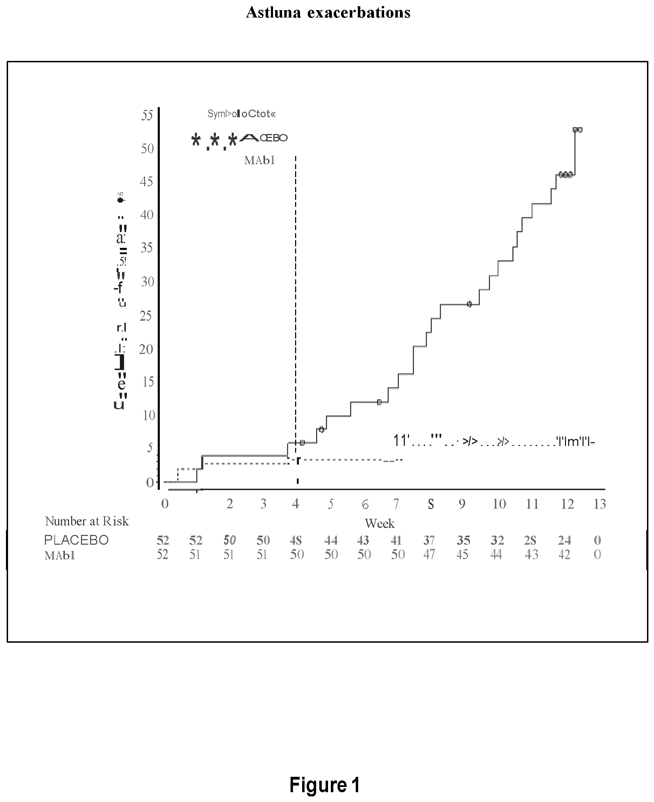

With mAb1, the time to exacerbation was longer (Figure 1), and the risk of exacerbation was reduced relative to placebo (hazard ration 0,10; 95% CI 0.03, 0.34; P< 0.001). An analysis of the time to asthma exacerbation by Kaplan-Meier Plot revealed that the effect of treatment with mAb1 is sustained over time, including after 8 weeks when patients are at higher risk of developing exacerbations due to steroid withdrawal (Figure 1).

Only 1 patient from the placebo group had a composite asthma event. A composite asthma event is defined as a 30% or greater reduction from baseline in morning PEF on 2 consecutive days together with ≥6 additional reliever puffs of albuterol or levalbuterol in a 24-hour period (compared to baseline) on 2 consecutive days.

### (ii) Other Efficacy Endpoints

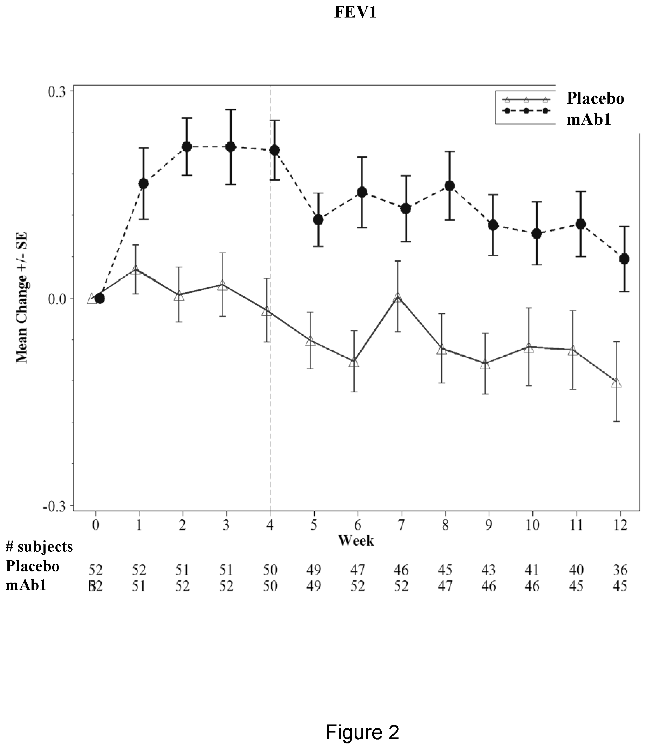

Lung function parameters (FEV1, AM PEF and PM PEF), asthma symptom-based endpoints (ACQ score, nighttime awakenings), and albuterol use were assessed for each patient at each visit. Results observed for these parameters (weekly change from baseline) are depicted in Figures 2-7, respectively. In addition, the SNOT-22 score was assessed at baseline and at the end of treatment. For all parameters, the baseline and Week 12 (LOCF) mean values along with the mean difference between treatment groups (ANOVA model for SNOT-22) are summarized in Table 7. In Table 7, the column labeled "Difference vs. Placebo" reflects the placebo-corrected value from baseline that takes into account changes that are observed in the value of the parameter as compared to the changes that were observed for that parameter in the placebo-treated group.

**Table 7: Secondary Parameters of Lung Function and Symptom Scores**

| | **N** | **Baseline Mean (SD)** | **Least-Squared Mean Change (SD)** | **Difference vs. Placebo** | **p value** |
|---|---|---|---|---|---|
| **FEV1 (L)** | | | | | |
| Placebo | 52 | 2.54 (0.66) | -0.22 (0.06) | -- | |
| mAb1 | 52 | 2.47 (0.65) | 0.05 (0.06) | 0.27 (0.11, 0.42) | 0.0009 |

| **AM PEF (L/min)** | | | | | |
|---|---|---|---|---|---|
| Placebo | 52 | 406.9 (110.7) | -20.7 (9.1) | -- | |
| mAb1 | 51 | 393.0 (101.1) | 13.9 (8.8)† | 34.6 (10.6, 58.5) | 0.0051 |

| **PM PEF (L/min)** | | | | | |
|---|---|---|---|---|---|
| Placebo | 51 | 416.6 (116.8) | -18.4 (8.9)† | -- | |
| mAb1 | 52 | 414.6 (102.3) | 4.3 (8.5) | 22.7 (-0.7, 46.0) | 0.0567 |

| **Albuterol Use (Puffs/Day)** | | | | | |
|---|---|---|---|---|---|
| Placebo | 52 | 2.0 (1.8) | 0.7 (0.3) | -- | |
| mAb1 | 50 | 2.2 (2.4) | -1.3 (0.3)‡ | -2.0 (-2.9, -1.2) | <0.0001 |

| **ACQ Score** | | | | | |
|---|---|---|---|---|---|
| Placebo | 52 | 2.08 (0.52) | -0.27 (0.16) | -- | |
| mAb1 | 52 | 2.09 (0.46) | -1.00 (0.16) | -0.73 (-1.15, -0.30) | 0.0011 |

| **Night-time Awakenings (No. of times/night)** | | | | | |
|---|---|---|---|---|---|
| Placebo | 52 | 0.2 (0.5) | 0.1 (0.1) | -- | |
| mAb1 | 52 | 0.4 (0.8) | -0.2 (0.1) | -0.2 (-0.5, -0.0) | 0.0518 |

| **SNOT22 Average Score** | | | | | |
|---|---|---|---|---|---|
| | **N** | **Baseline Mean (SD)** | **Least-Squared Mean Change (SD)** | **Difference vs. Placebo** | **p value** |
| Placebo | 51 | 26.24 (15.62) | 0.23 (2.15)† | -- | |
| mAb1 | 50 | 30.92 (14.77) | -8.26 (2.20)‡ | -8.49 (-13.96, -3.03) | 0.0027 |

| | | | | | |
|---|---|---|---|---|---|
| † 51 patients with at least 1 post-baseline assessment. ‡ 50 patients with at least 1 post-baseline assessment. | | | | | |

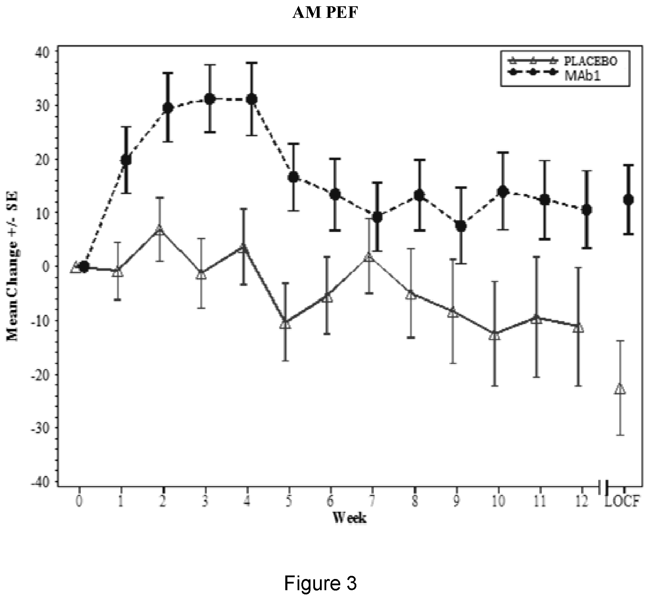

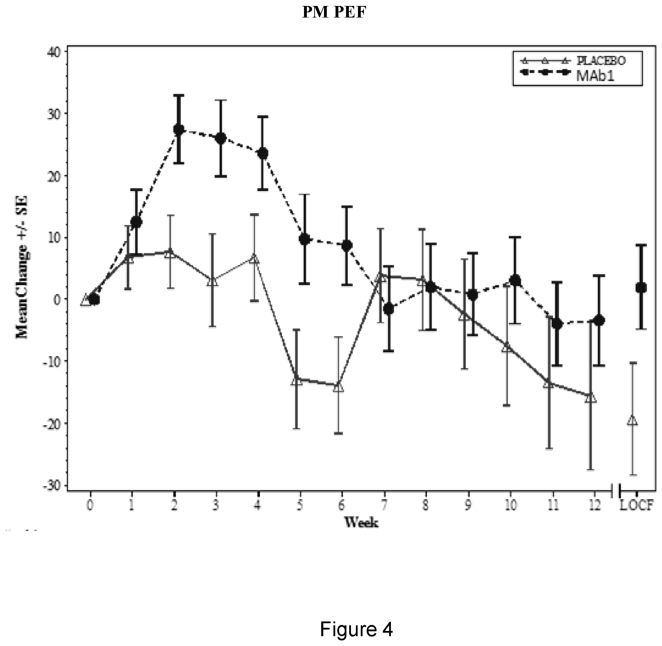

Treatment with mAb1 resulted in a significant change from baseline in FEV1 at Week 1, which was maintained through Week 12 (Figure 2) despite LABA and ICS withdrawal, with a small decrease in FEV1 at Week 5 coinciding with LABA withdrawal. Similar improvements were observed in morning PEF, but less so in evening PEF (Figures 3 and 4). The least-squared (LS) mean change from baseline to week 12 in FEV1 was -0.22 L for placebo and 0.05 L for the mAb1 group. (p=0.0009).

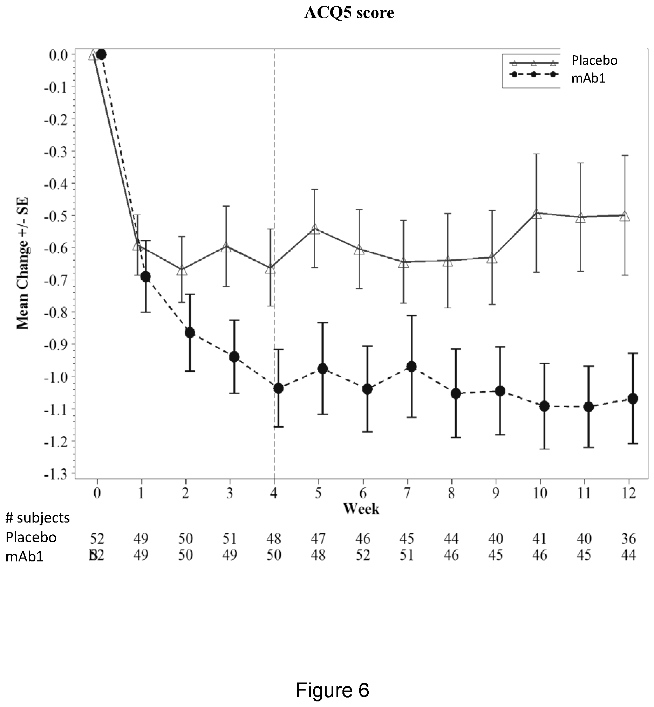

ACQ5 score improved in both treatment groups at Week 1 (Figure 6). However, while ACQ5 improved further with mAb1 between Weeks 1 and 4, the placebo effect stabilized, maintaining the difference through Week 12.

Morning symptom scores increased from baseline to Week 12 with placebo. With mAb1, there was an initial decrease that remained below baseline through Week 12 (Figure 26A). A similar pattern (with greater variability) was observed for evening asthma symptom scores (Figure 26B).

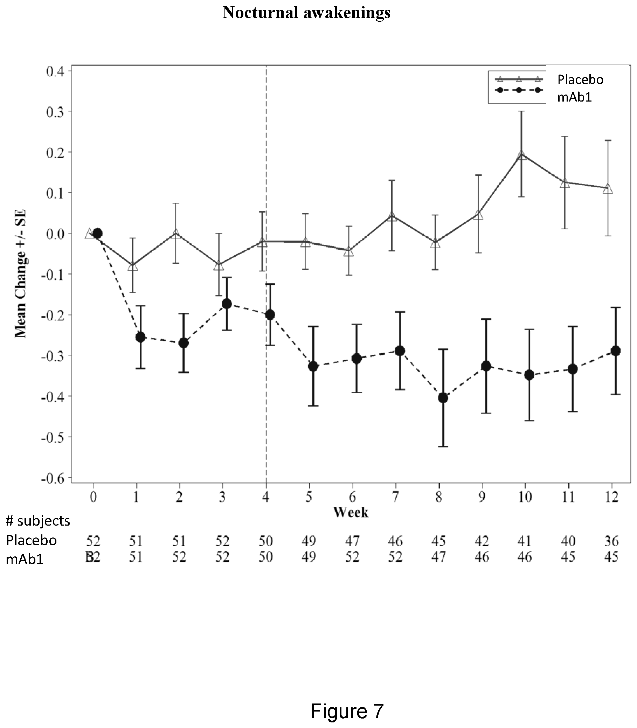

Nocturnal awakenings were stable from the placebo group through Week 6, then increased from Weeks 6 to 12. In contrast, nocturnal awakenings decreased in the mAb1 group by Week 1 and remained improved versus baseline through Week 12 (Figure 7).

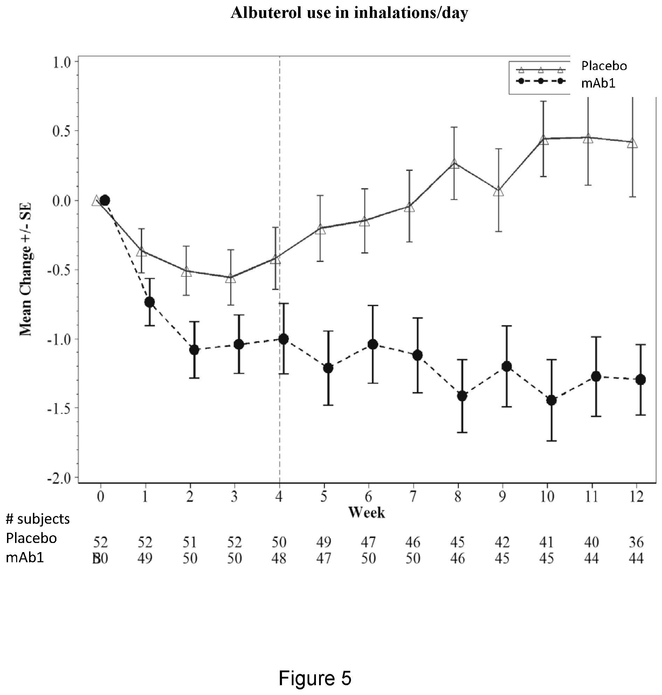

Changes in albuterol/levalbuterol use (Figure 5) were similar to other secondary endpoints: an initial decrease followed by a return towards baseline with placebo. With mAb1, the initial decrease was maintained over time.

There was a non-significant difference at baseline between the SNOT-22 values, with the mean placebo score at 26.24 and the mean mAb1 score at 39.02. At week 12, the LS mean change was a slight increase of 0.23 points for the placebo group and a mean decrease (improvement) of 8.26 points for the mAb1 group. This represented a magnitude of improvement of 8.49 points for the mAb1 group (p=0.0027).

**Table 8. Secondary Endpoints**

| **Outcome** | **Placebo (N = 52)** | **mAb1 ( N = 52)** | **Difference vs Placebo (95% CI)**** | **P Value** |
|---|---|---|---|---|
| Kaplan-Meier estimate at 12 weeks | 46.0 (31.8, 60.2) | 5.8 (0.0, 2.1) | 0.10 (0.03 to 0.34) | <0.001 |
| Change in morning asthma symptom scores, baseline to week 12 | 0.3 ± 0.1 | -0.4 ± 0.1 | -0.7 (-0.9 to -0.4) | <0.001 |
| Change in evening asthma symptom scores, baseline to week 12 | 0.1 ± 0.1 | -0.6 ± 0.1 | -0.7 (-0.9 to -0.4) | <0.001 |

**Table 9. Change From Baseline at Week 12 in SNOT-22 Items Relevant to Upper Airway Disease.**

| **SNOT-22 Subscale** | **Least-Squares Mean Change ± Standard Error** | | **Difference vs Placebo (95% CI)** | **P Value** |
|---|---|---|---|---|
| | **Placebo (N = 52)** | **mAb1 ( N = 52)** | | |
| Need to blow nose | -0.25 ± 0.17* | 0.95 ± 0.17† | -0.70 (-1.13, -0.26) | 0.002 |
| Nasal blockage | -0.20 ± 0.19* | -0.94 ± 0.19† | 0.75 (-1.22, -0.28) | 0.002 |
| Decreased sense of smell/taste | 0.04 ± 0.18* | -1.13 ± 0.18† | -1.16 (-1.62, -0.71) | < 0.001 |

| | | | | |
|---|---|---|---|---|
| *51and †50 patients with at least 1 post-baseline assessment respectively | | | | |

For all secondary endpoints, Week 12 measurements favored mAb1 treatment and were significant, except for evening PEF and nocturnal awakenings (Table 7 and 8). Significant improvements with mAb1 were also observed for the three SNOT-22 items relevant to upper airway disease (Table 9)

### (iii) Safety

mAb1 was generally safe and well tolerated. Treatment-emergent adverse events (TEAEs) were reported similarly by 40 (76.9%) placebo-treated patients and by 42 (80.8%) mAb1-treated patients (Table 10). TEAEs were non-specific, generally mild to moderate in intensity, and the majority recovered by the end of the study. An increased reporting of the following TEAEs was observed for mAb1 in comparison with placebo: injection site reactions were reported by 15 (28.8%) mAb1 patients and by 5 (9.6%) placebo patients; nasopharyngitis was reported by 7 (13.5%) mAb1 patients and 2 (3.8%) placebo patients; headache was reported by 6 (11.5%) mAb1 patients and 3 (5.85) placebo patients and nausea was reported by 4 (7.7%) mAb1 patients and 1 (1.9%) placebo patients.

**Table 10. Adverse Events.**

| **Adverse event** | | **Placebo (N = 52)** | **mAb1 300 mg (N = 52)** |
|---|---|---|---|
| | | *no. of patients (%)* | |
| Any adverse event | | 40 (76.9) | 42 (80.8) |
| Any serious adverse event | | 3 (5.8) | 1 (1.9) |
| Study discontinuation owing to adverse event | | 3 (5.8) | 3 (5.8) |
| Death | | 0 | 0 |
| Most common AEs* | | | |
| | Injection site reactions† | 5 (9.6) | 15 (28.8) |
| | Nasopharyngitis | 2 (3.8) | 7 (13.5) |
| | Upper respiratory tract infection | 9 (17.3) | 7 (13.5) |
| | Headache | 3 (5.8) | 6 (11.5) |
| | Nausea | 1 (1.9) | 4 (7.7) |
| | Arthropod bite | 0 | 3 (5.8) |
| | Muscle spasms | 0 | 3 (5.8) |
| | Nasal congestion | 1 (1.9) | 3 (5.8) |
| | Rash | 1 (1.9) | 3 (5.8) |
| | Urticaria | 0 | 3 (5.8) |
| | Viral upper respiratory tract infection | 0 | 3 (5.8) |

| | | | |
|---|---|---|---|
| *≥ 3 patients in any treatment group by Preferred Term † Injection site reaction includes events reported as: injection site pain, injection site reaction, injection site erythema, injection site rash, injection site haematoma, injection site urticaria, injection site dermatitis, injection sites inflammation, injection site nodule, injection site pruritus and injection site swelling. | | | |

There were no deaths reported during the study period. Of the 4 treatment emergent serious adverse events (SAEs) reported: 1 mAb1 patient experienced bipolar disorder and 3 placebo patients experienced SAEs of asthma with pneumonia, gunshot wound with left pneumothorax, and right ankle fracture. None of these SAEs were considered as related to the IMP and all but the recent ankle fracture were recovered by the end of the study. There were no deaths.

A total of 6 patients discontinued the study due to a TEAE: 3 patients in the mAb1 group (bipolar disorder, asthma with wheezing, and angioedema) and 3 patients in the placebo group (upper respiratory tract infection, psoriasis and asthma). The TEAE of angioedema occurred in a 42-year old African-American female after the ninth study treatment dose as a pruritic, popular rash observed at, and distant to, the injection site. It persisted for one week, resolved after study treatment discontinuation, and prednisome and diphenhydramine treatment. It was deemed treatment-related. This AE was subsequent to milder rashes at the injection site after the first and sixth study treatment doses.

Among the most common AEs occurring in ≥3 patients in any treatment group (Table 10), injection site reactions, nasopharyngitis, nausea, and headache occurred more frequently with mAb1 than placebo. No clinically significant changes in vital signs, physical examination, clinical laboratory or ECG findings were reported in either group.

### G. Conclusion

Significant improvements were observed for lung function and other asthma control parameters. Efficacy was observed early and sustained despite background therapy withdrawal. A relative reduction of approximately 87% (p <0.0001) in the primary endpoint of the incidence of asthma exacerbations in persistent, moderate-to-severe asthma patients with eosinophilia was observed after 12-week treatment with 300mg of mAb1 once weekly (5.8%) compared with placebo (44.2%). As shown in Table 7, clinically meaningful and statistically significant (without multiplicity adjustment) improvements with treatment compared with placebo were observed in lung function parameters (FEV1, PEF AM), asthma symptom scores (ACQ) and albuterol use. Positive trends were observed for PEF PM (p=0.0567) and nocturnal awakenings (p=0.0518). A statistically significant (without multiplicity adjustment) improvement was also observed for the SNOT-22 score. Within the active treatment group, a sustained improvement versus baseline was observed during the course of study for all parameters, despite LABA and ICS withdrawal. mAb1 was generally safe and well tolerated.

### Example 3: Biomarker studies

Biomarker analysis was conducted on samples taken from subjects who participated in clinical trials of mAb1 (see Example 2 above). In particular, serum/plasma biomarkers associated with TH2 inflammation, such as thymus and activation chemokine (TARC; CCL17), Immunoglobulin E (IgE), eotaxin-3, periostin, carcinoembryonic antigen (CEA), YKL-40 and blood eosinophils were measured in samples from patients at baseline and at different time points following initiation of study treatment(s). Baseline levels of these biomarkers were assessed for potential predictive value for treatment response. In addition, the fraction of exhaled NO (FeNO) and induced sputum eosinophils and neutrophils were measured as biomarkers of bronchial inflammation. Exhaled nitric oxide assessment was conducted prior to spirometry and following a fast of at least 1 hour using a NIOX instrument (Aerocrine AB, Solna, Sweden). Biomarkers were analyzed using a mixed model and the least square mean derived from the model are reported below.

Asthma subjects (N=104) were administered either mAb1 (300 mg) or placebo subcutaneously, on days 1, 8, 15, 22, 29, 36, 43, 50, 57, 64, 71, and 78 of the study (*i.e.*, 12 weekly doses) (*see* Example 2 herein). Samples for biomarker analysis were collected from the antibody- and placebo-treated subjects at week 0, 1, 4, 8 and 12. Antigen-specific IgE was detected using the Phadiatop^{®} test.

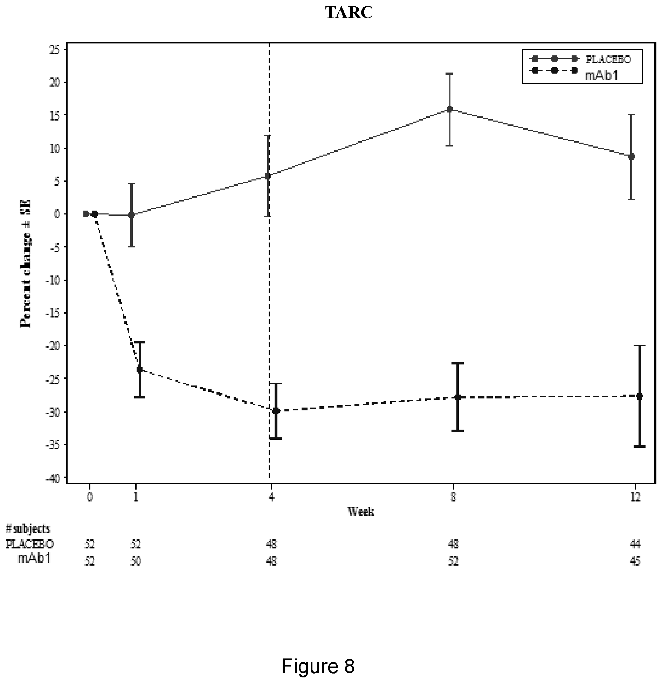

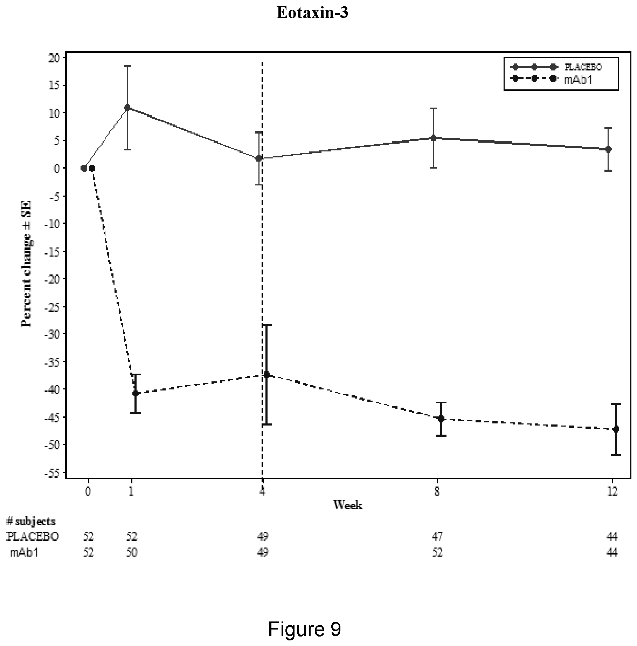

TARC, eotaxin-3, and IgE remained unchanged in response to placebo (Figures 8, 9 and 10). In contrast, a rapid reduction in TARC (mean % change -22.7% vs +0.3%; p= 0.0003) (Figure 8) and eotaxin-3 (mean % change -39.62% vs 12.69%; p<0.0001) (Figure 9) was observed within one week in patients treated with mAb1 and persisted until week 12: TARC: -26.0% vs +7.6% placebo (p=0.0005); Eotaxin-3: -45.67% vs +5.13% placebo (p<0.0001).

TARC levels responded within a week following exposure to mAb1 at 300 mg administered subcutaneously. TARC levels plateau at approximately 50% of the baseline level in mAb1-treated subjects, regardless of ICS withdrawal. The data suggest that TARC expression is more directly linked to IL-4R signaling, than FEV1 changes (which drop in parallel to ICS withdrawal [after Week 4]) and that IL-4R blockage induces a shift towards a TH1 signature, as observed with, for example, IFNgamma administration. It might be possible to titrate the mAb1 dose using TARC (and for example CXCL10) in particular in patients requiring long term treatment and at risk for TH1 type immune diseases.

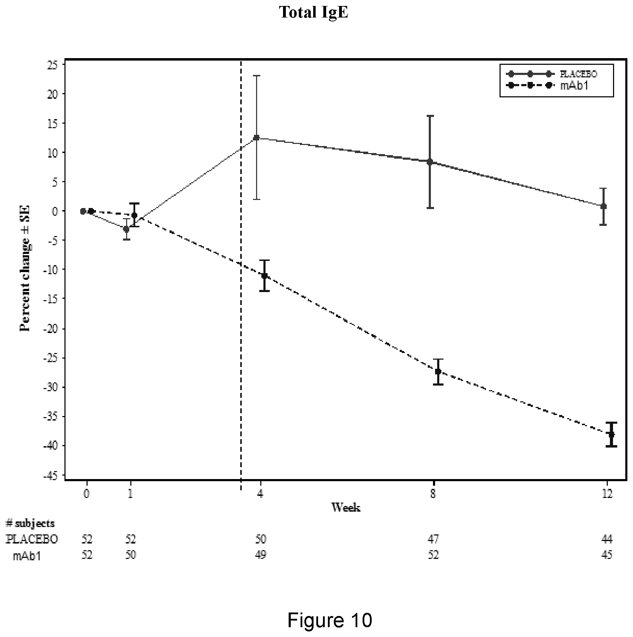

Total serum IgE also decreased following mAb1 treatment. Total serum IgE response was more heterogenous and delayed compared to TARC response. Mean (SD) baseline IgE levels were 694.68 IU/L (1837.82) for the placebo group (n=52) and 657.66 (1482.25) for the mAb1 group (n=52), whereas the median was 169.95 for the placebo group and 206.15 for the mAb1 group. Despite this heterogeneity, a trend towards IgE decrease in mAb1-exposed patients compared with placebo was observed - however, starting at week 4 only. Serum IgE was significantly reduced in the mAb1 group compared with placebo (mean % change, -10.1% vs +13.5%; p=0.0325) starting from week 4 and continued to decrease until week 12 (mean % change, -36.8% for REGN668/SAR231893 vs -5.5% for placebo; p<0.0001) (Figure 10).

Changes from baseline and placebo at Week 12 for FeNO, TARC, eotaxin-3, and IgE all favored mAb1 (all P< 0.001) (Table 11). No differences from baseline or between treatments were observed in YKL-40 or CEA.

**Table 11. Percent Change From Baseline at Week 12 in Pharmacodynamic Endpoints.**

| **Outcome** | **Least-Squares Mean Percent Change ± Standard Error** | | **P Value** |
|---|---|---|---|
| | **Placebo (N = 52)** | **mAb1 ( N = 52)** | |
| FeNO | 3̅5̅.̅0̅ ± 10.8 | 28̅.̅7̅ ± 11.2 | < 0.001 |
| TARC | 7.6 ± 6.9 | -26.0 ± 6.9 | < 0.001 |
| Eotaxin-3 | 5.1 ± 4.7 | -45.7 ± 4.7 | < 0.001 |
| IgE | 5.5 ± 3.6 | -36.8 ± 3.6 | < 0.001 |
| Blood eosinophils | 2.7 ± 15.8 | 41.6 ± 15.7 | 0.078 |

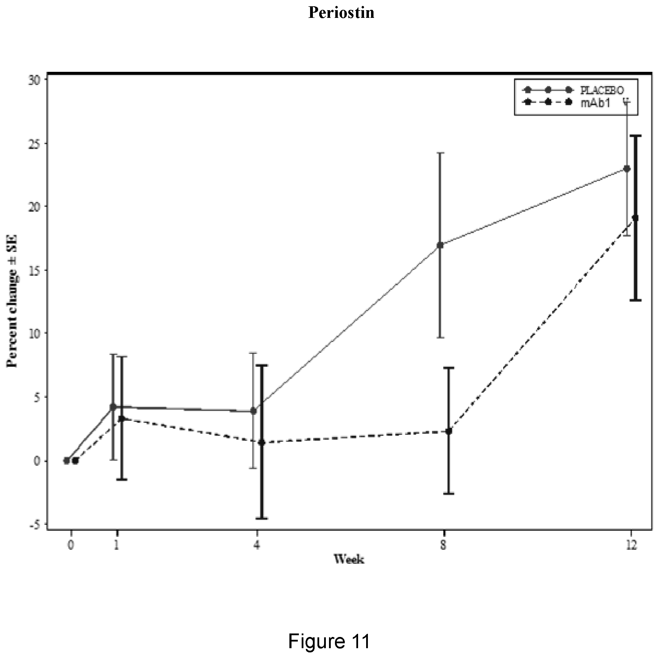

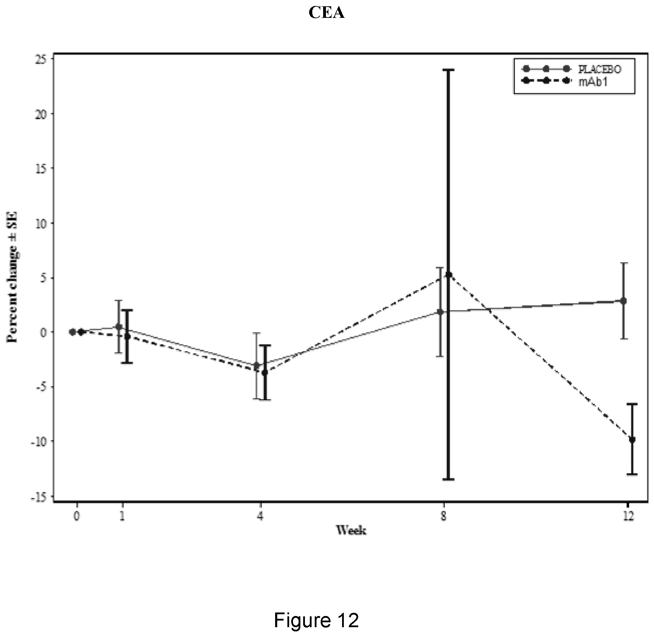

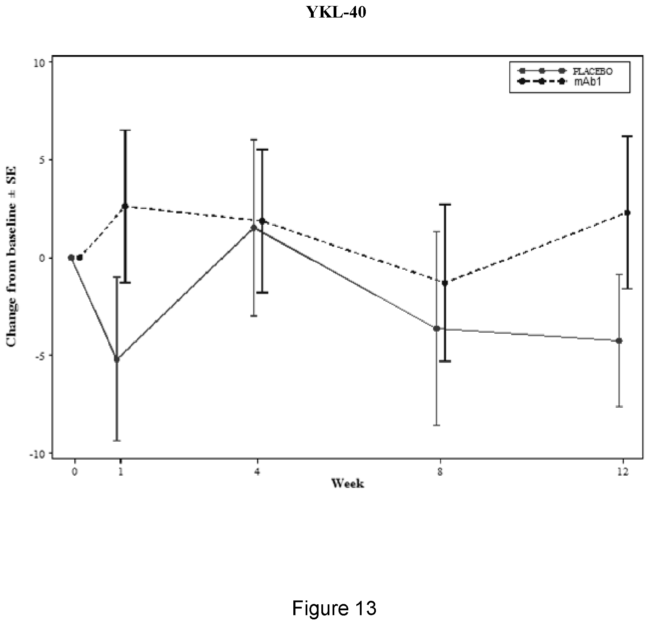

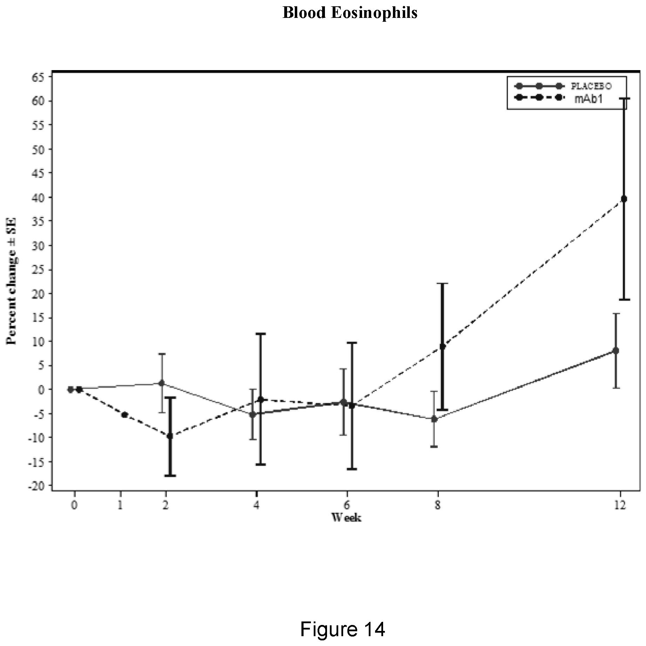

There was a transient decrease in periostin levels, followed by an increase with LABA/ICS withdrawal (Figure 11). Administration of mAb1 delayed the increase, but did not prevent the increase above baseline. No consistent treatment effect was observed with CEA (Figure 12) and YKL-40 (Figure 13). The number of blood eosinophils remained unchanged through Week 6, but then increased at Weeks 8 and 12 (Figure 14). Peripheral blood eosinophil numbers were unchanged on placebo throughout treatment. The difference between the treatments was not significant, with the borderline increase driven by larger blood eosinophil elevations in only a few patients treated with mAb1. Little or no increases were observed in the majority of patients (Table 12).

**Table 12. Proportions of Patients Achieving Thresholds of Change in Blood Eosinophil Levels.**

| Change in eosinophils | Number (%) of patients | |
|---|---|---|
| | Placebo (n = 52) | mAb1 (n = 52) |
| > 15% Decrease | 1̅3̅ (̅3̅0̅.̅2̅)̅ | 21 (47.7) |
| 15% Decrease - 0% change | 7 (16.3) | 6 (13.6) |
| 0%-15% Increase | 8 (18.6) | 4 (9.1) |
| 15% - 100% Increase | 13 (30.2) | 6 (13.6) |
| 100% - 200% increase | 2 (4.7) | 3 (6.8) |
| > 200% increase | 0 | 4 (9.1) |

Because only 3 mAb1 patients experienced asthma exacerbation during the study, no conclusion could be drawn regarding the association between baseline biomarker levels and asthma exacerbations.

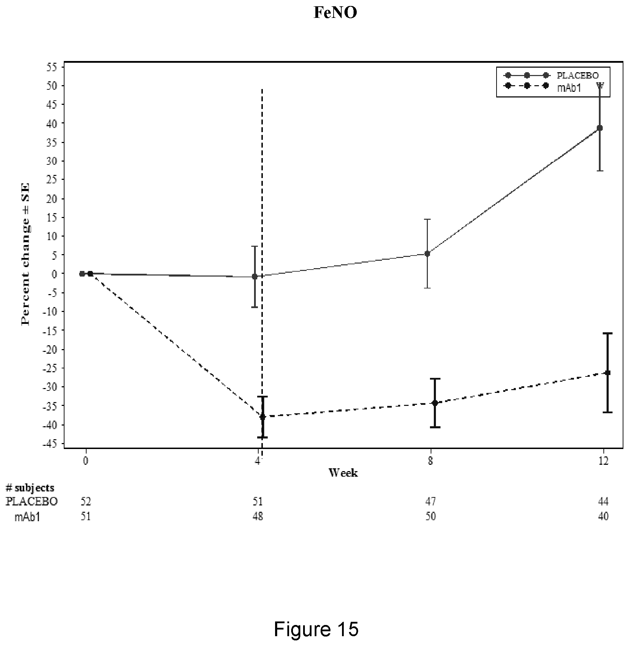

mAb1 treatment was also associated with a significant decrease from baseline in FeNO at Week 4, and FeNo remained below baseline through Week 12, regardless of ICS withdrawal (mean % change at week 12: -28.7 for mAb1 vs 35.0 for placebo; p<0.0001) (Figure 15). In contrast, placebo FeNo values remained stable through Week 8, followed by an increase at Week 12 coincident with ICS withdrawal.

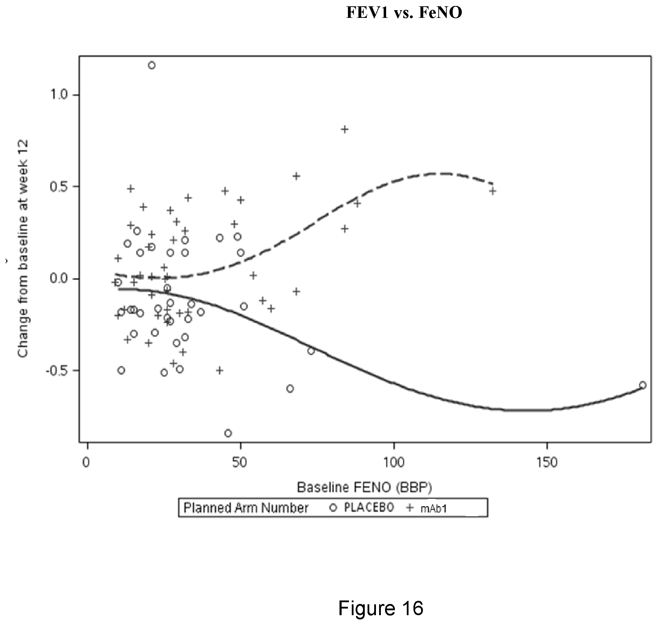

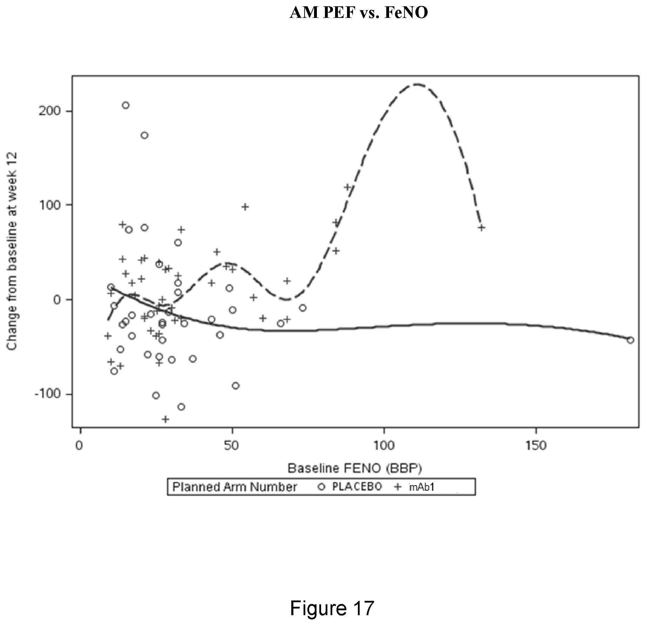

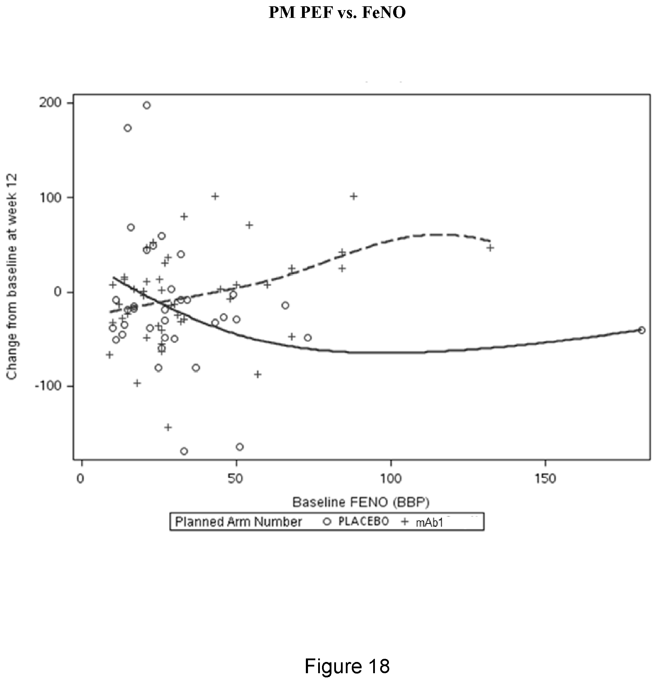

Forced expiratory volume in 1 second (FEV₁) improvement significantly correlated with FeNO reduction (r=-0.408, p=0.009) at week 12 (Figure 16). Similarly, improvements in AM-PEF and PM-PEF correlated with FeNO reduction (Figures 17 and 18). Other correlations with FeNO were not significant. See Table 13.

**Table 13. Correlation between FEV₁ and PD Endpoints.**

| **Outcome** | **C̅o̅r̅r̅e̅l̅a̅t̅i̅o̅n̅** | **P Value** |
|---|---|---|
| FeNO | -̅0̅.̅4̅0̅8̅ | <0.009 |
| TARC | -0.248 | 0.10 |
| Eotaxin-3 | -0.146 | 0.34 |
| IgE | -0.279 | 0.06 |
| Blood eosinophils | 0.165 | 0*.*28 |

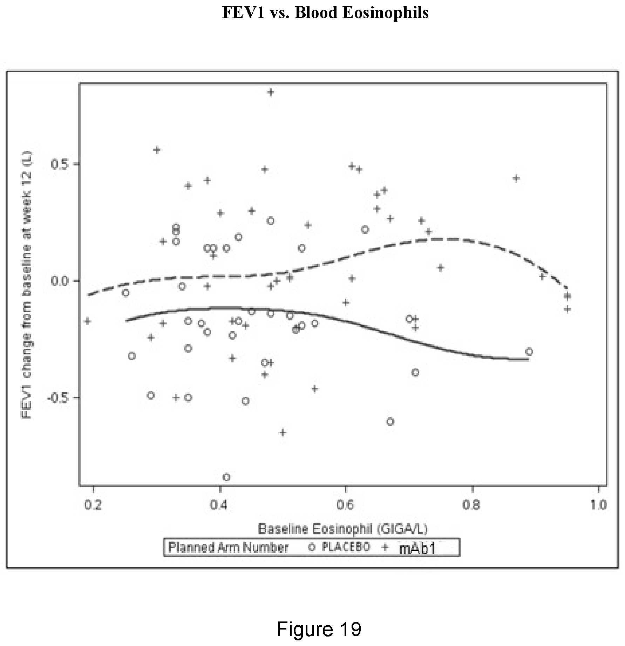

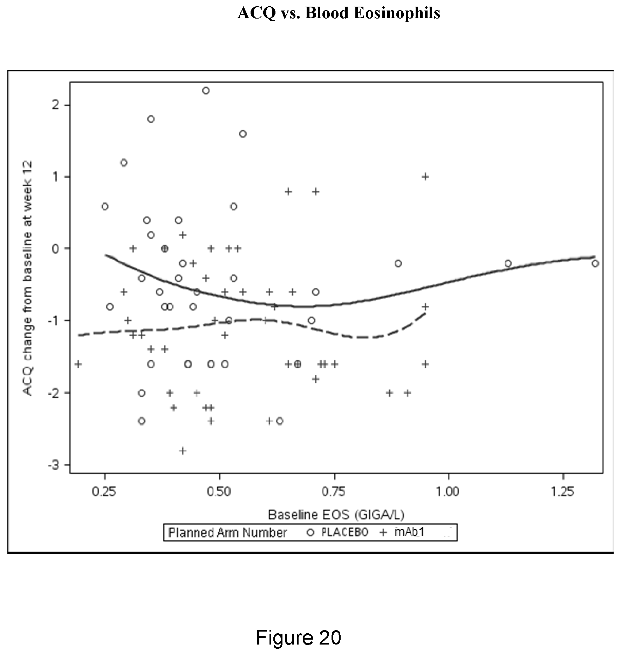

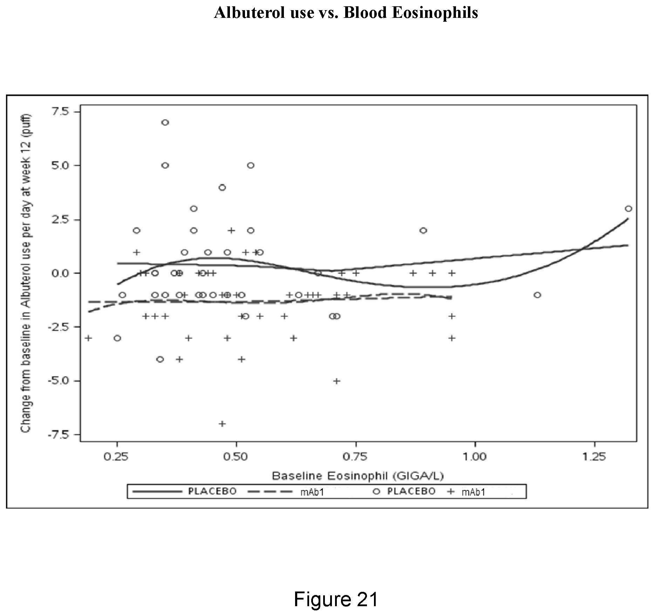

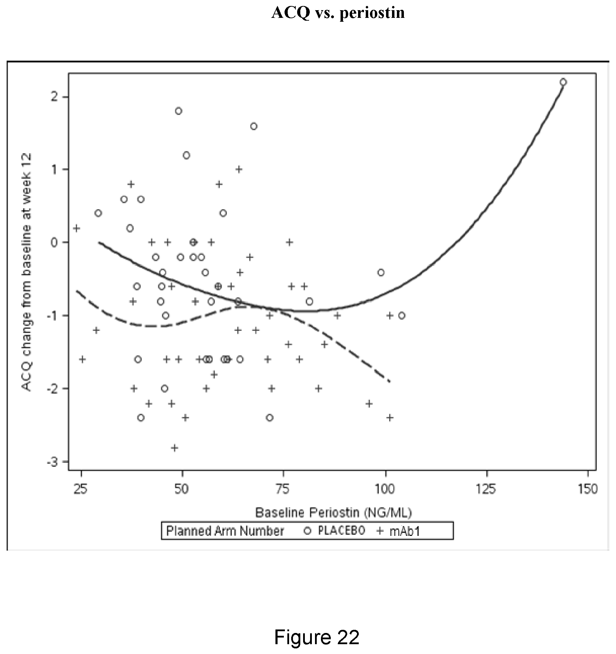

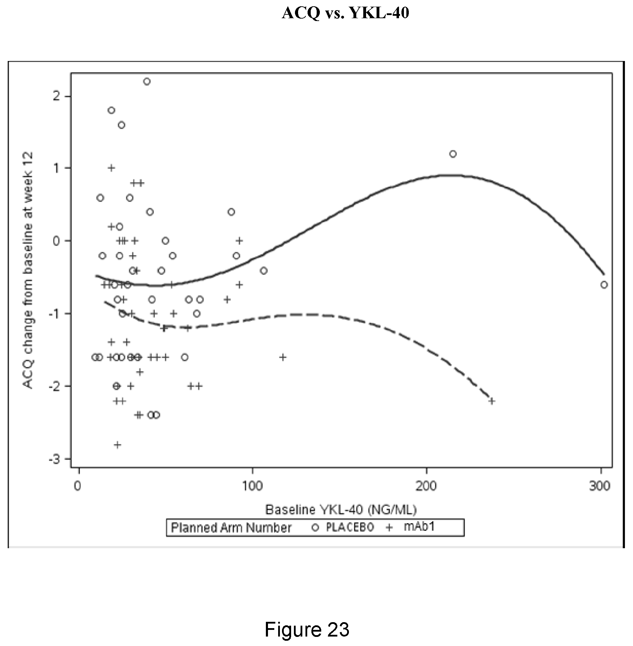

Scatter plot analysis of baseline eosinophils versus change from baseline in FEV1 at week 12 did not seem to suggest association of baseline eosinophils and treatment effect, as measured by change from baseline in FEV1 at week 12 in the study population (baseline eosinophils ≥ 0.3 Giga/L) (Figure 19). Baseline eosinophils correlated with decreased ACQ (Figure 20) and decreased albuterol/levalbuterol use (Figure 21). Periostin and YKL-40 at baseline correlated with decreased ACQ (Figures 22 and 23).

The FEV1 change from baseline at week 12 was compounded by the withdrawal of ICS (starting at week 4). Similar analyses did not suggest association between baseline TARC or IgE and change from baseline in FEV1 at week 12 in the study population (baseline eosinophils ≥ 0.3 Giga/L).

### Summary

These results show that mAb1 significantly reduced serum biomarkers associated with Th2 inflammation (TARC, eotaxin-3 and IgE) and bronchial inflammation (FeNO) in adult asthma patients. The correlation between FeNO reduction and FEV₁ improvement suggests a relationship between IL-4/IL-13 mediated anti-inflammatory activity and improvement in pulmonary function in moderate-to-severe, uncontrolled asthma.

The present invention is not to be limited in scope by the specific embodiments described herein. Indeed, various modifications in addition to those described herein will become apparent to those skilled in the art from the foregoing description and the accompanying figures. Such modifications are intended to fall within the scope of the appended claims.

### Example 4: Blockade of the IL-4/IL-13 Signaling Pathway Inhibits IgE Production and Airway Remodeling in a Mouse Model of House Dust Mite-Induced Eosinophilic Asthma

### INTRODUCTION

House dust mite allergen (HDM) has been shown to induce the Th2 immune response, including an influx of Th2 cells into the lung, and IL-4-induced trans-endothelial migration of eosinophils into the lungs. Eosinophils are the predominant effector cells in allergic reactions and the release of granule contents (including IL-4) from eosinophils contributes to inflammation. In asthmatic patients, Th2 driven production of IL-4 promotes eosinophil migration from blood into lungs via eotaxin, a potent eosinophil chemoattractant (Mochizuki et al., J. Immunol.,1998, 160(1):60-68). Moreover, when localized at the inflammatory site, eosinophils produce and secrete IL-4, thus contributing to ongoing Th2-driven inflammation (Bjerke et al., Respir. Med., 1996, 90(5):271-277). In patients with allergic asthma, a challenge with HDM increased the level of IgE and Th2 cytokines in serum for up to 5 weeks after the allergen challenge (van de Pol *et al., Allergy,* 2012, *67(1)*:67-73).

In this Example, an HDM-induced model of chronic asthma was used to evaluate the pharmacodynamic effects of anti-IL-4R antibodies on markers of airway inflammation in mice. Further, the effects of anti-IL-4R antibodies on collagen deposition in airways were evaluated in this model, since collagen deposition correlates with the extent of airway remodeling.

### MATERIALS AND METHODS

Two different anti-IL-4Rα antibodies were used in the experiments of this Example: "mAb1", a fully human monoclonal antibody specific for human IL-4Rα (i.e., the anti-IL-4R antibody used in the other working examples set forth herein); and "anti-mIL-4Rα", a mouse monoclonal antibody specific for the mouse IL-4Rα protein. mAb1 does not cross-react with mouse IL-4Rα; therefore, mAb1 was evaluated in humanized mice in which both human IL-4 and the ectodomain of IL-4Rα were engineered to replace their corresponding murine sequences in the mice (IL-4^{hu/hu} IL-4Rα^{hu/hu}). The mouse anti-mouse IL-4Rα antibody "anti-mIL-4Rα," on the other hand, was tested in wild-type (Balb/c) mice. Also tested in these experiments was a mouse IL-13Rα2-mFc fusion protein that acts as a decoy receptor, blocking IL-13 signaling by sequestration of the IL-13 cytokine.

For the HDM-induced asthma model, mice were sensitized by daily intranasal application of HDM (50 µg per mouse in 20 µL of PBS) for 10 days, followed by rest (resolution period of 2 weeks). Allergen challenge was administered by intranasal application of HDM (50 µg per mouse in 20 µL of PBS) three times a week for 8 weeks. For each administration of HDM, either during sensitization or challenge period, the mice were lightly anesthetized with isoflurane.

Mice were acclimated in the experimental facility for a minimum of 5 days before initiating the experimental procedure. For the entire duration of the experiment, animals remained housed in the experimental facility under standard conditions in a 12-hour day/night cycle with access to food and water *ad libitum.* The number of mice per cage was limited to a maximum of 5 mice.

A total number of 48 humanized mice in which the human IL-4 ligand and the human ectodomain of IL-4Rα, were engineered to replace their corresponding murine sequences (IL-4^{hu/hu} IL-4Rα^{hu/hu}) were used for two experiments. IL-4^{hu/hu} IL-4Rα^{hu/hu} mice were of a mixed background C57BI/6NTac (75%)/129S6SvEvTac(25%). In addition, 20 wild type littermate mice on an identical mixed background were used in one of the three experiments. In each experiment, mice were sensitized with HDM (or with PBS in the control group) daily for ten days, followed by a resolution period from day 11 to day 29. From day 30, animals were challenged with HDM three times a week for 8 weeks, until day 81, and then euthanized on day 85 for analysis. Mice were divided into six experimental groups as follows:
(1) Non-sensitized, not treated: PBS was applied intra-nasally during the sensitization and challenge periods. Mice were not treated with antibodies (IL-4^{hu/hu} IL-4Rα^{hu/hu} mice n = 9; wild type littermates n = 5);
(2) HDM-sensitized, not treated: HDM was applied intra-nasally during the sensitization and challenge periods. Mice were not treated with antibodies (IL-4^{hu/hu} IL-4Rα^{hu/hu} mice n = 7; wild type littermates n = 5);
(3) HDM-sensitized, treated with anti-mIL-4Rα: HDM was applied intra-nasally during the sensitization and challenge periods. Mice were injected i.p. with anti-mIL-4Rα at dose 50 mg/kg, twice a week, from week 7 to week 12, for a total of 12 doses during a 6 week period (wild type littermates n = 5);
(4) HDM-sensitized, treated with anti-human mAb1: HDM was applied intra-nasally during the sensitization and challenge periods. Mice were injected i.p. with mAb1 at dose 50 mg/kg, twice a week, from week 7 to week 12, for a total of 12 doses during a 6 week period (IL-4^{hu/hu} IL-4Rα^{hu/hu} mice n = 12);
(5) HDM-sensitized, treated with mouse IL-13Rα2-mFc fusion protein: HDM was applied intra-nasally during the sensitization and challenge periods. Mice were injected i.p. with the IL-13Rα2-mFc at dose 25 mg/kg, twice a week, from week 7 to week 12, for total of 12 doses during a 6 week period (IL-4^{hu/hu} IL-4Rα^{hu/hu} mice n = 7; wild type littermates n = 5);
(6) HDM-sensitized, treated with isotype control antibody: HDM was applied intra-nasally during the sensitization and challenge periods. Mice were injected i.p. with the isotype control Ab at dose 50 mg/kg, twice a week, from week 7 to week 12, for total of 12 doses during a 6 week period (IL-4^{hu/hu} IL-4Rα^{hu/hu} mice n = 7).

Mice were euthanized on day 85, blood was collected for serum immunoglobulin level assays, and lung (one lobe) was used to generate either i) bronchoalveolar lavage (BAL) fluid, ii) a digested single-cell suspension sample for flow cytometric analysis, iii) a fixed formalin specimen for staining and histology analysis, or iv) a sample for analysis using the Sircol^{™} Collagen Assay to quantify the collagen content per lung lobe.

BAL fluid was obtained from euthanized animals by first exposing the trachea and introducing a 23G lavage tube through a small incision in the tracheal wall. Sterile PBS (1 mL) was then injected into the lungs, and BAL fluid was recovered through the lavage tube using a syringe. 100 µL of BAL was loaded onto a Cytospin that was spun for 5 minutes at 500 rpm to extract the cells onto microscope slides. The slides were dried and H & E stained to visualize eosinophils.

Serum level of IgE was quantified using a commercially available ELISA kit. Briefly, serially diluted serum samples were incubated with anti-IgE capture antibody on 96-well plates and the IgE was detected by biotinylated anti-mouse IgE secondary antibody. Purified mouse IgE that was HRP-labeled was used as a standard.

HDM-specific IgG1 serum levels were quantified by ELISA. Briefly, HDM-coated plates were incubated with serially diluted serum samples, following by incubation with anti-mouse IgG1-HRP conjugated antibody. The relative levels of IgG1 serum levels were represented as titer units (OD450 was multiplied by a dilution factor required to achieve OD450 ≤ 0.5). Collected lung lobes were flash frozen in liquid nitrogen and stored at -80°C until the extraction step. To extract the collagen, lungs were homogenized in ice-cold NaCl/NaHCO₃ solution and centrifuged at 9000xg for 10 min. This step was repeated three times, and resulting pellet was digested by pepsin in acetic acid for 18 hours at 4°C. Samples were centrifuged, and the supernatant was collected and mixed with Sircol Dye Reagent to stain for collagen content. Samples were washed with Acid-Salt Wash Reagent to remove unbound Sircol Dye and then mixed with Alkali Reagent. 200 µL of each sample was transferred into a 96-well plate, and OD at 555 nm was measured. A collagen standard was used for final quantification of collagen content in each sample.

Lungs were collected from euthanized mice and kept in complete DMEM medium on ice until digesting with a mixture of collagenase and DNAse in HBSS buffer for 20 minutes at 37°C. Collagenase activity was quenched by addition of 0.5M EDTA, samples were centrifuged, and red cells were lysed with ACK buffer. The cell suspensions obtained for each sample were divided into three separate pools and stained with antibody mix 1 (anti-CD11c-APC Ab, anti-SiglecF-PE Ab, anti-F4/80-FITC Ab, anti-CD45-PerCp-Cy5.5 Ab), or mix 2 (anti-CD11c-APC Ab, anti-CD11b-PerCp-Cy5.5 Ab, anti-CD103-FITC Ab, anti-MHCII-PE Ab), or mix 3 (anti-CD19-PE Ab, anti-Ly6G-APC Ab, anti-CD3-FITC, anti-CD11b-PerCp-Cy5.5 Ab) for 25 minutes at 4°C. Stained cells were fixed in Cytofix/Cytoperm solution for 30 minutes in 4°C, and stored in PBS until flow cytometry analysis by FACSCanto (BD Biosciences).

From the HDM-induced chronic model of eosinophilic asthma (EA), left lung lobes were collected from 4 mice per group for microarray analysis of gene expression using GeneChip^{®} technology. Gene expression levels in the mice that were sensitized and challenged with HDM and then treated with an isotype control Ab were compared to gene expression levels in mice that were mock (PBS) sensitized and challenged and did not undergo antibody treatment. The threshold for a change in gene expression was set at >1.5-fold. The population of genes identified as being differentially expressed in mice that were sensitized and challenged with HDM were then further analyzed in the anti-IL-4Rα-treated group relative to the isotype control-treated group. The threshold for a change in gene expression in the IL-4Rα-Ab treated group relative to the isotype control-treated group was set at >2-fold.

### RESULTS

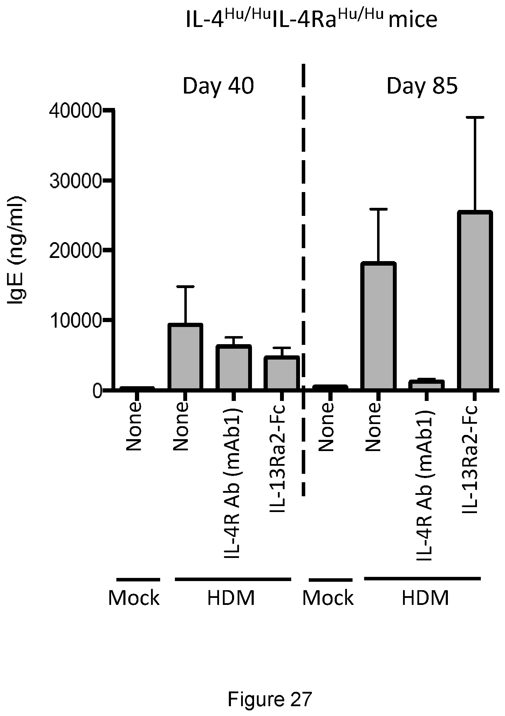

HDM sensitization and challenge resulted in increased levels of IgE and HDM-specific IgG1. IgE increase was completely blocked by both anti-IL-4Rα Abs but not by IL-13Rα2-Fc treatment (Figures 27A and 27B); HDM-specific IgG1 levels were not affected by any treatment (data not shown).

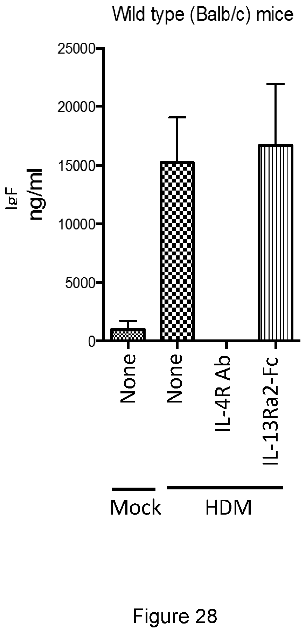

HDM sensitization and challenge also caused an increase in collagen content in the lungs of the mice. Collagen content in the lungs of mice treated with both IL-4Rα Abs and IL-13Rα2-Fc protein was reduced to levels observed in mock sensitized & challenged mice (Figures 28A and 28B).

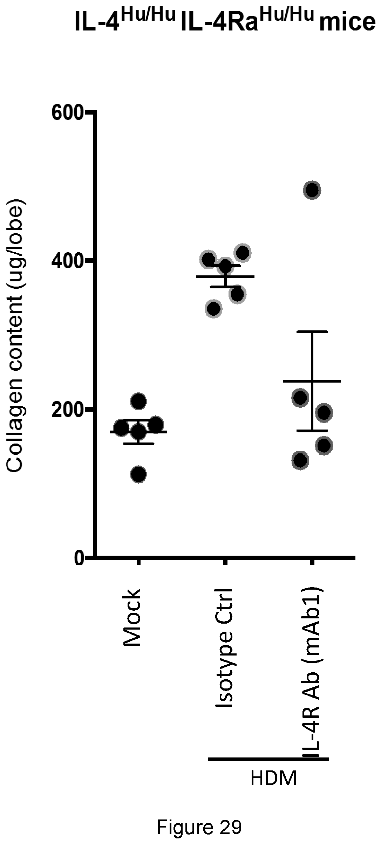

In addition, mAb1 treatment prevented influx of eosinophils, neutrophils, and inflammatory dendritic cells into lung (Figure 29, Panels A and B).

Microarray analysis of mRNA isolated from lung tissue of HDM-induced IL-4^{hu/hu} IL-4Rα^{hu/hu} mice that were treated with an isotype control antibody revealed differential expression of 1468 genes (826 up-regulated and 642 downregulated genes) as compared to mock sensitized and mock challenged mice. mAb1 treatment of HDM-induced IL-4^{hu/hu} IL-4Rα ^{hu/hu} mice resulted in expression changes in only 521 genes (as compared to mock sensitized/challenged mice), effectively blocking about 65% genes affected by HDM sensitization/challenge (> 1.5 fold change, p<0.05). Of particular interest is the finding that mAb1 mediated downregulation of gene expression of several members of IL-1 cytokine family, specifically IL-1α (2.9-fold), IL-33 (2.6-fold) and IL-18 binding protein (1.5-fold). IL-1β gene expression did not increase in the HDM-induced, isotype control treated group (as compared to mock sensitized mice), but was decreased (1.5-fold) in the mAb1-treated groups. Gene expression of Th1 inflammatory cytokines IL-12β and IFN-γ was also downregulated by mAb1 as compared to the isotype control treated group. Notably, eight genes coding chemokine ligands involved in cell homing and trafficking were downregulated in the mAb1-treated group when compared to the isotype control treated group: Ccl11 (~9-fold reduction), Ccl8 and Cxcl2 (both ~5-fold reduction), Cxcl1, Ccl7, Ccl6 (all ~3-fold reduction), Ccl2 and Ccl9 (about 2-fold reduction).

### CONCLUSIONS

This Example shows that blockade of IL-4 signaling via Type I and Type II receptors by anti-IL-4Rα antibodies suppresses inflammatory and fibrotic changes in lungs of HDM-challenged mice as well as gene signature changes driven by HDM.

Other embodiments are in the claims.
The invention pertains also to the following:
1. A method for reducing the incidence of one or more asthma exacerbations in a subject in need thereof comprising administering to the subject a therapeutically effective amount of a pharmaceutical composition comprising an interleukin-4 receptor (IL-4R) antagonist.
2. The method of item 1, wherein the asthma exacerbation is selected from the group consisting of:
   (a) a 30% or greater reduction from baseline in morning peak expiratory flow (PEF) on two consecutive days;
   (b) six or more additional reliever puffs of albuterol or levalbuterol in a 24 hour period (compared to baseline) on two consecutive days; and
   (c) a deterioration of asthma requiring:
      (i) systemic (oral and/or parenteral) steroid treatment, or
      (ii) an increase in inhaled corticosteroids to at least 4 times the last dose received prior to discontinuation, or
      (iii) hospitalization.
3. The method of item 1 or 2, wherein the IL-4R antagonist is an antibody or an antigen-binding fragment thereof that specifically binds to IL-4R.
4. The method of item 3, wherein the pharmaceutical composition comprises 75 mg to 600 mg of the antibody or antigen-binding fragment thereof that specifically binds to IL-4R.
5. The method of item 4, wherein the pharmaceutical composition comprises 300 mg of the antibody or antigen-binding fragment thereof that specifically binds to IL-4R.
6. The method of item 3, wherein the antibody or antigen-binding fragment thereof that specifically binds to IL-4R comprises heavy and light chain complementarity determining region (CDR) sequences from a heavy chain variable region (HCVR)/light chain variable region (LCVR) sequence pair selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, and 262/264.
7. The method of item 6, wherein the antibody or antigen-binding fragment thereof that specifically binds to IL-4R comprises heavy and light chain CDR sequences from the HCVR/LCVR sequence pair comprising SEQ ID NOs: 162/164.
8. The method of item 7, wherein the antibody or antigen-binding fragment thereof that specifically binds to IL-4R comprises three heavy chain complementarity determining region (HCDR) sequences comprising SEQ ID NOs: 148, 150, 152, respectively, and three light chain complementarity determining region (LCDR) sequences comprising SEQ ID NOs: 156, 158 and 160, respectively.
9. The method of item 8, wherein the antibody or antigen-binding fragment thereof that specifically binds to IL-4R comprises an HCVR comprising the amino acid sequence of SEQ ID NO: 162 and an LCVR comprising the amino acid sequence of SEQ ID NO: 164.
10. The method of any one of items 1-9, wherein the pharmaceutical composition is administered to the subject systemically, subcutaneously, intravenously, or intranasally.
11. The method of any one of items 1-10, wherein a second therapeutic agent is administered to the subject before, after, or concurrent with the pharmaceutical composition.
12. The method of item 11, wherein the second therapeutic agent is selected from the group consisting of: a TNF inhibitor, an IL-1 inhibitor, an IL-5 inhibitor, an IL-8 inhibitor, an IgE inhibitor, a leukotriene inhibitor, a corticosteroid, a methylxanthine, an NSAID, nedocromil sodium, cromolyn sodium, a long-acting beta₂ agonist, an anti-fungal agent, and a combination thereof.
13. The method of item 12, wherein the second therapeutic agent comprises a combination of an inhaled corticosteroid and a long-acting beta₂ agonist.
14. The method of item 13, wherein the inhaled corticosteroid is fluticasone or budesonide.
15. The method of item 13, wherein the long-acting beta₂ agonist is salmeterol or formoterol.
16. The method of item 13, wherein the inhaled corticosteroid is fluticasone and the long-acting beta₂ agonist is salmeterol.
17. The method of item 13, wherein the inhaled corticosteroid is budesonide and the long-acting beta₂ agonist is formoterol.
18. A method for improving one or more asthma-associated parameter(s) in a subject in need thereof comprising administering to the subject a therapeutically effective amount of a pharmaceutical composition comprising an interleukin-4 receptor (IL-4R) antagonist, wherein the improvement in an asthma-associated parameter is selected from the group consisting of:
   (a) an increase from baseline of forced expiratory volume in 1 second (FEV1) in liters;
   (b) an increase from baseline of morning peak expiratory flow rate (AM PEF) in liters/minute;
   (c) an increase from baseline of evening peak expiratory flow rate (PM PEF) in liters/minute;
   (d) a decrease from baseline of daily albuterol/levalbuterol use in inhalations/day;
   (e) a decrease from baseline of five-item Asthma Control Questionnaire (ACQ5) score;
   (f) a decrease from baseline of nighttime awakenings (no. of times per night) measured daily; and
   (g) a decrease from baseline of 22-item Sino-Nasal Outcome Test (SNOT-22) score.
19. The method of item 18, wherein the improvement in an asthma-associated parameter is an increase of FEV1 from baseline of at least 0.10 L.
20. The method of item 18, wherein the improvement in an asthma-associated parameter is an increase in AM PEF from baseline of at least 10.0 L/min.
21. The method of item 18, wherein the improvement in an asthma-associated parameter is an increase in PM PEF from baseline of at least 1.0 L/min.
22. The method of item 18, wherein the improvement in an asthma-associated parameter is a decrease in albuterol/levalbuterol use from baseline of at least 1 puff(s) per day.
23. The method of item 18, wherein the improvement in an asthma-associated parameter is a decrease in ACQ5 score from baseline of at least 0.5 points.
24. The method of item 18, wherein the improvement in an asthma-associated parameter is a decrease in nighttime awakenings from baseline of at least 0.2 times per night.
25. The method of item 18, wherein the improvement in an asthma-associated parameter is a decrease in SNOT-22 score from baseline of at least 5 points.
26. The method of any one of items 18-25, wherein the IL-4R antagonist is an antibody or an antigen-binding fragment thereof that specifically binds to IL-4R.
27. The method of any one of items 18-26, wherein the pharmaceutical composition comprises 75 mg to 600 mg of the antibody or antigen-binding fragment thereof that specifically binds to IL-4R.
28. The method of item 27, wherein the pharmaceutical composition comprises 300 mg of the antibody or antigen-binding fragment thereof that specifically binds to IL-4R.
29. The method of item 26, wherein the antibody or antigen-binding fragment thereof that specifically binds to IL-4R comprises heavy and light chain complementarity determining region (CDR) sequences from a heavy chain variable region (HCVR)/light chain variable region (LCVR) sequence pair selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, and 262/264.
30. The method of item 29, wherein the antibody or antigen-binding fragment thereof that specifically binds to IL-4R comprises heavy and light chain CDR sequences from the HCVR/LCVR sequence pair comprising SEQ ID NOs: 162/164.
31. The method of item 30, wherein the antibody or antigen-binding fragment thereof that specifically binds to IL-4R comprises three heavy chain complementarity determining region (HCDR) sequences comprising SEQ ID NOs: 148, 150, 152, respectively, and three light chain complementarity determining (LCDR) sequences comprising SEQ ID NOs: 156, 158 and 160, respectively.
32. The method of item 31, wherein the antibody or antigen-binding fragment thereof that specifically binds to IL-4R comprises an HCVR comprising the amino acid sequence of SEQ ID NO: 162 and an LCVR comprising the amino acid sequence of SEQ ID NO: 164.
33. The method of any one of items 18-32, wherein the pharmaceutical composition is administered to the subject systemically, subcutaneously, intravenously, or intranasally.
34. The method of any one of items 18-33, wherein a second therapeutic agent is administered to the subject before, after, or concurrent with the pharmaceutical composition.
35. The method of item 34, wherein the second therapeutic agent is selected from the group consisting of: a TNF inhibitor, an IL-1 inhibitor, an IL-5 inhibitor, an IL-8 inhibitor, an IgE inhibitor, a leukotriene inhibitor, a corticosteroid, a methylxanthine, an NSAID, nedocromil sodium, cromolyn sodium, a long-acting beta₂ agonist, an anti-fungal agent, and a combination thereof.
36. The method of item 35, wherein the second therapeutic agent comprises a combination of an inhaled corticosteroid and a long-acting beta₂ agonist.
37. The method of item 36, wherein the inhaled corticosteroid is fluticasone or budesonide.
38. The method of item 36, wherein the long-acting beta₂ agonist is salmeterol or formoterol.
39. The method of item 36, wherein the inhaled corticosteroid is fluticasone and the long-acting beta₂ agonist is salmeterol.
40. The method of item 36, wherein the inhaled corticosteroid is budesonide and the long-acting beta₂ agonist is formoterol.
41. A method for treating moderate to severe eosinophilic asthma in a subject in need thereof comprising administering to the subject a therapeutically effective amount of a pharmaceutical composition comprising an interleukin-4 receptor (IL-4R) antagonist, wherein the subject is identified as having a blood eosinophil level of at least 300 cells per microliter and/or a sputum eosinophil level of at least 3%.
42. The method of item 41, wherein the IL-4R antagonist is an antibody or an antigen-binding fragment thereof that specifically binds to IL-4R.
43. The method of item 41 or 42, wherein the pharmaceutical composition comprises 75 mg to 600 mg of the antibody or antigen-binding fragment thereof that specifically binds to IL-4R.
44. The method of item 43, wherein the pharmaceutical composition comprises 300 mg of the antibody or antigen-binding fragment thereof that specifically binds to IL-4R.
45. The method of item 42, wherein the antibody or antigen-binding fragment thereof that specifically binds to IL-4R comprises heavy and light chain complementarity determining region (CDR) sequences from a heavy chain variable region (HCVR)/light chain variable region (LCVR) sequence pair selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, and 262/264.
46. The method of item 44, wherein the antibody or antigen-binding fragment thereof that specifically binds to IL-4R comprises heavy and light chain CDR sequences from the HCVR/LCVR sequence pair comprising SEQ ID NOs: 162/164.
47. The method of item 46, wherein the antibody or antigen-binding fragment thereof that specifically binds to IL-4R comprises three heavy chain complementarity determining region (HCDR) sequences comprising SEQ ID NOs: 148, 150, 152, respectively, and three light chain complementarity determining (LCDR) sequences comprising SEQ ID NOs: 156, 158 and 160, respectively.
48. The method of item 47, wherein the antibody or antigen-binding fragment thereof that specifically binds to IL-4R comprises an HCVR comprising the amino acid sequence of SEQ ID NO: 162 and an LCVR comprising the amino acid sequence of SEQ ID NO: 164.
49. The method of any one of items 41-48, wherein the pharmaceutical composition is administered to the subject systemically, subcutaneously, intravenously, or intranasally.
50. The method of any one of items 41-49, wherein a second therapeutic agent is administered to the subject before, after, or concurrent with the pharmaceutical composition.
51. The method of item 50, wherein the second therapeutic agent is selected from the group consisting of: a TNF inhibitor, an IL-1 inhibitor, an IL-5 inhibitor, an IL-8 inhibitor, an IgE inhibitor, a leukotriene inhibitor, a corticosteroid, a methylxanthine, an NSAID, nedocromil sodium, cromolyn sodium, a long-acting beta₂ agonist, an anti-fungal agent, and a combination thereof.
52. The method of item 51, wherein the second therapeutic agent comprises a combination of an inhaled corticosteroid and a long-acting beta₂ agonist.
53. The method of item 52, wherein the inhaled corticosteroid is fluticasone or budesonide.
54. The method of item 52, wherein the long-acting beta₂ agonist is salmeterol or formoterol.
55. The method of item 52, wherein the inhaled corticosteroid is fluticasone and the long-acting beta₂ agonist is salmeterol.
56. The method of item 52, wherein the inhaled corticosteroid is budesonide and the long-acting beta₂ agonist is formoterol.
57. A method for reducing the incidence of asthma exacerbations or improving one or more asthma-associated parameter(s) in a subject in need thereof comprising sequentially administering to a subject in need thereof a single initial dose of a pharmaceutical composition comprising an interleukin-4 receptor (IL-4R) antagonist, followed by one or more secondary doses of the pharmaceutical composition comprising the IL-4R antagonist.
58. The method of item 57, wherein the IL-4R antagonist is an antibody or an antigen-binding fragment thereof that specifically binds to IL-4R.
59. The method of item 57 or 58, wherein the pharmaceutical composition comprises 75 mg to 600 mg of the antibody or antigen-binding fragment thereof that specifically binds to IL-4R.
60. The method of item 59, wherein the pharmaceutical composition comprises 300 mg of the antibody or antigen-binding fragment thereof that specifically binds to IL-4R.
61. The method of item 58, wherein the antibody or antigen-binding fragment thereof that specifically binds to IL-4R comprises heavy and light chain complementarity determining region (CDR) sequences from a heavy chain variable region (HCVR)/light chain variable region (LCVR) sequence pair selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, and 262/264.
62. The method of item 61, wherein the antibody or antigen-binding fragment thereof that specifically binds to IL-4R comprises heavy and light chain CDR sequences from the HCVR/LCVR sequence pair comprising SEQ ID NOs: 162/164.
63. The method of item 62, wherein the antibody or antigen-binding fragment thereof that specifically binds to IL-4R comprises three heavy chain complementarity determining region (HCDR) sequences comprising SEQ ID NOs: 148, 150, 152, respectively, and three light chain complementarity determining (LCDR) sequences comprising SEQ ID NOs: 156, 158 and 160, respectively.
64. The method of item 63, wherein the antibody or antigen-binding fragment thereof that specifically binds to IL-4R comprises an HCVR comprising the amino acid sequence of SEQ ID NO: 162 and an LCVR comprising the amino acid sequence of SEQ ID NO: 164.
65. The method of any one of items 57-64, wherein the pharmaceutical composition is administered to the subject systemically, subcutaneously, intravenously, or intranasally.
66. The method of any one of items 57-65, wherein a second therapeutic agent is administered to the subject before, after, or concurrent with the initial dose and/or the secondary dose of the pharmaceutical composition.
67. The method of item 66, wherein the second therapeutic agent is selected from the group consisting of: a TNF inhibitor, an IL-1 inhibitor, an IL-5 inhibitor, an IL-8 inhibitor, an IgE inhibitor, a leukotriene inhibitor, a corticosteroid, a methylxanthine, an NSAID, nedocromil sodium, cromolyn sodium, a long-acting beta₂ agonist, an anti-fungal agent, and a combination thereof.
68. The method of item 67, wherein the second therapeutic agent comprises a combination of an inhaled corticosteroid and a long-acting beta₂ agonist.
69. The method of item 68, wherein the inhaled corticosteroid is fluticasone or budesonide.
70. The method of item 68, wherein the long-acting beta₂ agonist is salmeterol or formoterol.
71. The method of item 68, wherein the inhaled corticosteroid is fluticasone and the long-acting beta₂ agonist is salmeterol.
72. The method of item 68, wherein the inhaled corticosteroid is budesonide and the long-acting beta₂ agonist is formoterol.
73. The method of any one of items 57-72, wherein each secondary dose is administered 1 to 8 weeks after the immediately preceding dose.
74. The method of any one of items 57-72, wherein at least 8 secondary doses of the IL-4R antagonist are administered to the subject, and wherein each secondary dose is administered 1 week after the immediately preceding dose.
75. The method of any one of items 57-74, wherein the initial dose and the one or more secondary doses each comprise 75 mg to 600 mg of the IL-4R antagonist.
76. The method of item 75, wherein the initial dose and the one or more secondary doses each comprise 300 mg of the IL-4R antagonist.
77. A method for reducing the incidence of asthma exacerbations or improving one or more asthma-associated parameter(s) in a subject in need thereof comprising administering to the subject 300 mg of a pharmaceutical composition comprising an antibody or antigen-binding fragment thereof that specifically binds to IL-4R, wherein the pharmaceutical composition is administered to the subject at a dosing frequency of once a week.
78. The method of item 77, wherein the antibody or antigen-binding fragment thereof that specifically binds to IL-4R comprises heavy and light chain complementarity determining region (CDR) sequences from a heavy chain variable region (HCVR)/light chain variable region (LCVR) sequence pair selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, and 262/264.
79. The method of item 78, wherein the antibody or antigen-binding fragment thereof that specifically binds to IL-4R comprises heavy and light chain CDR sequences from the HCVR/LCVR sequence pair comprising SEQ ID NOs: 162/164.
80. The method of item 79, wherein the antibody or antigen-binding fragment thereof that specifically binds to IL-4R comprises three heavy chain complementarity determining region (HCDR) sequences comprising SEQ ID NOs: 148, 150, 152, respectively, and three light chain complementarity determining (LCDR) sequences comprising SEQ ID NOs: 156, 158 and 160, respectively.
81. The method of item 80, wherein the antibody or antigen-binding fragment thereof that specifically binds to IL-4R comprises an HCVR comprising the amino acid sequence of SEQ ID NO: 162 and an LCVR comprising the amino acid sequence of SEQ ID NO: 164.
82. The method of any one of items 77-81, wherein the pharmaceutical composition is administered to the subject systemically, subcutaneously, intravenously, or intranasally.
83. The method of any one of items 77-82, wherein a second therapeutic agent is administered to the subject before, after, or concurrent with the pharmaceutical composition.
84. The method of item 83, wherein the second therapeutic agent is selected from the group consisting of: a TNF inhibitor, an IL-1 inhibitor, an IL-5 inhibitor, an IL-8 inhibitor, an IgE inhibitor, a leukotriene inhibitor, a corticosteroid, a methylxanthine, an NSAID, nedocromil sodium, cromolyn sodium, a long-acting beta₂ agonist, an anti-fungal agent, and a combination thereof.
85. The method of item 84, wherein the second therapeutic agent comprises a combination of an inhaled corticosteroid and a long-acting beta₂ agonist.
86. The method of item 85, wherein the inhaled corticosteroid is fluticasone or budesonide.
87. The method of item 85, wherein the long-acting beta₂ agonist is salmeterol or formoterol.
88. The method of item 85, wherein the inhaled corticosteroid is fluticasone and the long-acting beta₂ agonist is salmeterol.
89. The method of item 85, wherein the inhaled corticosteroid is budesonide and the long-acting beta₂ agonist is formoterol.
90. A method for treating asthma comprising: (a) selecting a patient that exhibits a blood eosinophil level of at least 300 cells per microliter and/or a sputum eosinophil level of at least 3%; and (b) administering to the patient a pharmaceutical composition comprising an IL-4R antagonist.
91. The method of item 90, wherein the IL-4R antagonist is an antibody or an antigen-binding fragment thereof that specifically binds to IL-4R.
92. The method of item 91, wherein the antibody or antigen-binding fragment thereof that specifically binds to IL-4R comprises three heavy chain complementarity determining region (HCDR) sequences comprising SEQ ID NOs: 148, 150, 152, respectively, and three light chain complementarity determining (LCDR) sequences comprising SEQ ID NOs: 156, 158 and 160, respectively.
93. The method of item 92, wherein the antibody or antigen-binding fragment thereof that specifically binds to IL-4R comprises an HCVR comprising the amino acid sequence of SEQ ID NO: 162 and an LCVR comprising the amino acid sequence of SEQ ID NO:164.
94. A method for reducing or eliminating an asthma patient's dependence on inhaled corticosteroids (ICS) and/or long-acting beta-agonists (LABA) for the treatment of one or more asthma exacerbations comprising:
   (a) selecting a patient who has moderate-to-severe asthma that is partially controlled or uncontrolled with a background asthma therapy comprising an ICS, a LABA, or a combination thereof;
   (b) administering to the patient a defined dose of an IL-4R antagonist at a defined frequency for an initial treatment period while maintaining the patient's background asthma therapy for the initial treatment period; and
   (c) gradually reducing or eliminating the dosage of ICS and/or LABA administered to the patient over the course of a subsequent treatment period while continuing to administer the IL-4R antagonist to the patient at the defined frequency and dose used during the initial treatment period.
95. The method of item 94, wherein the IL-4R antagonist is an anti-IL-4R antibody or an antigen binding fragment thereof.
96. The method of item 94 or 95, wherein the ICS is fluticasone, budesonide, or mometasone.
97. The method of any one of items 94-96, wherein the LABA is salmeterol or formoterol.
98. The method of any one of items 94-97, wherein the ICS/LABA combination is fluticasone/salmeterol, budesonide/formoterol, or mometasone/formoterol.
99. The method of any one of items 94-98, wherein the dosage of LABA is eliminated at the end of the initial treatment period.
100. The method of any one of items 94-99, wherein the dosage of LABA and /or ICS is gradually reduced or eliminated over the course of 2 to 8 weeks.
101. A method for treating moderate-to-severe asthma comprising: (a) selecting a patient with an elevated level of a biomarker selected from the group consisting of thymus and activation-regulated chemokine (TARC), IgE, eotaxin-3, periostin, carcinoembryonic antigen (CEA), YKL-40, and fractional exhaled nitric oxide (FeNO); and (b) administering to the patient a therapeutically effective amount of an IL-4R antagonist.
102. The method of item 101, wherein the IL-4R antagonist is an anti-IL-4R antibody or an antigen binding fragment thereof.
103. A method for monitoring the effectiveness of treatment of moderate-to-severe asthma in a subject comprising: (a) determining one or more of the expression level of one or both of TARC and eotaxin-3, or total serum level of IgE in a biological sample acquired from the subject before treatment with an IL-4R antagonist, (b) determining one or more of the expression level of one or both of TARC and eotaxin-3, or total serum level of IgE in a biological sample acquired from the subject after treatment with the IL-4R antagonist, (c) comparing the level determined in step (a) with the level in step (b), and (d) concluding that the treatment is effective when the level determined in step (b) is lower than the level determined in step (a), or concluding that the treatment is not effective when the level determined at step (b) is the same as or higher than the level determined at step (a).
104. The method of item 103, wherein the level in step (b) is determined 1 week, 2 weeks, 3 weeks, 4 weeks, or 5 weeks after determining the level in step (a).
105. The method of item 103, wherein if the level in step (b) is not less than the level in step (a), then administration of the IL-4R antagonist is stopped, or the dose of IL-4R antagonist is increased.
106. The method of item 105, wherein the level in step (b) is determined 1 week, 2 weeks, 3 weeks, 4 weeks, or 5 weeks after determining the level in step (a).
107. A method for monitoring a subject's response to treatment with an IL-4R antagonist, wherein the subject has moderate-to-severe asthma comprising:
   (a) acquiring information regarding the expression level of one or both of TARC or eotaxin-3, or total serum level of IgE in a biological sample from the subject following administration of the IL-4R antagonist to the subject; and
   (b) providing an indication that treatment should be continued if the expression level of TARC or eotaxin-3, or total serum level of IgE has decreased as compared to the level before treatment with the IL-4R antagonist.
108. An IL-4R antagonist for use in the treatment and/or prevention of asthma and related conditions.
109. A pharmaceutical composition comprising an anti-IL4R antibody antagonist or an antigen binding fragment thereof for use in the treatment and/or prevention of asthma and related conditions.
110. The pharmaceutical composition of item 109, wherein the pharmaceutical composition comprises 75 mg to 600 mg of the antibody or antigen-binding fragment thereof that specifically binds to IL-4R.
111. The pharmaceutical composition of item 110, wherein the pharmaceutical composition comprises 300 mg of the antibody or antigen-binding fragment thereof that specifically binds to IL-4R.
112. The pharmaceutical composition of any one of items 108-110, wherein the antibody or antigen-binding fragment thereof that specifically binds to IL-4R comprises heavy and light chain complementarity determing region (CDR) sequences from a heavy chain variable region (HCVR)/light chain variable region (LCVR) sequence pair selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, and 262/264.
113. The pharmaceutical composition of item 112, wherein the antibody or antigen-binding fragment thereof that specifically binds to IL-4R comprises heavy and light chain CDR sequences from the HCVR/LCVR sequence pair comprising SEQ ID NOs: 162/164.
114. The pharmaceutical composition of any one of items 109-113, wherein the antibody or antigen-binding fragment thereof that specifically binds to IL-4R comprises three heavy chain complementarity determining region (HCDR) sequences comprising SEQ ID NOs: 148, 150, 152, respectively, and three light chain complementarity determining region (LCDR) sequences comprising SEQ ID NOs: 156, 158 and 160, respectively.
115. The pharmaceutical composition of item 113, wherein the antibody or antigen-binding fragment thereof that specifically binds to IL-4R comprises an HCVR comprising the amino acid sequence of SEQ ID NO: 162 and an LCVR comprising the amino acid sequence of SEQ ID NO: 164.
116. The pharmaceutical composition of any one of items 109-115, wherein the pharmaceutical composition is administered to the subject systemically, subcutaneously, intravenously, or intranasally.
117. The pharmaceutical composition of any one of items 109-116, wherein a second therapeutic agent is administered to the subject before, after, or concurrent with the pharmaceutical composition.
118. The pharmaceutical composition of item 117, wherein the second therapeutic agent is selected from the group consisting of: a TNF inhibitor, an IL-1 inhibitor, an IL-5 inhibitor, an IL-8 inhibitor, an IgE inhibitor, a leukotriene inhibitor, a corticosteroid, a methylxanthine, an NSAID, nedocromil sodium, cromolyn sodium, a long-acting beta₂ agonist, an anti-fungal agent, and a combination thereof.
119. The pharmaceutical composition of item 118, wherein the second therapeutic agent comprises a combination of an inhaled corticosteroid and a long-acting beta₂ agonist.
120. The pharmaceutical composition of item 119, wherein the inhaled corticosteroid is fluticasone or budesonide.
121. The pharmaceutical composition of item 119, wherein the long-acting beta₂ agonist is salmeterol or formoterol.
122. The pharmaceutical composition of item 119, wherein the inhaled corticosteroid is fluticasone and the long-acting beta₂ agonist is salmeterol.
123. The pharmaceutical composition of item 119, wherein the inhaled corticosteroid is budesonide and the long-acting beta₂ agonist is formoterol.
124. The pharmaceutical composition of any one of items 109-123, wherein the related conditions are seletected from the group consisting of chronic rhino sinusitis, allergic rhinitis, allergic fungal rhino sinusitis, allergic broncho-pulmonary aspergillosis, unified airway disease, Churg-Strauss syndrome, vasculitis, chronic obstructive pulmonary disease (COPD), and exercise-induced bronchospasm.
125. A pharmaceutical composition comprising an anti-IL4R antibody antagonist or an antigen binding fragment thereof for use in reducing the incidence of one or more asthma exacerbations in a subject in need thereof.
126. The pharmaceutical composition of item 125, wherein the asthma exacerbation is selected from the group consisting of:
   (a) a 30% or greater reduction from baseline in morning peak expiratory flow (PEF) on two consecutive days;
   (b) six or more additional reliever puffs of albuterol or levalbuterol in a 24 hour period (compared to baseline) on two consecutive days; and
   (c) a deterioration of asthma requiring:
      (i) systemic (oral and/or parenteral) steroid treatment, or
      (ii) an increase in inhaled corticosteroids to at least 4 times the last dose received prior to discontinuation, or
      (iii) hospitalization.
127. A pharmaceutical composition comprising an anti-IL4R antibody antagonist or an antigen binding fragment thereof for use in improving one or more asthma-associated parameter(s) in a subject in need thereof.
128. The pharmaceutical composition of item 127, wherein the improvement in an asthma-associated parameter is selected from the group consisting of:
   (a) an increase from baseline of forced expiratory volume in 1 second (FEV1) in liters;
   (b) an increase from baseline of morning peak expiratory flow rate (AM PEF) in liters/minute;
   (c) an increase from baseline of evening peak expiratory flow rate (PM PEF) in liters/minute;
   (d) a decrease from baseline of daily albuterol/levalbuterol use in inhalations/day;
   (e) a decrease from baseline of five-item Asthma Control Questionnaire (ACQ5) score;
   (f) a decrease from baseline of nighttime awakenings (no. of times per night) measured daily; and
   (g) a decrease from baseline of 22-item Sino-Nasal Outcome Test (SNOT-22) score.
129. A pharmaceutical composition comprising an anti-IL4R antibody antagonist or an antigen binding fragment thereof for use in the treatment of asthma and related conditions in a patient having an elevated level of a biomarker selected from the group consisting of thymus and activation-regulated chemokine (TARC), IgE, eotaxin-3, periostin, carcinoembryonic antigen (CEA), YKL-40, and fractional exhaled nitric oxide (FeNO).
130. The pharmaceutical composition of item 129, wherein the related conditions are seletected from the group consisting of chronic rhino sinusitis, allergic rhinitis, allergic fungal rhino sinusitis, allergic broncho-pulmonary aspergillosis, unified airway disease, Churg-Strauss syndrome, vasculitis, chronic obstructive pulmonary disease (COPD), and exercise-induced bronchospasm.
131. A pharmaceutical composition comprising an anti-IL4R antibody antagonist or an antigen binding fragment thereof for use in the treatment of astham or moderate to severe eosinophilic asthma in a subject in need thereof wherein the treatment comprises testing the patient for the presence of a blood eosinophil level of at least 300 cells per microliter and/or a sputum eosinophil level of at least 3% and beginning/continuing administration of the pharmaceutical composition if such blood eosinophil level and/or sputum eosinophil level is found.

## Claims

1. A pharmaceutical composition comprising an interleukin-4 receptor (IL-4R) antagonist for use in:
(1) treating asthma in a subject in need thereof;
(2) improving one or more asthma-associated parameter(s) in a subject in need thereof; or
(3) reducing the incidence of asthma exacerbations in a subject in need thereof,
wherein the IL-4R antagonist is an antibody or antigen-binding fragment thereof that specifically binds IL-4R, wherein the antibody or antigen-binding fragment thereof comprises three heavy chain complementarity determining region (HCDR) sequences comprising SEQ ID NOs: 148, 150, 152, respectively, and three light chain complementarity determining region (LCDR) sequences comprising SEQ ID NOs: 156, 158 and 160, respectively.

2. The pharmaceutical composition for use of claim 1, wherein the antibody or antigen-binding fragment thereof comprises a heavy chain variable region (HCVR) having the amino acid sequence of SEQ ID NO:162 and a light chain variable region (LCVR) having the amino acid sequence of SEQ ID NO:164.

3. The pharmaceutical composition for use of claim 1 or 2, wherein the subject has eosinophilic asthma.

4. The pharmaceutical composition for use of any one of claims 1 to 3, wherein the subject has moderate to severe asthma, optionally moderate to severe eosinophilic asthma.

5. The pharmaceutical composition for use of any one of claims 1 to 4, wherein the subject has a blood eosinophil level of at least 300 cells per microliter, and/or a sputum eosinophil level of at least 3%.

6. The pharmaceutical composition for use of any one of claims 1 to 5, wherein the treatment results in a reduction in the incidence of asthma exacerbations.

7. The pharmaceutical composition for use of any one of claims 1 to 6, wherein the asthma exacerbations are:
(a) a 30% or greater reduction from baseline in morning peak expiratory flow ("AM PEF") on two consecutive days;
(b) six or more additional reliever puffs of albuterol or levalbuterol in a 24-hour period compared to baseline on two consecutive days; and/or
(c) a deterioration of asthma requiring at least one of:
(i) systemic (oral and/or parenteral) steroid treatment;
(ii) an increase in inhaled corticosteroids to at least 4 times the baseline level; and/or
(iii) hospitalization.

8. The pharmaceutical composition for use of claim 6, wherein the asthma exacerbations are severe asthma exacerbations.

9. The pharmaceutical composition for use of any one of claims 1 to 5, wherein the improvement in an asthma-associated parameter is:
(a) an increase from baseline of:
(i) forced expiratory volume in 1 second (FEV1);
(ii) morning peak expiratory flow rate (AM PEF); and/or
(iii) evening peak expiratory flow rate (PM PEF); and/or
(b) a decrease from baseline of:
(i) daily albuterol or levalbuterol use;
(ii) five-item Asthma Control Questionnaire (ACQ5) score; and/or
(iii) average nighttime awakenings.

10. The pharmaceutical composition for use of any one of claims 1 to 9, wherein the pharmaceutical composition is administered to the subject who has been taking a regular course of inhaled corticosteroid (ICS)/long-acting beta2 agonist (LABA) for two or more weeks immediately preceding the administration of the pharmaceutical composition.

11. The pharmaceutical composition for use of any one of claims 1 to 10, wherein the subject is administered one or more additional therapeutic agents in combination with the pharmaceutical composition.

12. The pharmaceutical composition for use of claim 11, wherein the one or more additional therapeutic agents is another IL-4R antagonist, an IL-1 antagonist, an IL-6 antagonist, an IL-6R antagonist, a TNF antagonist, an IL-8 antagonist, an IL-9 antagonist, an IL-17 antagonist, an IL-5 antagonist, an IgE antagonist, a CD48 antagonist, an anti-fungal agent, an NSAID, a long-acting beta2 agonist, an inhaled corticosteroid, or combinations thereof.

13. The pharmaceutical composition for use of claim 12, wherein the one or more additional therapeutic agents is a combination comprising a long-acting beta2 agonist and an inhaled corticosteroid.

14. The pharmaceutical composition for use of any one of claims 1 to 13, wherein the pharmaceutical composition is administered to the subject subcutaneously.

15. The pharmaceutical composition for use of any one of claims 1 to 14, wherein the pharmaceutical composition comprises 300 mg of the antibody.

16. The pharmaceutical composition for use of any one of claims 1 to 15, wherein:
(a) the pharmaceutical composition is administered to the subject subcutaneously with a standard needle and syringe; or
(b) the pharmaceutical composition is administered to the subject subcutaneously using a pen delivery device, preferably wherein the pen delivery device is reusable or disposable, more preferably wherein a reusable pen delivery device comprises a replaceable cartridge that contains the pharmaceutical composition, and wherein a disposable pen delivery device comprises the pharmaceutical composition held in a reservoir within the device.
